(19) Europäisches Patentamt
European Patent Office
Office européen des brevets

(11) Veröffentlichungsnummer: **0 078 241**
**A2**

(12) # EUROPÄISCHE PATENTANMELDUNG

(21) Anmeldenummer: 82810439.8

(22) Anmeldetag: 22.10.82

(51) Int. Cl.³: C 07 D 307/83
A 61 K 31/365, A 61 K 31/40
A 61 K 31/445, A 61 K 31/535
A 61 K 31/55, A 61 K 7/42

(30) Priorität: 28.10.81 CH 6882/81

(43) Veröffentlichungstag der Anmeldung:
04.05.83 Patentblatt 83/18

(84) Benannte Vertragsstaaten:
AT BE CH DE FR IT LI LU NL SE

(71) Anmelder: CIBA-GEIGY AG
Patentabteilung Postfach
CH-4002 Basel(CH)

(72) Erfinder: Wenk, Paul, Dr.
Quellenweg 7
CH-4123 Allschwil(CH)

(72) Erfinder: Breitenstein, Werner, Dr.
St. Galler-Ring 179
CH-4054 Basel(CH)

(72) Erfinder: Baumann, Marcus, Dr.
Rührbergerstrasse 6
CH-4058 Basel(CH)

(54) **Furane.**

(57) Die Erfindung betrifft neue Furane, insbesondere Benzofuranone der Formel

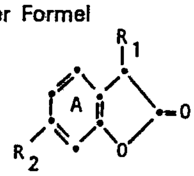

(1)

worin $R_1$ für Wasserstoff oder einen aliphatischen Rest steht, $R_2$ eine durch einen zweiwertigen Kohlenwasserstoffrest disubstituierte Aminogruppe bedeutet und der aromatische Ring A zusätzlich substituiert sein kann, und ihre Salze und/oder Isomeren, Verfahren zur Herstellung von Verbindungen der Formel (I) und ihrer Salze und Isomeren, solche Verbindungen enthaltende pharmazeutische Präparate sowie deren Verwendung als Arzneimittelwirkstoffe und/ oder zur Herstellung pharmazeuzeutischer Präparate. Die Verbindungen der Formel (I) zeichnen sich durch ausgeprägte antiinflammatorische und analgetische Eigenschaften aus.

EP 0 078 241 A2

Croydon Printing Company Ltd

CIBA-GEIGY AG
Basel (Schweiz)

4-13615/+

Furane

Die Erfindung betrifft neue Furane, insbesondere Benzofuranone der allgemeinen Formel

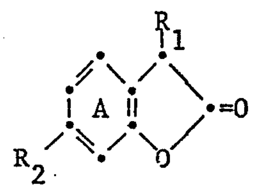

(I),

worin $R_1$ für Wasserstoff oder einen aliphatischen Rest steht, $R_2$ eine durch einen zweiwertigen Kohlenwasserstoffrest disubstituierte Aminogruppe bedeutet und der aromatische Ring A zusätzlich substituiert sein kann, und ihre Salze und/oder Isomeren, Verfahren zur Herstellung von Verbindungen der Formel (I) und ihrer Salze und Isomeren, solche Verbindungen enthaltende pharmazeutische Präparate sowie deren Verwendung als Arzneimittelwirkstoffe und/oder zur Herstellung pharmazeutischer Präparate.

Isomere von Verbindungen der Formel (I) sind beispielsweise die zu den 2,3-Dihydro-2-oxo-benzo[b]furan-Derivaten der Formel (I) im tautomeren Gleichgewicht befindlichen 2-Hydroxy-benzo[b]furan-Verbindungen der Formel

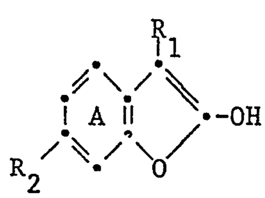

(I').

Ein aliphatischer Rest $R_1$ ist insbesondere gesättigt und unsubstituiert und stellt in erster Linie einen Niederalkylrest dar.

Der aromatische Ring A kann zusätzlich durch einen aliphatischen Rest, wie Niederalkyl, Hydroxyniederalkyl, Halogenniederalkyl, Niederalkenyl oder gegebenenfalls verzweigtes, insbesondere zwei benachbarte C-Atome überbrückendes, 3- oder 4-gliedriges Alkylen mit 3-8 C-Atomen, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehfach substituiert oder,bis auf $R_2$, unsubstituiert sein.

Eine durch einen zweiwertigen Kohlenwasserstoffrest disubstituierte Aminogruppe weist als solchen einen zweiwertigen aliphatischen Rest, der auch durch Aza, N-Niederalkylaza, Oxa oder Thia unterbrochen sein kann, wie Niederalkylen, Niederalkenylen oder durch jeweils Aza, N-Niederalkylaza, Oxa oder Thia unterbrochenes Niederalkylen bzw. Niederalkenylen,auf, wobei Niederalkylen bzw. Niederalkenylen jeweils auch verzweigt sein können. Ferner können derartige cyclische Amine $R_2$ auch ein oder zwei ortho-anellierte Benzosysteme aufweisen. $R_2$ stellt vorzugsweise jeweils 5- bis 8-gliedriges Niederalkylen-, Niederalkenylen-, Aza-niederalkylen-, N'-Niederalkylaza-niederalkylen-, Oxa-niederalkylen-, Thia-niederalkylen-, Aza-niederalkenylen-, N'-Niederalkylaza-niederalkenylen-, Oxa- bzw. Thia-niederalkenylen-amino dar, wobei Niederalkylen bzw. Niederalkenylen auch verzweigt sein und entsprechend 4 bis 14, vorzugsweise 4 bis 7 C-Atome aufweisen kann.

Als Beispiele für derartige Reste $R_2$ seien genannt: Pyrrolidin-1-yl, 2- oder 3-Pyrrolin-1-yl, Pyrrol-1-yl, Piperidin-1-yl, Azepin-1-yl, Imidazolidin-1-yl, 2-, 3- oder 4-Imidazolin-1-yl, Oxazolidin-3-yl, 4-Oxazolin-3-yl, Thiazolidin-3-yl, 4-Thiazolidin-3-yl , Piperazin-1-yl, Morpholin-4-yl, Thiomorpholin-4-yl, 3-Methyl-imidazolidin-1-yl, 4-Methyl-piperazin-1-yl.

Ferner bedeutet $R_2$ Niederalkylen- bzw. Niederalkenylen-amino mit einem oder zwei ortho-anellierten Benzosystemen, wie Indol-1-yl, Indolin-1-yl, Isoindol-2-yl, Isoindolin-2-yl, Carbazol-9-yl oder β-Carbolin-9-yl.

Vor- und nachstehend sind unter mit "nieder" bezeichneten organischen Resten oder Verbindungen vorzugsweise solche zu verstehen, die bis und mit 7, vor allem bis und mit 4 Kohlenstoffatome enthalten.

Die im Rahmen des vorliegenden Textes verwendeten Allgemeindefinitionen haben in erster Linie die folgenden Bedeutungen:

Niederalkyl ist z.B. Methyl, Ethyl, n-Propyl, Isopropyl, n-Butyl, Isobutyl, sek.-Butyl, tert.-Butyl und umfasst ferner entsprechend Pentyl-, Hexyl- oder Heptylreste.

Hydroxyniederalkyl ist z.B. Hydroxymethyl, 2-Hydroxyethyl oder 2- oder 3-Hydroxypropyl. Halogenniederalkyl ist z.B. Chlormethyl oder Trifluormethyl.

Niederalkenyl ist z.B. Vinyl, 1- bzw. 2-Propenyl, 1-, 2- oder 3-Butenyl oder Butadien-1,3-yl.

3- oder 4-gliedriges Alkylen weist insbesondere 3- bis 8 C-Atome auf und ist geradkettig, wie Tri- oder Tetramethylen, oder verzweigt, wie 2,4-Butylen, 1,4- bzw. 2,4-Pentylen oder 2-Methyl-1,3-propylen.

Niederalkoxy ist z.B. Methoxy, Ethoxy, n-Propyloxy, Isopropyloxy, n-Butyloxy, Isobutyloxy, sek.-Butyloxy, tert.-Butyloxy und umfasst ferner entsprechende Pentyloxy-, Hexyloxy- oder Heptyloxyreste.

Niederalkylthio ist z.B. Methyl-, Ethyl, n-Propyl-, Isopropyl-, n-Butyl-, Isobutyl-, sek-Butyl- oder tert.-Butylthio. Niederalkansulfinyl bzw. -sulfonyl ist z.B. Methan-, Ethan-, n-Propan- oder Isopropan-sulfinyl bzw. -sulfonyl.

Halogen ist z.B. Halogen bis und mit Atomnummer 35, wie Fluor, Chlor oder Brom, und umfasst ferner Jod.

Niederalkanoyloxy ist z.B. Acetyloxy, Propionyloxy, Butyryloxy, Isobutyryloxy, sek.- oder tert.-Butyryloxy.

Niederalkanoyl ist z.B. Acetyl, Propionyl, Butyryl, Isobutyryl oder tert.-Butyryl.

Niederalkylen ist z.B. 4- bis 7-gliedriges Niederalkylen und weist z.B. 4- bis 10, insbesondere 4 bis 6 C-Atome auf, wie Tetra-, Penta- oder Hexamethylen, ferner Heptamethylen.

Niederalkenylen weist eine oder zwei Doppelbindungen auf und ist z.B. 4- bis 7-gliedriges Niederalkenylen, z.B. mit 4 bis 10, insbesondere 4 bis 6 C-Atome, wie But-2-en-1,4-ylen, Buta-1,3-dien-1,4-ylen, Pent-2-en-1,5-ylen, Penta-1,3-dien-1,5-ylen, Penta-1,4-dien-1,5-ylen, Hex-3-en-2,5-ylen oder Hexa-2,4-dien-2,4-ylen.

Durch Aza bzw. N-Niederalkylaza unterbrochenes Niederalkylen ist z.B. 4- bis 7-gliedriges Monoaza- bzw. N'-Niederalkylmonoaza-niederalkylen, wie 2-Aza-tetramethylen, 3-Aza-pentamethylen oder 3-Methylaza-pentamethylen.

Durch Oxa bzw. Thia unterbrochenes Niederalkylen ist z.B. Monooxa- bzw. Monothia-niederalkylen, wie 3-Oxa- bzw. 3-Thia-pentamethylen.

Durch Aza bzw. N-Niederalkylaza unterbrochenes Niederalkenylen mit einer oder zwei Doppelbindungen ist z.B. 2-Aza-buten-1-ylen, 2-Aza-buten-2-ylen, 2-Aza-buten-3-ylen, 2-Methylaza-buten-3-ylen oder 2-Aza-butadien-1,3-ylen.

Salze von erfindungsgemässen Verbindungen der Formel (I) sind vorzugsweise pharmazeutisch verwendbare Salze, wie pharmazeutisch verwendbare Säureadditionssalze. Diese werden beispielsweise mit starken anorganischen Säuren, wie Mineralsäuren, z.B. Schwefelsäure, Phosphorsäure oder Halogenwasserstoffsäuren, mit starken organischen Carbonsäuren, wie Niederalkancarbonsäuren, z.B. Essigsäure, wie gegebenenfalls ungesättigte Dicarbonsäuren, z.B. Oxal-, Malon-, Malein- oder Fumarsäure, oder wie Hydroxycarbonsäuren, z.B. Weinsäure oder Citronensäure, oder mit Sulfonsäuren, wie Niederalkan- oder gegebenenfalls substituierte Benzolsulfonsäuren, z.B. Methan- oder p-Toluolsulfonsäure gebildet. Weist der 1,2-Phenylenrest Ph als Substituenten Hydroxy auf, können entsprechende Verbindungen Salze mit Basen bilden. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze.

Isomere der Formel (I) liegen insbesondere als Strukturisomere vor. Weisen z.B. Verbindungen der Formel (I) chirale C-Atome auf, können sie als Diastereomere, Diastereomerengemische, Racemate oder in Form eines reinen Enantiomeren vorliegen, während z.B. die Tautomeren der Formel (I') z.B. geometrische Isomere, z.B. E/Z-Isomere, bilden.

Die Verbindungen der Formel (I) weisen wertvolle pharmakologische Eigenschaften auf. Insbesondere besitzen sie eine ausgeprägte antiinflammatorische Wirkung, die sich z.B. durch Reduktion des durch Carrageenin erzeugten Pfotenödems bei der Ratte ab einer Dosis von etwa 0,1 mg/kg p.o. analog der von Pasquale et al., Ag. and Actions, 5, 256 (1975), beschriebenen Methode sowie im Adjuvans-Arthritis-Modell an der Ratte ab einer Dosis von etwa 1.0 mg/kg p.o. analog L. Risterer et al., Pharmacology, 2, (1969), nachweisen lässt. Ausserdem hemmen Verbindungen der Formel (I) in vitro ab einer Konzentration von etwa 10 μ mol/l die Prostaglandinsynthese aus Arachidonsäure analog der von H.L. White et al. Prostaglandins, 7, 123 (1974), beschriebenen Methode.

Weiterhin weisen die Verbindungen der Formel (I) eine deutliche antinociceptive Wirkungskomponente auf, die sich z.B. aus der von L.C. Hendershot et al., J. Pharmacol. exp. Therap. 125, 237 (1959), beschriebenen Reduktion des durch Phenyl- p-Benzochinon induzierten Writhing-Syndroms an der Maus ab einer Dosis von etwa 0,1 mg/kg p.o. ableiten lässt.

Ferner zeigen die Verbindungen der Formel (I) die Fähigkeit, aus dem Bereich des UV-Spektrums die auf der Epidermis Erythreme erzeugenden Strahlen (zwischen 290 und 320 nm) zu absorbieren, während die Substanzen für die bräunend wirkenden Strahlen von etwa 320 bis etwa 400 nm durchlässig sind.

Infolgedessen lassen sich diese Verbindungen als Antiinflammatorika, (periphere) Analgetika und/oder Lichtschutzmittel, z.B. für kosmetische Zwecke, verwenden.

Die Erfindung betrifft beispielsweise Verbindungen der Formel (I), worin $R_1$ Wasserstoff oder einen gesättigten und unsubstituierten aliphatischen Rest bedeutet, $R_2$ eine durch einen zweiwertigen aliphatischen Rest, der auch durch Aza, N-Niederalkylaza, Oxa oder Thia unterbrochen sein kann, disubstituierte Aminogruppe darstellt und der aromatische Ring A zusätzlich durch einen aliphatischen Rest, Niederalkoxy, Niederalkylthio, Niederalkansulfinyl, Niederalkansulfonyl, Hydroxy, Halogen, Niederalkanoyloxy, Niederalkanoyl und/oder Nitro

ein- oder mehrfach substituiert oder, bis auf $R_2$, unsubstituiert ist, und ihre Salze, insbesondere pharmazeutisch verwendbare Salze, und Isomeren.

Die Erfindung betrifft beispielsweise Verbindungen der Formel (I), worin $R_1$ Wasserstoff oder Niederalkyl bedeutet, $R_2$ eine durch Niederalkylen, Niederalkenylen, Aza-niederalkylen, N'-Niederalkylaza-niederalkylen, Aza-niederalkenylen, N'-Niederalkylaza-niederalkenylen oder Oxa- bzw. Thia-niederalkenylen disubstituierte Aminogruppe, wobei Niederalkylen bzw. Niederalkenylen jeweils 4- bis 10 C-Atome aufweist und auch verzweigt sein kann sowie mit einem oder zwei Benzosystemen ortho-anelliert sein kann, darstellt und der aromatische Ring A zusätzlich durch Niederalkyl, Hydroxyniederalkyl, Halogenniederalkyl, Niederalkenyl, gegebenenfalls verzweigtes 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze, insbesondere pharmazeutisch verwendbare Salze, und Isomeren.

Die Erfindung betrifft beispielsweise Verbindungen der Formel (I), worin $R_1$ Wasserstoff oder Niederalkyl bedeutet, $R_2$ eine durch Niederalkylen, Niederalkenylen oder durch Aza, N-Niederalkylaza, Oxa oder Thia unterbrochenes Niederalkylen bzw. durch Aza oder N-Niederalkylaza unterbrochenes Niederalkenylen substituierte Aminogruppe darstellt und der aromatische Ring A zusätzlich durch Niederalkyl, Niederalkoxy, Hydroxy, Halogen, Niederalkanoyloxy, 3- oder 4-gliedriges Alkylen und/oder Trifluormethyl substituiert sein kann, und ihre Salze, insbesondere pharmazeutisch verwendbare Salze, und Isomeren.

Die Erfindung betrifft insbesondere Verbindungen der Formel

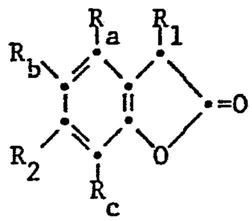

(Ia),

worin $R_1$ Wasserstoff oder Niederalkyl, wie Methyl, bedeutet, $R_2$ jeweils 5- bis 8-gliedriges Niederalkylenamino, wie Pyrrolidin-1-yl, Niederalkenylenamino, wie Pyrrol-1-yl oder 3-Pyrrolin-1-yl, Aza-niederalkylen-amino, wie Piperazin-1-yl, N'-Niederalkylaza-niederalkylen-amino, wie 4-Methyl-piperazin-1-yl, Aza-niederalkenylen-amino, wie Imidazol-1-yl, N'-Niederalkylaza-niederalkenylen-amino, wie 3-Methyl-imidazol-1-yl, Oxa- bzw. Thia-niederalkylen-amino, wie Morpholino-4-yl oder Thiomorpholin-4-yl, Isoindol-2-yl, Isoindolin-2-yl, Indolin-1-yl oder Indol-1-yl darstellt und $R_a$, $R_b$ sowie $R_c$ unabhängig voneinander Wasserstoff, Niederalkyl, wie Methyl, Hydroxyniederalkyl, wie Hydroxymethyl, Halogenniederalkyl, wie Trifluormethyl, Niederalkenyl, wie 2-Propenyl, Niederalkoxy, wie Methoxy, Niederalkylthio, wie Methylthio, Niederalkansulfinyl, wie Methansulfinyl, Niederalkansulfonyl, wie Methansulfonyl, Hydroxy, Halogen, wie Brom oder Chlor, Niederalkanoyloxy, wie Acetyloxy, Niederalkanoyl, wie Acetyl, oder Nitro bedeuten oder $R_a$ gemeinsam mit $R_b$ 3- oder 4-gliedriges Alkylen, wie Tetramethylen, darstellen und $R_c$ die vorstehend für $R_c$ angegebenen Bedeutungen hat, und ihre Salze, insbesondere pharmazeutisch verwendbare Salze und Isomeren.

Die Erfindung betrifft insbesondere Verbindungen der Formel (Ia), worin $R_1$ Wasserstoff oder Niederalkyl, wie Methyl, bedeutet, $R_2$ jeweils 5- bis 8-gliedriges Niederalkylenamino, wie Pyrrolidin-1-yl, Niederalkenylenamino, wie Pyrrol-1-yl, Monoaza-niederalkylenamino, wie Piperazin-1-yl, N'-Niederalkylmonoaza-niederalkylenamino, wie 4-Methyl-piperazin-1-yl, Monooxa-niederalkylenamino, wie Morpholin-4-yl, Monothia-niederalkylenamino, wie Thiomorpholin-4-yl, Monoaza-niederalkenylenamino, wie Imidazol-1-yl, oder N'-Niederalkylaza-niederalkenylenamino, wie 3-Methyl-imidazol-1-yl, darstellt und $R_a$,

$R_b$ sowie $R_c$ unabhängig voneinander Wasserstoff, Niederalkyl, wie Methyl, Niederalkoxy, wie Methoxy, Hydroxy, Halogen, wie Chlor, Niederalkanoyloxy, wie Acetyloxy,oder Trifluormethyl bedeuten oder $R_a$ gemeinsam mit $R_b$ 3- oder 4-gliedriges Alkylen, wie Tetramethylen, darstellen und $R_c$ die vorstehend für $R_c$ angegebenen Bedeutungen hat, und ihre Salze, insbesondere pharmazeutisch verwendbare Salze, und Isomeren.

Die Erfindung betrifft vor allem Verbindungen der Formel (Ia), worin $R_1$ Wasserstoff oder Niederalkyl mit bis und mit 4 C-Atomen, wie Methyl, bedeutet, $R_2$ 5- bis 8-gliedriges Niederalkylen-amino mit 4 bis 10 C-Atomen, wie Pyrrolidin-1-yl oder 3,4-Dimethyl-pyrrolidin-1-yl, 5- bis 8-gliedriges Niederalkenylen-amino mit einer oder zwei Doppelbindungen und mit 4 bis 10 C-Atomen, wie 3-Pyrrolin-1-yl oder Pyrrol-1-yl, Monooxa-niederalkylen-amino mit 4 bis 7 C-Atomen, wie Morpholin-4-yl, Indolin-1-yl oder Indol-1-yl darstellt und $R_a$ und $R_b$ unabhängig voneinander Wasserstoff, Niederalkyl, insbesondere mit bis und mit 4 C-Atomen, wie Methyl, oder Halogen, insbesondere bis und mit Atomnummer 35, wie Chlor oder Brom, oder $R_a$ und $R_b$ gemeinsam 3- bis 4-gliedriges Alkylen, wie Tetramethylen, bedeuten und $R_c$ Wasserstoff darstellt, und ihre Salze, insbesondere pharmazeutisch verwendbare Salze, und Isomeren.

Die Erfindung betrifft vor allem Verbindungen der Formel (Ia), worin $R_1$ Wasserstoff oder Niederalkyl, insbesondere mit bis und mit 4 C-Atomen, wie Methyl,bedeutet, $R_2$ 5- bis 8-gliedriges Niederalkylenamino, wie Pyrrolidin-1-yl, 5- bis 8-gliedriges Niederalkenylenamino, wie Pyrrol-1-yl, oder Monooxa-niederalkylenamino, wie Morpholin-4-yl, darstellt und $R_a$ sowie $R_b$ unabhängig voneinander Wasserstoff, Niederalkyl, insbesondere mit bis und mit 4 C-Atomen, wie Methyl, oder Halogen, insbesondere bis und mit Atomnummer 35, wie

Chlor oder Brom, oder $R_a$ gemeinsam mit $R_b$ 3- bis 4-gliedriges Alkylen, wie Tetramethylen, bedeuten, und $R_c$ Wasserstoff darstellt, und ihre Salze, insbesondere pharmazeutisch verwendbaren Salze, und Isomeren.

Die Erfindung betrifft in erster Linie Verbindungen der Formel (Ia), worin $R_1$ Wasserstoff oder Niederalkyl mit bis und mit 4 C-Atomen, wie Methyl, bedeutet, $R_2$ 5- bis 8-gliedriges Niederalkylenamino, wie Pyrrolidin-1-yl, 5- bis 8-gliedriges Niederalkenylenamino, wie Pyrrol-1-yl, oder Monooxa-niederalkylenamino, wie Morpholin-4-yl, darstellt und $R_a$ und $R_c$ Wasserstoff bedeuten und $R_b$ Wasserstoff, Niederalkyl mit bis und mit 4 C-Atomen, insbesondere Methyl, oder Halogen bis und mit Atomnummer 35, insbesondere Chlor oder Brom, darstellt, und ihre Salze, insbesondere pharmazeutisch verwendbare Salze, und Isomeren.

Die Erfindung betrifft in aller erster Linie Verbindungen der Formel (Ia), worin $R_1$ Wasserstoff oder Niederalkyl mit bis und mit 4 C-Atomen, insbesondere Methyl, bedeutet, $R_2$ 1-Pyrrolyl, 3-Pyrrolin-1-yl, Pyrrolidin-1-yl oder Piperidin-1-yl darstellt, $R_a$ und $R_c$ jeweils Wasserstoff bedeuten und $R_b$ Niederalkyl mit bis und mit 4 C-Atomen, insbesondere Methyl, oder Halogen bis und mit Atomnummer 35, insbesondere Chlor oder Brom, darstellt, und ihre Salze, insbesondere pharmazeutisch verwendbare Salze, und Isomeren.

Die Erfindung betrifft vor allem Verbindungen der Formel (Ia), worin $R_1$ Wasserstoff oder Niederalkyl mit bis und mit 4 C-Atomen, insbesondere Methyl, bedeutet, $R_2$ 1-Pyrrolyl, 4-Morpholinyl, 3-Pyrrolin-1-yl oder unverzweigtes 4- bis 6-gliedriges Alkylenamino, wie Piperidin-1-yl, darstellt, $R_a$ und $R_c$ jeweils Wasserstoff bedeuten und $R_b$ Wasserstoff, Niederalkyl mit bis und mit 4 C-Atomen, insbesondere Methyl, oder Halogen bis und mit Atomnummer 35, insbesondere Chlor oder Brom, darstellt, oder $R_c$ Wasserstoff bedeutet und $R_a$ und $R_b$ gemeinsam 3- bis 4-gliedriges Alkylen, insbesondere Tetramethylen,

oder einer der Reste $R_a$ und $R_b$ Halogen bis und mit Atomnummer 35, insbesondere Brom, und der andere Niederalkyl mit und bis 4 C-Atomen, insbesondere Methyl, darstellen, und ihre Salze, insbesondere pharmzeutisch verwendbare Salze, und Isomeren.

Die Erfindung betrifft in allerersten Linie Verbindungen der Formel (Ia), worin $R_1$ Niederalkyl mit bis und mit 4 C-Atomen, insbesondere Methyl, bedeutet, $R_2$ 5- bis 8-gliedriges Niederalkenylenamino, insbesondere Pyrrol-1-yl,

darstellt, $R_a$ und $R_c$ Wasserstoff bedeuten und $R_b$ Niederalkyl mit bis und mit 4 C-Atomen, insbesondere Methyl, darstellt, und ihre Salze, insbesondere pharmazeutisch verwendbare Salze, und ihre Isomeren.

Die Erfindung betrifft insbesondere die in den Beispielen genannten neuen Verbindungen, ihre Salze, vor allem pharmazeutisch verwendbare Salze, und Isomeren, ferner die in den Beispielen beschriebenen Verfahren zu ihrer Herstellung.

Die Verbindungen der vorliegenden Erfindung werden nach an sich bekannter Weise hergestellt, beispielsweise indem man

a) eine Verbindung der Formel

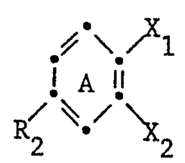

(II)

oder ein Salz davon, worin $X_1$ eine Gruppe der Formel $-CH(R_1)-X_3$ und $X_3$ Carboxy oder funktionell abgewandeltes Carboxy bedeutet und $X_2$ Hydroxy oder funktionell abgewandeltes Hydroxy darstellt oder worin $X_1$ Wasserstoff und $X_2$ eine Gruppe der Formel $-O-CO-CH(R_1)-X_4$ bedeutet, in der $X_4$ Hydroxy oder funktionell abgewandeltes Hydroxy darstellt, cyclisiert oder

b) in einer Verbindung der Formel

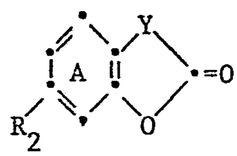

(III),

einem Salz oder Isomeren davon, worin Y einen in die Gruppe der Formel $>CH(R_1)$ überführbaren Rest darstellt, Y in die Gruppe der Formel $>CH(R_1)$ überführt oder

c) in einer Verbindung der Formel

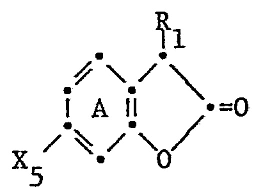

(IV),

oder einem Salz oder Isomeren davon, worin $X_5$ eine in $R_2$ überführbare Gruppe darstellt, $X_5$ in $R_2$ überführt oder

d) in einer Verbindung der Formel

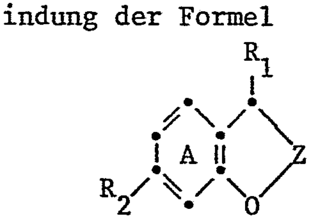

(V)

oder einem Salz davon, worin Z eine in die Carbonylgruppe überführbare Gruppe bedeutet, Z in die Carbonylgruppe überführt oder

e) in einer Verbindung der Formel

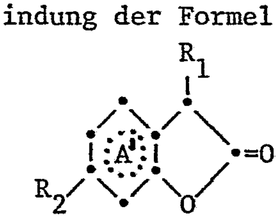

(VI)

oder einem Salz davon, worin der Ring A' ein in den Ring A überführbarer Ring ist, den Ring A' in den Ring A überführt und/oder, wenn erwünscht, ein verfahrensgemäss erhältliches

Salz in die freie Verbindung oder in ein anderes Salz überführt, und/ oder, wenn erwünscht, eine verfahrensgemäss erhältliche freie Verbindung in eine andere freie Verbindung oder in ein Salz überführt und/ oder gewünschtenfalls ein verfahrensgemäss erhältliches Isomerengemisch in ihre Komponenten auftrennt.

Zu Variante a): Ausgangsstoffe der Formel II können als Salze vorliegen, beispielsweise als Säureadditionssalze oder, sofern die Gruppe $X_3$ Carboxy bzw. der aromatische Ring A Hydroxy enthält, als Salze mit Basen.

Funktionell abgewandeltes Carboxy $X_3$ bedeutet in erster Linie eine Oxogruppe aufweisendes, funktionell abgewandeltes Carboxy, wie verestertes, amidiertes oder anhydridisiertes Carboxy, ferner eine Orthocarbonsäureester-, Orthoanhydridgruppierung oder gegebenenfalls funktionell abgewandeltes Thiocarboxy. Unter verestertem Carboxy versteht man z.B. mit einem gegebenenfalls substituierten Alkohol, wie gegebenenfalls substituiertes Alkanol bzw. Cycloalkanol, z.B. Niederalkanol bzw. 4- bis 7-gliedriges Cycloalkanol, oder einem gegebenenfalls substituierten Phenol verestertes Carboxy, wie Niederalkoxycarbonyl, z.B. Ethoxycarbonyl, Cycloalkoxycarbonyl, z.B. Cyclohexyloxycarbonyl, oder Phenoxycarbonyl. Anhydridisiertes Carboxy ist beispielsweise ein symmetrisches oder gemischtes Anhydrid mit anorganischen Säuren, wie Halogenwasserstoffsäuren, oder mit organischen Carbonsäuren, wie gegebenenfalls substituierten Niederalkancarbonsäuren, beispielsweise Halogencarbonyl, z.B. Chlorcarbonyl, oder Niederalkanoyloxycarbonyl, z.B. Acetyloxycarbonyl. Amidiertes Carboxy weist als Aminogruppe beispielsweise eine freie oder mono- bzw. disubstituierte Aminogruppe auf. Unsubstituiertes Carbamoyl leitet sich von Ammoniak, mono- bzw. disubstituiertes Carbamoyl von primären bzw. sekundären Aminen ab. Als Beispiele für entsprechend amidiertes Carboxy kommen z.B. in Frage Carbamoyl, durch gegebenenfalls substituiertes Phenyl monosubstituiertes durch Niederalkyl mono- oder disubstituiertes oder durch 4- bis 7-gliedriges Alkylen bzw. Oxa-, Aza-, N-Niederalkylaza- oder Thiaalkylen disubstituiertes Carbamoyl, wie Niederalkylenamino-carbonyl, Morpholino- oder Thiomorpholino-carbonyl, oder durch gegebenenfalls Aryl aufweisendes Niederalkyl mono- oder disubstituiertes Carbamoyl, wie N-Mono- oder N-Diniederalkylcarbamoyl.

Orthoestergruppierungen sind beispielsweise Trialkoxymethyl-, wie Triniederalkoxymethylgruppen. Entsprechende Orthoanhydridgruppierungen sind beispielsweise Trihalogenmethylverbindungen.

Unter funktionell abgewandeltem Hydroxy $X_2$ bzw. $X_4$ ist beispielsweise eine Oxygruppe aufweisendes funktionell abgewandeltes Hydroxy, wie verestertes Hydroxy oder verethertes Hydroxy zu verstehen. Verestertes Hydroxy ist beispielsweise mit einer organischen Carbonsäure, wie Niederalkancarbonsäure, verestertes Hydroxy und bedeutet z.B. Niederalkanoyloxy, z.B. Acetyloxy. Verethertes Hydroxy ist beispielsweise Alkoxy, wie Niederalkoxy, z.B. Methoxy oder Ethoxy. $X_4$ bedeutet weiterhin mit einer Mineralsäure, wie Halogenwasserstoffsäure, oder mit einer Sulfonsäure, wie Niederalkan- oder gegebenenfalls substituierte Benzolsulfonsäure, verestertes Hydroxy, z.B. Halogen, Methan- oder p-Toluolsulfonyloxy.

Die Cyclisierung von Verbindungen der Formel (II) erfolgt in üblicher, insbesondere in der aus der Literatur für analoge Umsetzungen bekannten Weise. So arbeitet man erforderlichenfalls in Gegenwart eines katalytischen Mittels, wie eines sauren Mittels. Als solche eignen sich beispielsweise starke anorganische bzw. organische Protonsäuren, wie Mineralsäuren, z.B. Halogenwasserstoffsäuren, Schwefelsäure oder Polyphosphorsäure, Sulfonsäuren, wie Alkan- oder gegebenenfalls substituierte Benzolsulfonsäuren, z.B. Methan- oder p-Toluolsulfonsäuren, oder organische Carbonsäuren, wie Niederalkancarbonsäuren, z.B. Essig. Weiterhin können Lewis-Säuren für die Cyclisierung von Verbindungen der Formel (II) verwendet werden, insbesondere für die Cyclisierung von Verbindungen der Formel (II), worin $X_1$ Wasserstoff und

$X_2$ eine Gruppe der Formel $-O-CO-CH(R_1)-X_4$ bedeutet. Als Lewis-Säuren, d.h. Elektronenakzeptoren, werden z.B. Verbindungen von Elementen der dritten und fünften Hauptgruppe sowie der zweiten und achten Nebengruppe des Periodensystems verwendet. In Betracht kommen vor allem Halogenide des Bors, Aluminiums, Zinns, Antimons und Eisens, z.B. Bortrifluorid, Aluminiumchlorid, Zinn-(IV)-chlorid, Zinkchlorid und Eisen-(III)-chlorid, sowie Niederalkanoate des Thalliums, wie Thallium-(III)-acetat. Die Cyclisierung von Verbindungen der Formel (II) wird unter inerten Bedingungen, wie unter Inertgas, z.B. Stickstoff oder Argon, in Ab- oder Anwesenheit eines inerten Lösungsmittels und/oder unter Druck, z.B. in einer geschlossenen Apparatur, sowie bei einer geeigneten Reaktionstemperatur, z.B. bei etwa 0° bis etwa 250°C, durchgeführt. Lösungsmittel sind beispielsweise solche, die das bei der Reaktion entstehende Wasser binden, wie Anhydride, z.B. Acetanhydrid, oder mit deren Hilfe das Wasser aus dem Reaktionsgemisch entfernt werden kann, z.B. durch azeotrope Destillation, wie mit Hilfe von aromatischen Kohlenwasserstoffen, z.B. Toluol, Benzol oder Xylolen, ferner unpolare Lösungsmittel, wie Ether, z.B. Dioxan, halogenierte Alkene, z.B. Methylenchlorid oder Chloroform.

In einer bevorzugten Ausführungsform der Cyclisierung verwendet man Verbindungen der Formel (II), worin $X_1$ eine Gruppe der Formel $-CH(R_1)-X_3$ bedeutet, in der $X_3$ Carboxy ist, und $X_2$ Hydroxy bedeutet. Dabei genügt die spurenweise Verwendung von katalytisch wirkenden Säuren. Arbeitet man in Abwesenheit von entsprechenden Protonsäuren, so wird vorteilhaft das sich bei der Reaktion bildende Wasser, beispielsweise durch azeotrope Destillation, aus dem Reaktionsgemisch entfernt oder durch geeignete wasser-bindende Mittel, wie Alkancarbonsäureanhydride, z.B. Acetanhydrid, oder durch substituierte Diimide, wie Dicycloalkylcarbodiimid, z.B. Dicyclohexylcarbodiimid, gebunden.

In einer weiteren bevorzugten Ausführungsform cyclisiert man Verbindungen der Formel (II), worin $X_1$ eine Gruppe der Formel $-CH(R_1)-X_3$ und $X_3$ Carboxy oder verestertes bzw. amidiertes Carboxy bedeutet und $X_2$ mit einem Alkanol verethertes Hydroxy darstellt, durch Erwärmen mit Iodwasserstoffsäure oder Bromwasserstoffsäure und gegebenenfalls einem Niederalkancarbonsäureanhydrid, insbesondere Acetanhydrid, direkt und ohne

Isolierung von Zwischenprodukten zu den entsprechenden Verbindungen der Formel (I).

Die Ausgangsstoffe der Formel (II) bzw. deren Salze sind nach an sich bekannten Verfahren erhältlich. So geht man beispielsweise von Verbindungen der Formel (IIa)

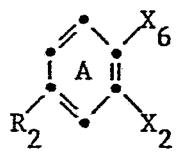

(IIa) ,

oder Salzen davon aus, worin $X_6$ eine Gruppe der Formel $R_1-CH_2-CO-$ bedeutet und setzt diese mit Ammoniumpolysulfid unter Druck bzw. mit Schwefel und einem primären oder sekundären Amin, vorteilhafterweise mit Morpholin oder Thiomorpholin, um. In einer so erhältlichen Verbindung der Formel (II), worin $X_1$ eine Gruppe der Formel $-CH(R_1)-X_3$, $R_1$ Wasserstoff und $X_3$ entsprechend substituiertes Thiocarbamoyl bzw. Carbamoyl oder Ammoniumcarboxylat bedeutet, wird $X_3$ solvolytisch, beispielsweise durch Hydrolyse in Carboxy oder insbesondere durch Alkoholyse in eine entsprechend veresterte Carboxygruppe $X_3$ übergeführt.

In einer fakultativen Zusatzumsetzung können Verbindungen der Formel (IIa), worin $X_6$ eine Gruppe der Formel $R_1-CH_2-CO-$ bedeutet, wobei $R_1$ Wasserstoff ist, in solche Verbindungen der Formel (IIa) übergeführt werden, worin $X_6$ eine Gruppe der Formel $R_1-CH_2-CO$ und $R_1$ einen aliphatischen Rest bedeutet. Dies geschieht üblicherweise durch Behandeln mit einem reaktionsfähigen veresterten aliphatischen Alkohol, wie einem Niederalkylhalogenid, in Gegenwart einer starken Base, wie eines Alkalimetallalkoholates, z.B. Natriummethylat. Verbindungen der Formel (II), worin $X_2$ reaktionsfähiges verestertes Hydroxy bedeutet und $X_1$ eine Gruppe der Formel $-CH(R_1)-X_3$ und $X_3$ funktionell abgewandeltes Carboxy, z.B. darstellt, werden vorteilhaft unter hydrolytischen Bedingungen in situ ohne Isolierung zu entsprechenden Verbindungen der Formel (I) umgesetzt. In Verbindungen der Formel (II), worin $X_2$ verethertes Hydroxy bedeutet, wird vorteilhaft die Ethergruppierung gespalten, z.B. durch Behandeln mit einer starken Säure, wie eine

Halogenwasserstoffsäure, z.B. Iodwasserstoffsäure, oder mit Pyridinhydrochlorid.

In einer weiteren, besonders bevorzugten Ausführungsform des Cyclisierungsverfahrens gelangt man zu Verbindungen der Formel (Ia), worin $R_1$ Methyl bedeutet und $R_a$, $R_b$ und $R_c$ jeweils Wasserstoff oder Niederalkyl bedeuten oder $R_a$ und $R_b$ gemeinsam 3- bzw. 4-gliedriges Alkylen darstellen und $R_c$ die vorstehende Bedeutung hat, indem man Verbindungen der Formel

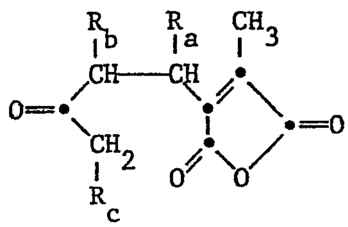

(IIb) ,

welche bekannt oder wie nachstehend beschrieben herstellbar sind, mit Aminen der Formel $R_2$-H (IIc) oder deren Säureadditionssalzen umsetzt. Dabei können z.B. intermediäre Verbindungen der Formel (II) gebildet werden, worin $X_1$ die Gruppe der Formel $-CH(CH_3)-COOH$ und $X_2$ Hydroxy bedeuten, die unter den Reaktionsbedingungen direkt zu den entsprechenden Verbindungen der Formel (Ia) cyclisieren.

Die Umsetzung erfolgt beispielsweise bei erhöhter Temperatur, z.B. in der Schmelze oder bei der Rückflusstemperatur des Lösungsmittels, z.B. in einem Temperaturbereich von etwa 80° C bis etwa 200° C. Geeignete inerte Lösungsmittel sind beispielsweise höher siedende Kohlenwasserstoffe, wie aromatische Kohlenwasserstoffe, z.B. Benzol, Toluol oder Xylole. Die Amine der Formel (IIc) werden insbesondere als Säureadditionssalze, z.B. vorteilhaft als Benzoate, eingesetzt.

Zur Herstellung von Verbindungen der Formel (IIb), worin $R_a$ Wasserstoff bedeutet, geht man von Verbindungen der Formel

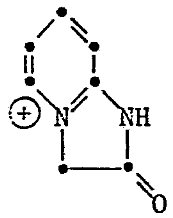

$A^{\ominus}$ (IId)

aus, die gegebenenfalls im aromatischen Teil substituiert sind und worin $A^{\ominus}$ das Anion einer anorganischen oder organischen Säure darstellt, und setzt diese mit Fumarsäure, Maleinsäure oder Maleinsäureanhydrid in Gegenwart einer Base um, wobei als Basen anorganische oder organische Basen in Frage kommen. Anorganische Basen sind beispielsweise Alkalimetallhydroxide oder -hydride, wie Natrium- oder Kaliumhydroxid oder Natrium- oder Kaliumhydrid. Als organische Amine werden beispielsweise tertiäre Amine, wie Trialkylamine, z.B. Triethylamine oder Tri-n-butylamine, oder cyclische Amine, wie Pyridin, Picolin, Chinolin oder Lutidin, verwendet.

Die auf diesem Wege zunächst erhältlichen freien Verbindungen werden durch Behandeln mit organischen oder anorganischen Säuren in die Verbindungen der Formel

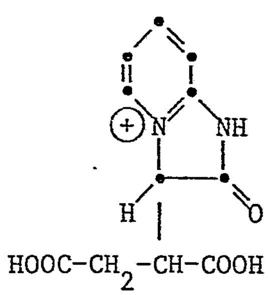

$A^{\ominus}$ (IIe) ,

übergeführt. Diese werden im weiteren Reaktionsverlauf, gegebenenfalls in Gegenwart einer der vorstehend genannten Basen, mit Verbindungen der Formel $R_a$-CH=C $(R_b)$-CO-$CH_2$-$R_c$ (IIf) zu Verbindungen der Formel

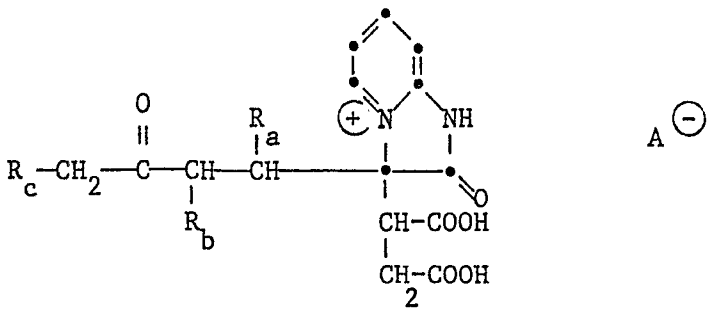

(IIg)

umgesetzt, die im nächsten Reaktionsschritt durch Erhitzen, z.B. auf Temperaturen zwischen 80 und 160° C, unter Decarboxylierung in Verbindungen der Formel

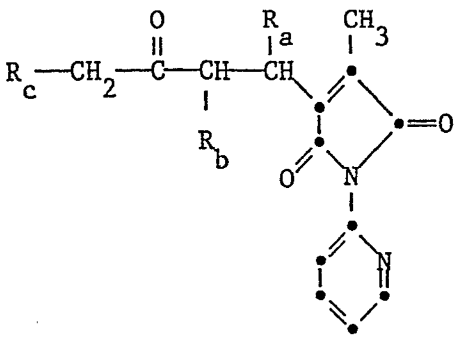

(IIh)

übergeführt werden. Die thermische Ueberführung von Verbindungen der Formel (IIg) in Verbindungen der Formel (IIh) wird beispielsweise in einem gegebenenfalls halogenierten aromatischen Lösungsmittel, wie Benzol, Toluol, einem Xylol oder Chlorbenzol, oder in einer Niederalkancarbonsäure, wie Eisessig, durchgeführt. Anschliessend werden die Verbindungen der Formel (IIh) zu Verbindungen der Formel (IIb) hydrolysiert. Die Hydrolyse wird beispielsweise in wässrigem oder wässrig-organischem Medium vorgenommen. Als organische Lösungsmittel eignen sich vor allem hochsiedene polare Lösungsmittel, wie Ether, z.B. Dioxan oder Tetrahydrofuran, N,N-Dialkylamide, z.B. N,N-Dimethylformamid oder N,N-Dimethylacetamid, oder cyclische Amide, wie N-Methylpyrrolidon. Die Hydrolyse erfolgt beispielsweise mit Hilfe einer anorganischen oder organischen Säure, wobei als anorganische Säuren Mineralsäuren, wie Halogenwasserstoffsäure oder Schwefelsäure, und als organische Säuren Sulfonsäuren, wie Niederalkan- oder gegebenenfalls substituierte Benzolsulfonsäuren, wie Methan- oder p-Toluolsulfonsäure, oder gegebenenfalls substituierte Alkancarbonsäuren, wie Eisessig, in Betracht kommen.

Zur Herstellung von Verbindungen der Formel (IIb), worin $R_a$ von Wasserstoff verschieden ist, geht man von Verbindungen der Formel (IId) aus und setzt diese zunächst mit Verbindungen der Formel (IIf) und anschliessend mit Fumarsäure, Maleinsäure oder insbesondere mit Maleinsäureanhydrid zu Verbindungen der Formel (IIg) um, die wiederum, wie vorstehend beschrieben, weiter zu den entsprechenden Verbindungen der Formel (IIb) weiterreagieren.

Zu Variante b): Die Ausgangsstoffe der Formel (III) können als Salze, insbesondere Säureadditionssalze, eingesetzt werden.

Eine in die Gruppe der Formel $>CH(R_1)$ überführbare Gruppe Y lässt sich beispielsweise reduktiv in die Gruppe $>CH(R_1)$ überführen.

Eine Verbindung der Formel III enthält beispielsweise als in $>CH(R_1)$ überführbare Gruppe Y eine Gruppe der Formel $>C(R_1)-COOH$.

Solche Verbindungen werden nach an sich bekannten Methoden zu Verbindungen der Formel (I) decarboxyliert. Die Decarboxylierung wird üblicherweise bei erhöhter Temperatur, z.B. in einem Temperaturbereich von etwa 50 bis 250° C, gegebenenfalls in Gegenwart eines katalytisch wirkenden Mittels, z.B. eines Edelmetalles, wie Kupferpulver, oder eines Amins, wie eines aromatischen Amins, z.B. Anilin oder Chinolin, und gegebenenfalls in einem inerten Lösungsmittel durchgeführt. Als inerte Lösungsmittel kommen beispielsweise hochsiedende gegebenenfalls halogenierte Kohlenwasserstoffe, wie halogenierte Aromaten, z.B. Chlorbenzol oder ein Xylol, in Frage.

Zur Herstellung von Ausgangsstoffen der Formel (III), worin Y eine Gruppe der Formel $>C(R_1)-COOH$ bedeutet, geht man von Verbindungen der Formel

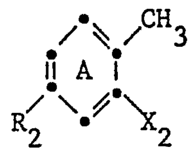

(IIIa) ,

worin $X_2$ Hydroxy oder funktionell abgewandeltes Hydroxy bedeutet, oder deren Salzen aus und halogeniert die Methylgruppe. Hierzu verwendet man beispielsweise N-Halogen-succinimid, wie die entsprechenden Brom- oder Chlor-Derivate, Sulfurylchlorid, Brom oder Chlor, wobei vorzugsweise in Gegenwart eines Radikalbildners, wie eines Peroxides, z.B. Benzoylperoxid, oder einer Azoverbindung, z.B. Azobisisobutyronitril,oder durch Energiezufuhr, wie Bestrahlen, z.B. mit UV-Licht,gearbeitet wird. Anschliessend wird das entsprechende Halogen durch Umsetzung mit einem Alkalimetallcyanid, wie Natrium- oder Kaliumcyanid, durch eine Cyanogruppe ausgetauscht. Das so erhaltene Acetonitril wird mit einem Dialkylcarbonat, wie Diethylcarbonat, in Gegenwart eines Alkalimetalls, wie Natrium, zu einer Verbindung der Formel

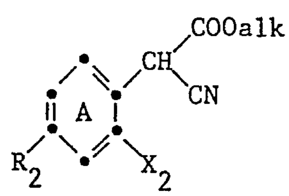

(IIIb) ,

worin alk einen Alkylrest bedeutet, umgesetzt. Im nächsten Reaktionsschritt kann, wenn erwünscht, der Rest $R_1$ durch Behandeln mit einem entsprechenden Halogenid oder Tosylat in Gegenwart einer starken Base, wie eines Alkalimetallalkoxids, z.B. Natriummethoxid, Kaliummethoxid oder Kalium-tert.-butoxid,oder eines Alkalimetallamids oder -hydrids, z.B. Natriumamid oder Kaliumhydrid, eingeführt werden. Die anschliessende Hydrolyse führt zu Verbindungen der Formel

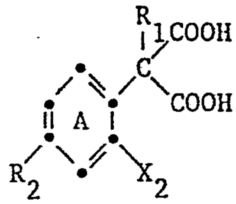

(IIIc) ,

die in Gegenwart einer Säure, beispielsweise einer Protonsäure, wie Mineralsäure, z.B. Halogenwasserstoffsäure oder Schwefelsäure, wie Alkan- oder gegebenenfalls substituierte Benzolsulfonsäure, z.B. p-Toluolsulfonsäure, oder wie Niederalkancarbonsäure, z.B. Essigsäure, oder einer Lewissäure, wie eines Halogenids von Elementen der dritten und fünften Hauptgruppe sowie der zweiten und achten Nebengruppe der Periodensystems, z.B. Aluminium- oder Eisen-(III)-chlorid, zu entsprechenden Verbindungen der Formel (III) cyclisiert werden.

In einer weiteren bevorzugten Ausführungsform kann man zu solchen Verbindungen der Formel (III) gelangen, worin Y die Gruppe $>C(R_1)-COOH$ darstellt, indem man von einer Verbindung der Formel (IIa) oder Salzen davon ausgeht, worin $X_6$ eine Gruppe der Formel $R_1-CH_2-CO-$ bedeutet und setzt diese mit Ammoniumpolysulfid unter Druck bzw. mit Schwefel und einem primären oder sekundären Amin, vorteilhafterweise mit Morpholin oder Thiomorpholin, um. In einer so erhältlichen Verbindung der Formel (II), worin $X_1$ eine Gruppe der Formel $-CH(R_1)-X_3$, $R_1$ Wasserstoff und $X_3$ entsprechend substituiertes Thiocarbamoyl bzw. Carbamoyl oder Ammoniumcarboxylat bedeutet, wird $X_3$ solvolytisch, beispielsweise durch Hydrolyse in Carboxy übergeführt.

In einer fakultativen Zusatzumsetzung können Verbindungen der Formel (IIa), worin $R_1$ Wasserstoff ist, in solche Verbindungen der Formel (IIa) übergeführt werden, worin $R_1$ einen aliphatischen Rest bedeutet. Dies geschieht üblicherweise durch Behandeln mit einem reaktionsfähigen veresterten aliphatischen Alkohol, wie einem Niederalkylhalogenid,

in Gegenwart einer starken Base, wie eines Alkalimetallalkoholates, z.B. Natriummethylat. In Verbindungen der Formel (II), worin $X_2$ verethertes Hydroxy bedeutet, wird vorteilhaft die Ethergruppierung gespalten, z.B. durch Behandeln mit einer starken Säure, wie eine Halogenwasserstoffsäure, z.B. Iodwasserstoffsäure, oder mit Pyridinhydrochlorid.

Die so erhältlichen Verbindungen der Formel

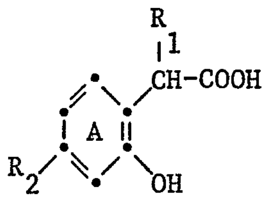

(IIIg)

werden mit Pivalinsäure-chlormethyl-ester in Gegenwart von Kaliumiodid in Dimethylformamid/Aceton behandelt. Hieraus resultiert eine entsprechende Verbindung der Formel (III) worin Y für die Gruppe der Formel $>C(R_1)-CH_2OH$ steht. Anschliessend kann die Oxidation der Hydroxymethylgruppe auf üblichem Weg zu Carboxy erfolgen, z.B. mit einer basischen Kaliumpermanganatlösung.

Die Gruppe Y kann weiterhin eine Gruppe der Formel $>C=R'_1$ darstellen, wobei $R'_1$ einen bivalenten aliphatischen Rest, z.B. Alkyliden, insbesondere Niederalkyliden, eine tautomere Form davon, wie Alkenylen, oder Alkenyliden, wie Niederalkenyliden, bedeutet. Entsprechende Verbindungen der Formel (III) können reduktiv in Verbindungen der Formel (I) überführt werden. Die Reduktion kann durch katalytische Hydrierung mit Wasserstoff, beispielsweise unter Schutzgas, wie Stickstoff, und in Gegenwart eines geeigneten Hydrierungskatalysators, oder durch Umsetzung mit gegebenenfalls komplexen Hydriden, wie Boran in Tetrahydrofuran oder wie Alkalimetallborhydriden zusammen mit Halogeniden von Elementen der dritten Hauptgruppe, z.B. mit Natriumborhydrid und Aluminiumchlorid bzw. Bortrifluorid in Diglyme, erfolgen. Als Hydrierungskatalysatoren kommen beispielsweise Elemente der achten Nebengruppe oder deren Derivate, wie Oxide oder Carbonate, in Frage, die gegebenenfalls

auf einem Trägermaterial, wie Erdalkalicarbonate, z.B. Bariumcarbonat, oder Aktivkohle, aufgezogen sind. Als Beispiele für solche Katalysatoren sind Raney-Nickel, Platinoxid oder Palladium/Kohle zu nennen. Als inerte Lösungs- bzw. Verdünnungsmittel kommen z.B. Ether, wie Dioxan oder Tetrahydrofuran, oder Alkohole, wie Niederalkanole, in Frage.

Die Hydrierung kann z.B. in einem Temperaturbereich von etwa -80° bis etwa 200°C erfolgen.

Zur Herstellung von Ausgangsstoffen der Formel (III), worin Y eine Gruppe der Formel $>C=R'_1$ bedeutet, geht man nach an sich bekannten Methoden vor. So werden beispielsweise Verbindungen der Formel (IIIc) in der Seitenkette $R_1$ halogeniert, z.B. mit Hilfe von Chlor oder Brom, N-Chlorsuccinimid oder N-Bromsuccinimid, gegebenenfalls in Gegenwart eines Radikalbildners, wie Benzoylperoxid oder Azobisisobutyronitril . Anschliessend wird auf üblichem Weg durch Decarboxylierung ein Aequivalent $CO_2$ abgespalten. Im nächsten Reaktionsschritt erfolgt eine Dehydrohalogenierung in Gegenwart einer Base, wie eines Alkalimetallalkoxids, z.B. Kalium-tert.-butoxid, zu den entsprechenden Verbindungen der Formel

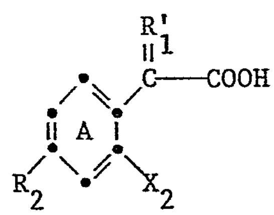

(IIId) ,

worin $X_2$ Hydroxy oder funktionell abgewandeltes Hydroxy bedeutet und welche beispielsweise in Gegenwart einer Säure, z.B. einer starken Proton- oder Lewissäure, cyclisiert werden. Protonsäuren sind beispielsweise Mineralsäuren, wie Halogenwasserstoffsäuren oder Schwefelsäure, Alkan- oder gegebenenfalls substituierte Benzolsulfonsäuren, z.B. p-Toluolsulfonsäure oder Alkancarbonsäuren, wie Eisessig. Als Lewissäuren eignen sich beispielsweise Halogenide des Bors, Aluminiums, Zinns, Antimons oder Eisens, wie Bortrifluorid oder Aluminiumchlorid. Die Cyclisierung wird vorzugsweise mit Iod- oder Bromwasserstoffsäure und Acetanhydrid bzw., sofern $X_2$ Hydroxy bedeutet, mit einem Carbodiimid, wie Dicyclohexylcarbodiimid, durchgeführt.

Weiterhin lassen sich beispielsweise solche Gruppen Y, worin Y eine Gruppe der Formel $>C(R_1)-X_{11}$ und $X_{11}$ Hydroxy, Alkylthio, Dialkylamino oder im Phenylteil jeweils gegebenenfalls substituiertes Diphenyl-sulfamoyl bedeutet oder worin Y für die Carbonylgruppe steht, reduktiv in die Gruppe der Formel $>CH(R_1)$ überführen.

Alkylthio $X_{11}$ ist z.B. Niederalkylthio, insbesondere Methyl oder Ethylthio, und Dialkylamino ist z.B. Diniederalkylamino, insbesondere Dimethylamino. Diphenyl-sulfamoyl kann jeweils im Phenylteil gegebenenfalls durch z.B. Halogen oder Niederalkyl substituiert sein, insbesondere wie Di-(p-bromphenyl)- oder Di-(p-toluol)-sulfamoyl.

Die Reduktion erfolgt in an sich bekannter Weise. So verwendet man ein geeignetes Reduktionsmittel und arbeitet unter inerten Bedingungen, wie gegebenenfalls unter Schutzgas, wie Stickstoff, in einem inerten Lösungs- oder Verdünnungsmittel, erforderlichenfalls unter Druck und/oder unter Kühlen oder Erwärmen. Als Lösungs- bzw. Verdünnungsmittel kommen z.B. Ether, wie Dioxan, oder Alkohole, wie Niederalkanole, in Frage. Die Umsetzung wird z.B. in einem Temperaturbereich von etwa -80° bis etwa 250°C durchgeführt.

Als Reduktionsmittel wird beispielsweise elementarer Wasserstoff, der durch einen Hydrierungskatalysator aktiviert wird, ferner ein gegebenenfalls komplexes Hydrid oder roter Phosphor in Gegenwart von Jodwasserstoff bzw. Jod verwendet. Geeignete Hydrierungskatalysatoren sind z.B. Elemente der VIII. Nebengruppe des Periodensystems oder ein Derivat, z.B. ein entsprechendes Oxid, davon. Solche Katalysatoren können auf einem Trägermaterial aufgezogen sein, z.B. auf Aktivkohle, einem Erdalkalimetallcarbonat oder -sulfat sowie auf einem Kieselgel. Beispiele für derartige Hydrierungskatalysatoren sind z.B. Platin, Platinoxid, Palladium, die gegebenenfalls auf Aktivkohle oder Bariumsulfat aufgezogen sind, oder Raney-Nickel. Als gegebenenfalls komplexe Hydride kommen beispielsweise Hydride von Elementen der 1. bis 3. Hauptgruppe oder daraus gebildete komplexe Hydride in Frage, wie Diboran, Aluminiumhydrid, Lithium-, Natriumborhydrid, Lithium- oder Natriumaluminiumhydrid, ferner andere zusammengesetzte Hydride, wie Lithium-triethylborhydrid.

In einer bevorzugten Ausführungsform des Verfahrens werden Hydroxy, Alkylthio, wie Niederalkylthio, insbesondere Methylthio, sowie Dialkylamino, wie Diniederalkylamino, insbesondere Dimethylamino, mit katalytisch aktiviertem elementarem Wasserstoff, reduziert, wobei als Hydrierungskatalysator z.B. Palladium/Kohle oder Raney-Nickel verwendet wird. Die Hydroxygruppe kann ferner durch roten Phosphor in Gegenwart von Iodwasserstoffsäure bzw. Iod unter Erwärmen, beispielsweise auf etwa 100° bis etwa 250°C, durch Wasserstoff ersetzt werden. In einer weiteren bevorzugten Verfahrensweise wird die gegebenenfalls im Phenylteil jeweils substituierte Di-phenyl-sulfamoylgruppe mit Hilfe eines geeigneten, gegebenenfalls komplexen Hydrides, z.B. mit Hilfe eines Alkalimetallborhydrids, unter Erwärmen, z.B. auf etwa 100° bis etwa 200°C, reduziert.

Die Carbonylgruppe Y wird vorzugsweise mit einem Hydrazin in Gegenwart einer Base, wie einem Alkalimetallhydroxid, analog der Wolff-Kishner-Reduktion bzw. der Huang-Minlon-Variante oder einem selektiven komplexen Hydrid, wie mit Natriumcyanoborhydrid in Gegenwart von p-Toluolsulfonylhydrazid, zu der Gruppe >CH($R_1$) reduziert, wobei $R_1$ für Wasserstoff steht.

Die Ausgangsverbindungen der Formel (III), worin Y die Gruppe der Formel >C($R_1$)-OH bedeutet, können beispielsweise erhalten werden, indem man eine Verbindung der Formel

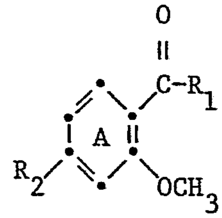

(IIIe)

mit Blausäure zum entsprechenden Cyanhydrin umsetzt.

Nach Hydrolyse der Cyanogruppe und Etherspaltung wird, vorzugsweise in situ, in Gegenwart einer Säure oder eines Dehydratisierungsmittels zur

entsprechenden Verbindung der Formel III cyclisiert.

Ausgangsverbindungen der Formel (III), worin Y die Gruppe der Formel $>C(R_1)-X_{11}$ und $X_{11}$ Alkylthio darstellt und $R_1$ Wasserstoff bedeutet, sind erhältlich, indem man von einer Verbindung der Formel (IIIe) ausgeht und diese mit einem Trihalogenmethan, wie Chloroform, in Gegenwart einer Base behandelt, durch Umsetzung mit einem Alkanthiol die Alkylthiogruppe einführt, anschliessend den Ether mit einer geeigneten Säure, z.B. einer starken Halogenwasserstoffsäure, spaltet und die Trihalogenmethylgruppe mit Hilfe einer Base, wie eines Alkalimetallhydroxids, zur Carboxygruppe hydrolysiert. Im letzten Schritt erfolgt die durch eine Säure, wie Protonsäure, katalysierte Cyclisierung zur entsprechenden Verbindung der Formel (III). In einer fakultativen Zusatzumsetzung können Verbindungen der Formel (I), worin $R_1$ Wasserstoff ist, in solche Verbindungen der Formel (I) übergeführt werden, worin $R_1$ einen aliphatischen Rest bedeutet. Dies geschieht üblicherweise durch Behandeln mit einem reaktionsfähigen veresterten aliphatischen Alkohol, wie einem Niederalkylhalogenid, in Gegenwart einer starken Base, wie eines Alkalimetallalkoholates, z.B. Natriummethylat.

Ebenfalls von einer Verbindung der Formel (IIIe) ausgehend kann man solche Verbindungen der Formel (III) erhalten, worin Y die Gruppe $>C(R_1)-X_{11}$ und $X_{11}$ gegebenenfalls Dialkylamino oder im Phenylteil jeweils gegebenenfalls substituiertes Di-phenyl-sulfamoyl bedeutet. Hierzu kann man beispielsweise eine Verbindung der Formel (IIIe) mit Natriumcyanid und Ammoniumcarbonat zum so erhältlichen Hydantoin umsetzen und dieses mit Hilfe einer Base, wie einem Alkalimetallhydroxid zur entsprechenden Aminosäure hydrolysieren. Anschliessend erfolgt die Ueberführung der freien Aminogruppe, z.B. durch Alkylierung mit einem Alkylhalogenid bzw. durch Acylierung mit dem entsprechenden Sulfonylhalogenid, in die gewünschte Gruppe $X_{11}$. Durch gleichzeitige Etherspaltung und Cyclisierung, z.B. mit Bromwasserstoffsäure und Acetanhydrid, kann man schliesslich zu den gewünschten Ausgangsverbindungen der Formel (III) gelangen.

Das Ausgangsmaterial der Formel (III), worin Y Carbonyl bedeutet, ist beispielsweise erhältlich durch Umsetzung einer Verbindung der Formel

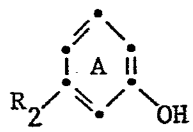

(IIIf)

mit Oxalylchlorid unter cyclisierenden Bedingungen, wie in Gegenwart einer starken Säure.

Zu Variante c): Die Ausgangsstoffe der Formel (IV) können in Form ihrer Salze, insbesondere Säureadditionssalze eingesetzt werden.

Ein in $R_2$ überführbarer Rest $X_5$ bedeutet beispielsweise Amino oder eine Gruppe der Formel $-NH-A_1-X_7$, wobei $A_1$ einen zweiwertigen Kohlenwasserstoffrest, wie Niederalkylen, Niederalkenylen, durch Aza, N-Niederalkylaza, Oxa oder Thia unterbrochenes Niederalkylen oder durch Aza, N-Niederalkylaza, Oxa oder Thia unterbrochenes Niederalkenylen, bedeutet und $X_7$ Hydroxy oder reaktionsfähiges verestertes Hydroxy darstellt. Reaktionsfähiges verestertes Hydroxy $X_7$ ist beispielsweise mit einer anorganischen Mineralsäure, wie Halogenwasserstoffsäure oder Schwefelsäure, mit einer organischen Sulfonsäure, wie Niederalkan- oder gegebenenfalls substituierte Benzolsulfonsäure, z.B. Methan- oder p-Toluolsulfonsäure, ferner mit einer organischen Carbonsäure, wie Niederalkancarbonsäure, z.B. Essigsäure, verestertes Hydroxy und bedeutet in erster Linie Halogen, wie Chlor oder Brom, Sulfonyloxy, wie p-Toluolsulfonyloxy.

Die Ueberführung von $-NH-A_1-X_7$ in $R_2$ erfolgt nach an sich bekannten Methoden. So arbeitet man beispielsweise in Gegenwart einer Base, wie eines Alkalimetall- oder Erdalkalimetallhydroxides bzw. -carbonats, z.B. Natriumhydroxid oder Kaliumhydrogencarbonat, unter inerten Bedingungen, wie unter Schutzgas, z.B. Stickstoff, und/oder in An- oder Abwesenheit eines inerten Lösungsmittels in einem Temperaturbereich von etwa 0° bis etwa 150° C. Als Lösungsmittel kommen z.B. Ether, wie Dioxan, Ketone, wie Aceton, Carbonsäuren, wie Eisessig, oder Amide, wie Dimethylformamid, in Frage.

Der Rest $R_2$ kann auch direkt eingeführt werden, durch Umsetzung von Verbindungen der Formel (IV), worin $X_5$ Amino darstellt, oder deren Salze in Gegenwart einer geeigneten Base mit Verbindungen der Formel $X_7-A_1-X_7$. Dabei können auch _in situ_ Verbindungen der Formel (IV), wor-

in $X_5$ eine Gruppe der Formel $-NH-A_1-X_7$ bedeutet, gebildet werden, die unter den Reaktionsbedingungen direkt zu entsprechenden Verbindungen der Formel (I) weiterreagieren.

Ein Rest $R_2$ kann, sofern dieser nicht-aromatischer Natur ist, weiterhin direkt eingeführt werden, indem man beispielsweise von Verbindungen der Formel (IV), worin $X_5$ Wasserstoff, einen Metall-aufweisenden Rest oder gegebenenfalls reaktionsfähiges verestertes Hydroxy bedeutet, oder deren Salzen ausgeht und diese mit Verbindungen der Formel $R_2-X_5'$ , worin $X_5'$ Wasserstoff, einen Metall-enthaltenden Rest oder gegebenenfalls reaktionsfähiges verestertes Hydroxy darstellt, oder deren Salzen umsetzt.

Ein Metall-aufweisender Rest ist beispielsweise ein Alkalimetallatom, wie Lithium oder Natrium. Reaktionsfähiges verestertes Hydroxy bedeutet beispielsweise mit einer Mineral-, wie Halogenwasserstoffsäure, oder einer Sulfonsäure, wie gegebenenfalls substituierte Benzolsulfonsäure, verestertes Hydroxy.

In erster Linie setzt man z.B. Verbindungen der Formel (IV) und $R_2-X_5'$ um, worin einer der Reste $X_5$ und $X_5'$ ein Alkalimetallatom, wie Lithium, und der andere Halogen, wie Brom, darstellt.

Für den Fall, dass $X_5$ Wasserstoff bedeutet und $X_5'$ für Hydroxy oder Halogen steht, wird die Umsetzung in Gegenwart einer Lewissäure durchgeführt. Bedeutet $X_5$ Halogen und $X_5'$ Wasserstoff, erfolgt die Reaktion in Gegenwart eines Kondensationsmittels.

Zur Herstellung von Ausgangsstoffen der Formel (IV) geht man beispielsweise von Verbindungen der Formel

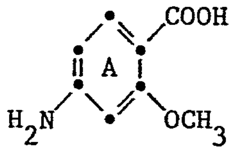

(IVb)

oder einem Salz davon aus und setzt diese beispielsweise mit einem Benzylhalogenid, wie Benzylchlorid, in Gegenwart einer Base, wie eines Alkalimetallhydroxides, z.B. Natriumhydroxid, und anschliessend mit verdünnter Säure, um. Die resultierende 4-N,N-Dibenzylamino-2-methoxy-benzoesäure wird mit Hilfe von Thionylchlorid in das entsprechende Säurechlorid überführt. Im nächsten Reaktionsschritt erfolgt die Umsetzung mit Diazomethan, wobei eine Verbindung der Formel

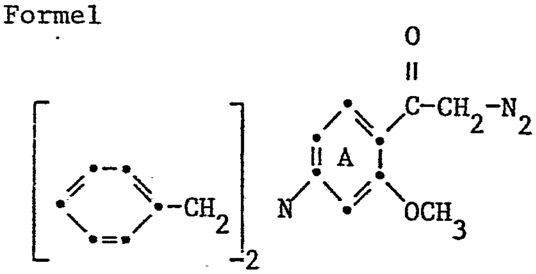

(IVc)

erhalten werden kann. Anschliessend wird die Verbindung der Formel (IVc) in Gegenwart von Silber oder Silberoxid bzw. durch UV-Bestrahlung solvolysiert, wobei die Hydrolyse zu der Verbindung der Formel

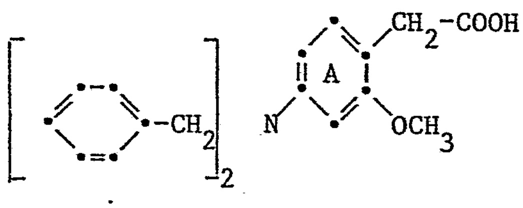

(IVd)

oder dessen Salz und die Alkoholyse zu dem entsprechenden Ester der Verbindung (IVd) führt.

Wenn erwünscht, kann in Verbindungen der Formel (IVd) der Rest $R_1$, wobei $R_1$ von Wasserstoff verschieden ist, beispielsweise durch Behandeln mit einem reaktionsfähigen veresterten aliphatischen Alkohol eingeführt werden. Die beiden Benzylgruppen werden anschliessend beispielsweise durch katalytische Hydrierung abgespalten. Das resultierende Reaktionsprodukt wird beispielsweise mit Hilfe von 48 %iger Bromwasserstoffsäure und Acetanhydrid zur Verbindung der Formel

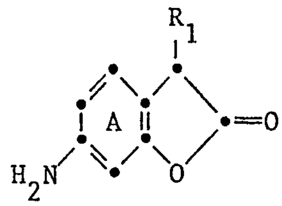

(IVe)

cyclisiert. Durch Umsetzung mit einer Verbindung der Formel $X_7-A_1-X_7$ in Gegenwart einer Base kann die Aminogruppe in die Gruppe $X_5$ übergeführt werden.

Verbindungen der Formel (I), worin $R_2$ Pyrrol-1-yl bedeutet, sind erhältlich, indem man Verbindungen der Formel (IV), worin $X_5$ Amino bedeutet, oder deren Salze mit 2-Buten-1,4-diol oder einem reaktionsfähig veresterten Derivat davon in Gegenwart einer Protonsäure, wie Niederalkancarbonsäure, zum Pyrrolidin-1-yl und dehydriert diesen in Gegenwart eines Dehydrierungskatalysators, wie eines Chinons, z.B. 2,3-Dichlor-5,6-dicyano-p-benzochinon oder Tetrachlor-p-benzochinon, oder eines Selenderivats, z.B. Selendioxid, oder eines Elements der VIII. Nebengruppe, z.B. Palladium, umsetzt oder indem man Verbindungen der Formel (IVe) oder deren Salze mit einem 2,5-Diniederalkoxy-tetrahydrofuran, wie 2,5-Dimethoxy-tetrahydrofuran, z.B. unter Erwärmen, behandelt.

Weiterhin kann der Pyrrolring $R_2$ aufgebaut werden, indem man beispielsweise die Aminogruppe $X_5$ in Verbindungen der Formel (IV), worin $X_5$ Amino bedeutet, oder deren Salzen mit einem gegebenenfalls reaktionsfähig veresterten Derivat von 1,3-Butadien-1,4-diol, z.B. mit 1,4-Dibrom-1,3-butadien, erforderlichenfalls unter Erwärmen und unter Schutzgas z.B. Stickstoff, und in einem inerten Lösungs- oder Verdünnungsmittel umsetzt.

Ferner kann der Pyrrolring $R_2$ analog Knorr-Paal durch Behandeln der Aminogruppe in Verbindungen der Formel (IV) mit gegebenenfalls acetalisiertem 1,4-Dioxo-butan aufgebaut werden, wobei unter inerten Bedingungen, beispielsweise unter Schutzgas und Erwärmen und in einem inerten Lösungsmittel gearbeitet werden kann.

Eine weitere Verfahrensvariante zum Aufbau des Pyrrolrings $R_2$ besteht z.B. in der Umsetzung von Verbindungen der Formel (IV), worin $X_5$ z.B. die Gruppe der Formel -NH-CH=CH-CH=CH-OH bzw. eine reaktionsfähige veresterte Form, ferner eine tautomere Form davon, die gegebenenalls acetalisiert ist,darstellt. Hierbei wird die Reaktion vorteilhaft unter inerten Bedingungen und unter Erwärmen durchgeführt.

Reaktionsfähiges verestertes Hydroxy bedeutet in diesem Zusammenhang jeweils z.B. mit einer Mineralsäure, wie Halogenwasserstoffsäure, z.B. Bromwasserstoffsäure, oder mit einer Sulfonsäure, wie Niederalkan- oder gegebenenfalls subsituierten Benzolsulfonsäure, p-Toluolsulfonsäure, verestertes Hydroxy.

Ebenso können genügend nucleophile Amine $R_2$-H direkt in solche Verbindungen der Formel (IV) eingeführt werden, worin $X_5$ für einen durch $R_2$ ersetzbaren Rest steht. Bedeutet beispielsweise $X_5$ Halogen, insbesondere Chlor, Brom oder Jod, kann die Umsetzung in An- oder Abwesenheit eines Lösungsmittels und je nach Wahl des Halogenatoms bei niedrigen Temperaturen bis zur Siedetemperatur des betreffenden Lösungsmittels erfolgen. Vorteilhaft befindet sich in der Nachbarposition von $X_5$ mindestens ein Substituent mit starken -I- oder -M-Effekt, wie Nitro, Halogen oder Trifluormethyl. In einzelnen Fällen ist es von Vorteil, die Umsetzung unter Druck oder bei erhöhter Temperatur durchzuführen. Vorteilhaft setzt man die Amine im Ueberschuss ein. Verwendet man z.B. Verbindungen der Formel (IV), worin $X_5$ für Wasserstoff steht, werden diese beispielsweise zunächst mit einem Oxidationsmittel, wie Blei (IV) acetat, z.B. in Gegenwart einer geeigneten Säure, wie Eisessig, und bei Raumtemperatur, behandelt und anschliessend mit entsprechenden Aminen $R_2$-H in einem inerten Lösungsmittel, wie einem Ether, z.B. Dioxan, unter Erwärmen, z.B. auf Rückflusstemperatur des betreffenden Lösungsmittels, umgesetzt.

Zu Variante d): Die Ausgangsstoffe der Formel (V) können in Form ihrer Salze, insbesondere als Säureadditionssalze, eingesetzt werden.

Eine in die Carbonylgruppe überführbare Gruppe Z ist beispielsweise die Methylengruppe oder stellt einen zur Carbonylgruppe hydrolysierbaren Rest dar.

Die Methylengruppe lässt sich beispielsweise oxidativ in die Carbonylgruppe überführen. Die Oxidation erfolgt in an sich bekannter Weise, beispielsweise mit Hilfe eines geeigneten Oxidationsmittels, unter inerten Bedingungen, z.B. in einem inerten Lösungs- oder Verdünnungsmittel und unter Kühlen oder Erwärmen.

Als Oxidationsmittel eignen sich beispielsweise Oxide von Elementen der VIII. Nebengruppe des Periodensystems, wie Osmiumtetroxid oder insbesondere Rutheniumtetroxid, sowie Hypochloride, wie Alkalimetall- oder Erdalkalimetallhypochlorite, z.B. Natrium- oder Calciumhypochlorit.

Als inerte Lösungs- bzw. Verdünnungsmittel kommen z.B. Wasser, Niederalkancarbonsäuren, wie Essigsäure, Ketone, wie Aceton, Ether, wie Dioxan oder Tetrahydrofuran, Amide, wie Dimethylformamid, oder Gemische derselben in Betracht.

Als hydrolytisch zur Carbonylgruppe hydrolysierbare Gruppen kommen beispielsweise Thiocarbonyl oder gegebenenfalls N-substituiertes Iminomethylen in Frage. Als Substituenten von Imino sind beispielsweise ein gegebenenfalls substituierter Kohlenwasserstoffrest, wie ein aliphatischer oder aromatischer Rest, z.B. Niederalkyl oder gegebenenfalls substituiertes Phenyl, oder eine von einer Carbonsäure oder einem Halbester der Kohlensäure abgeleitete Acylgruppe, wie Niederalkanoyl oder gegebenenfalls substituiertes Benzoyl, oder gegebenenfalls substituiertes Alkoxy-, wie Niederalkoxycarbonyl zu nennen.

Die Hydrolyse wird z.B. in An- oder Abwesenheit eines Lösungs- oder Verdünnungsmittels, wenn nötig, unter Kühlen oder Erwärmen und/oder unter Inertgas, z.B. Stickstoff, durchgeführt. Als Lösungs- bzw. Verdünnungsmittel kommen z.B. Wasser, Alkohole, wie Niederalkanole, Ketone, wie Aceton, Ether, wie Dioxan oder Tetrahydrofuran, Amide wie Dimethylformamid, oder Gemische derselben in Betracht.

Das Ausgangsmaterial der Formel (V), worin Z für Methylen steht, ist z.B. erhältlich, indem man eine Verbindung der Formel

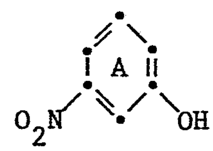

(Va)

mit einer Verbindung der Formel $R_1$-CO-$CH_2$-Cl (Vb) verethert und diesen Ether mit Hilfe von Titan (III)chlorid in einem Niederalkanol zur Verbindung der Formel

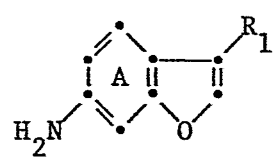

(Vc)

cyclisiert. Nach Ueberführung der freien Aminogruppe in $R_2$ beispielsweise durch übliche Alkylierung, wird die Doppelbindung des betreffenden Benzofurans durch Reduktion, z.B. mit Hilfe eines gegebenenfalls komplexen Hydrides, insbesondere mit Kaliumborhydrid, zur betreffenden Verbindung der Formel (V) hydriert.

Ausgangsmaterial der Formel (V), worin Z für Thiocarbonyl steht, ist beispielsweise zugänglich, indem man beispielsweise von Verbindungen der Formel (IIa) oder Salzen davon ausgeht, worin $X_6$ eine Gruppe der Formel $R_1$-$CH_2$-CO- bedeutet und diese mit Ammoniumpolysulfid unter Druck bzw. mit Schwefel und einem primären oder sekundären Amin, vorteilhafterweise mit Morpholin oder Thiomorpholin,

umsetzt. In einer so erhältlichen Verbindung der Formel (II), worin $X_1$ eine Gruppe der Formel $-CH(R_1)-X_3$, $R_1$ Wasserstoff und $X_3$ entsprechend substituiertes Thiocarbamoyl bzw. Carbamoyl oder Ammoniumcarboxylat bedeutet, wird $X_3$ solvolytisch, beispielsweise durch Hydrolyse in Carboxy übergeführt.

In einer fakultativen Zusatzumsetzung können Verbindungen der Formel (IIa), worin $X_6$ eine Gruppe der Formel $R_1-CH_2-CO$ und $R_1$ Wasserstoff ist, in solche Verbindungen der Formel (IIa) übergeführt werden, worin $X_6$ eine Gruppe der Formel $R_1-CH_2-CO$ und $R_1$ einen aliphatischen Rest bedeutet.

Dies geschieht üblicherweise durch Behandeln mit einem reaktionsfähigen veresterten aliphatischen Alkohol, wie einem Niederalkylhalogenid, in Gegenwart einer starken Base, wie eines Alkalimetallalkoholates, z.B. Natriummethylat. In so erhältlichen Verbindungen der Formel (II), worin $X_2$ verethertes Hydroxy bedeutet, wird vorteilhaft die Ethergruppierung gespalten, z.B. durch Behandeln mit einer starken Säure, wie eine Halogenwasserstoffsäure, z.B. Iodwasserstoffsäure, oder mit Pyridin-hydrochlorid. In so erhältlichen Verbindungen der Formel (IIIg) überführt man die Carboxygruppen in die Dithiocarboxygruppe beispielsweise über einen Ester mit einem Thiol und anschliessender Behandlung mit Phosphorpentasulfid. Im letzteren Schritt erfolgt die Cyclisierung zur entsprechenden Verbindung der Formel (V).

Zu Variante e): Die Ausgangsverbindungen der Formel (VI) können in Form ihrer Salze, insbesondere als Säureadditionssalze, vorliegen.

Ein in den Ring A überführbarer Ring A' enthält beispielsweise das Substitutionsmuster des Rings A und zwei Doppelbindungen sowie zusätzliche an zwei C-Atomen jeweils ein Wasserstoffatom. Entsprechend kann die Ueberführung in den Ring A durch Dehydrierung erfolgen.

Die Dehydrierung wird in an sich bekannter Weise durchgeführt. Dabei verwendet man ein geeignetes Dehydrierungsmittel, arbeitet erforderlichenfalls unter Erwärmen, z.B. in einem Temperaturintervall von etwa 100° bis etwa 300°C, in einem inerten Lösungs- oder Verdünnungsmittel, gegebenenfalls unter Schutzgas, wie Stickstoff, und/oder erforderlichenfalls unter Druck.

Als Dehydrierungsmittel kommen beispielsweise Nebengruppenelemente, vorzugsweise solche der VIII. Nebengruppen des Periodensystems, wie Palladium oder Platin, oder entsprechende Salze, wie Rutheniumtriphenyl-phosphid-chlorid, wobei die Mittel auf geeigneten Trägermaterialien, wie Aktivkohle, Aluminiumoxid oder einem Siliciumdioxid, aufgezogen sein können, in Betracht. Weitere bevorzugte Dehydrierungsmittel sind beispielsweise Chinone, wie p-Benzochinone, z.B. Tetrachlor-p-benzochinon oder 2,3-Dichlor-5,6-dicyano-p-benzochinon, oder wie Anthrachinone, z.B. Phenanthren-9,10-chinon. Ferner können N-Halogensuccinimide, wie N-Chlorsuccinimid, Manganverbindungen, wie Bariummanganat oder Mangandioxid, und Selenderivate, wie Selendioxid oder Diphenylselenium-bis-trifluoracetat, verwendet werden.

Das Ausgangsmaterial der Formel (VI), worin $R_1$ für Methyl steht, ist beispielsweise erhältlich,indem man von einer Verbindung der Formel (IIb) ausgeht und diese mit einem Säureadditionssalz einer Verbindung der Formel $R_2$-H umsetzt.

Eine erfindungsgemäss erhältliche Verbindung der Formel (I) kann in an sich bekannter Weise in eine andere Verbindung der Formel (I) umgewandelt werden.

Weist der aromatische Ring als Substituenten ein Wasserstoffatom auf, so kann dieses mit Hilfe eines Halogenierungsmittels in üblicher Weise durch ein Halogenatom ersetzt werden.

So erfolgt die Substitution von Wasserstoff durch Brom z.B. durch Bromierung mit Brom analog "Methoden der Organischen Chemie", Houben-Weyl (4. Edition), Vol.5/4, S. 233-249, in einem inerten Lösungsmittel. Weiterhin kann mit Hilfe folgender Bromierungsmittel bromiert werden: Hypobromsäure, Acylhypobromite oder andere organische Bromverbindungen, z.B. N-Bromsuccinimid, N-Bromacetamid, N-Bromphthalimid, Pyridiniumperbromid, Dioxandibromid, 1,3-Dibrom-5,5-dimethyl-hydantoin, 2,4,4,6-Tetrabrom-2,5-cyclohexadien-1-on.

Die entsprechende Chlorierung kann z.B. wie in Houben-Weyl (4. Edition), Vol.5/3, S. 651-673, beschrieben durchgeführt werden, vorteilhaft mit elementaren Chlor, z.B. in einem halogenierten Kohlenwasserstoff, wie Chloroform, und unter Kühlen, z.B. auf etwa -10° bis etwa +10°C.

Der Ersatz von Wasserstoff durch Jod kann z.B. mit elementarem Iod in Gegenwart von Quecksilberoxid oder Salpetriger Säure erfolgen. Anstelle von elementarem Jod lässt sich beispielsweise ein Alkalimetalliodid in Gegenwart eines Thalliumsalzes, z.B. Thallium(III)-trifluoracetat gemäss Tetrahedron Letters (1969), S. 2427 als Iodierungsmittel verwenden.

Ferner kann der Benzoteil des Ringsystems beispielsweise mit einem Niederalkanol bzw. einem Niederalkylhalogenid oder einem Phosphorsäureniederalkylester in Gegenwart von Lewis-Säuren alkyliert werden (Friedel-Crafts-Alkylierung). In einer Verbindung der Formel (I), worin der aromatische Ring A Brom enthält, kann beispielsweise das Brom durch Umsetzung mit einem Niederalkylbromid in Gegenwart eines Alkalimetalls durch Niederalkyl ersetzt werden.

Enthält der aromatische Ring A als Substituenten ein Wasserstoffatom, so kann diese in an sich bekannter Weise durch eine Acylgruppe ausgetauscht werden. So kann beispielsweise die Einführung des Acylgruppe analog der Friedel-Crafts-Acylierung (cf. G.A. Olah, Friedel-Crafts and Related Reactions, Vol.I, Interscience, New York, 1963-1965) durchgeführt werden, z.B. durch Umsetzung eines reaktiven funktionellen

Acylderivates, wie eines Halogenids oder Anhydrids, einer organischen Carbonsäure in Gegenwart einer Lewis-Säure, wie Aluminium-, Antimon (III)- oder (V)-, Eisen (III)-, Zink (II)-chlorid oder Bortrifluorid.

Enthält der aromatische Ring A als Substituenten Hydroxy, so lässt sich diese nach an sich bekannter Weise verethern. Die Umsetzung mit einer Alkoholkomponente, z.B. mit einem Niederalkanol, wie Ethanol, in Gegenwart von Säuren, z.B. Mineralsäure, wie Schwefelsäure, oder von Dehydratisierungsmitteln, wie Dicyclohexylcarbodiimid, führt zu Niederalkoxy. Umgekehrt kann man Ether in Phenole spalten, indem man die Etherspaltung mittels Säuren, wie Mineralsäuren, z.B. Halogenwasserstoffsäure, wie Bromwasserstoffsäure, oder wie Lewissäuren, z.B. Halogeniden von Elementen der 3. Hauptgruppe, wie Bortribromid, oder mittels Pyridin-hydrochlorid oder Thiophenol durchführt.

Weiter lässt sich Hydroxy in Niederalkanoyloxy umwandeln, beispielsweise durch Umsetzung mit einer gewünschten Niederalkancarbonsäure, wie Essigsäure, oder einem reaktionsfähigen Derivat davon, beispielsweise in Gegenwart einer Säure, wie eine Protonsäure, z.B. Chlor-, Bromwasserstoff-, Schwefel-, Phosphor- oder einer Benzolsulfonsäure, in Gegenwart einer Lewissäure, z.B. von Bortrifluorid-Etherat, oder in Gegenwart eines wasserbindenden Mittels, wie Dicyclohexylcarbodiimid. Umgekehrt kann verestertes Hydroxy, z.B. durch Basen-Katalyse, zu Hydroxy solvolysiert werden.

Falls der Ring A durch Niederalkylthio substituiert ist, kann man diesen auf übliche Weise zu entsprechendem Niederalkansulfinyl bzw. -sulfonyl oxidieren. Als geeignetes Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig- bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.
Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Uebergangsmetalloxide, wie Oxide von Elementen der VII. Nebengruppe, z.B. Vanadium-, Molybdän- oder Wolframoxid, zu nennen sind. Die Oxidation wird unter milden Bedingungen, z.B. bei Temperaturen von etwa -50° bis etwa +100°C, durchgeführt.
Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation des Niederalkylthio zum Niederalkansulfonyl. Jedoch setzt man hierbei üblicherweise das Oxidationsmittel im Ueberschuss ein.

Ist der Ring A der Formel I durch Niederalkylsulfinyl bzw. -sulfonyl substituiert, lassen sich diese nach an sich bekannten Methoden zu den entsprechenden Niederalkylthioverbindungen, von Niederalkansulfonylderivaten ausgehend, auch zu Niederalkan-sulfinyl reduzieren. Als Reduktionsmittel eignen sich beispielsweise katalytisch aktivierter Wasserstoff, wobei Edelmetalle bzw. Oxide, wie Palladium, Platin oder Rhodium bzw. deren Oxide, verwendet werden, gegebenenfalls auf geeignetem Trägermaterial, wie Aktivkohle oder Bariumsulfat, aufgezogen. Weiterhin kommen reduzierende Matallkationen, wie Zinn II-, Blei II-, Kupfer I-, Mangan II-, Titan II-, Vanadium II-, Molybdän III- oder Wolfram III-Verbindungen, Halogenwasserstoffe, wie Chlor-, Brom- oder Iodwasserstoff, Hydride, wie komplexe Metallhydride, z.B. Lithiumaluminium-, Natriumbor-, Tributylzinnhydrid, Phosphorverbindungen, wie Phosphorhalogenide, z.B. Phosphortrichlorid, -bromid, Phosphorpentachlorid oder Phosphoroxichlorid, Phosphine, wie Triphenylphosphin, oder Phosphorpentasulfid-Pyridin, oder Schwefelverbindungen, wie Mercaptane, Thiosäuren, wie Thiophosphorsäuren oder Dithiocarbonsäuren, Dithionit oder Schwefel-Sauerstoff-Komplexe, wie Iod-Pyridin-Schwefeldioxidkomplex, in Frage.

Enthalten die Verbindungen der Formel (I) ungesättigte Reste, wie Niederalkenyl oder Niederalkenylengruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Edelmetalle bzw. deren Derivate, z.B. Oxide, geeignet, wie Nickel, Raney-Nickel, Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at. und bei Temperaturen zwischen etwa -80° bis etwa 200°C, vor allem zwischen Raumtemperatur und etwa 100°C durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Umgekehrt können in cyclischen Systemen $R_2$ eine oder mehrere Mehrfachbindungen eingeführt werden. Hierzu verwendet man geeignete Dehydrierungsmittel, beispielsweise Nebengruppenelemente, vorzugsweise solche der VIII Nebengruppe des Periodensystems, z.B. Palladium oder Platin, oder entsprechende Edelmetallderivate, z.B. Ruthenium-triphenylphosphid-chlorid, wobei die Mittel auf geeigneten Trägermaterialien aufgezogen sein können. Weitere bevorzugte Dehydrierungsmittel sind beispielsweise Chinone, wie p-Benzochinone, z.B. Tetrachlor-p-benzochinon oder 2,3-Dichlor-5,6-dicyano-p-benzochinon, oder wie Anthrachinone, z.B. Phenanthren-9,10-chinon. Ferner können N-Halogensuccinimide, wie N-Chlorsuccinimid, Manganverbindungen, wie Bariummanganat oder Mangandioxid, und Selenderivate, wie Selendioxid oder Diphenylselenium-bis-trifluoracetat, verwendet werden.

Salze von Verbindungen der Formel (I) können in an sich bekannter Weise hergestellt werden. So erhält man beispielsweise Säureadditionssalze von Verbindungen der Formel (I) durch Behandeln mit einer Säure oder

einem geeigneten Ionenaustauscherreagenz. Salze können in üblicher Weise in die freien Verbindungen überführt werden, Säureadditionssalze z.B. durch Behandeln mit einem geeigneten basischen Mittel.

Infolge der engen Beziehung zwischen der neuen Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen Verbindungen einschliesslich ihrer Salze können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Ueberführung in diastereomere Salze oder Ester, z.B. durch Umsetzung eines sauren Endstoffes mit einer mit der racemischen Säure Salze bildenden optisch aktiven Base oder einer optisch aktiven Carbonsäure oder einem reaktiven Derivat davon, und Trennung des auf diese Weise erhaltenen Diastereomerengemisches, z.B.

auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, ihre Verwendung, z.B. als Arzneimittelwirkstoffe, Formulierungsverfahren und Verfahren zu ihrer Herstellung bilden ebenfalls einen Gegenstand der Erfindung.

Bei den erfindungsgemässen pharmazeutischen Präparaten, welche die erfindungsgemässe Verbindung oder pharmazeutisch verwendbare Salze davon enthalten, handelt es sich um solche zur topischen Anwendung, ferner zur enteralen, wie oralen oder rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand, sowie von der Applikationsweise ab.

Die neuen pharmazeutischen Präparate enthalten z.B. von etwa 10 % bis etwa 80 %, vorzugsweise von etwa 20 % bis etwa 60 % des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller

Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Regulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können

den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder supsendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Als topisch anwendbare pharmazeutische Präparate kommen in erster Linie Creme, Salben, Pasten, Schäume, Tinkturen und Lösungen in Frage, die von etwa 0,1 bis etwa 5 % des Wirkstoffs enthalten.

Creme sind Oel-in-Wasser Emulsionen die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachse oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitanfettsäureester (Tweens), ferner Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme verhindern, z.B. Polyalkohole, wie Glycerin, Sorbit, Propylenglykol und/oder Polyethylenglykole, ferner Konservierungsmittel, Riechstoffe etc.

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole bzw. Wollwachse enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe, etc.

Fettsalben sind wasserfrei und enthalten als Grundlage insbesondere Kohlenwasserstoff, z.B. Paraffin, Vaseline und/oder flüssige Paraffine, ferner natürliches oder partialsynthetisches Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden z.B. aus Druckbehältern verabreicht und sind in Aerosolform vorliegende flüssige Oel-in Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-ethanolische Grundlage auf, der u.a. Polyalkohole, z.B. Glycerin, Glykole, und/oder Polyethylenglykol, als Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Ethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei der Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Die Dosierung des Wirkstoffs hängt von der Warmblüter-Spezis, dem Alter und dem individuellen Zustand sowie der Applikationsweise ab. Im Normalfall ist für einen etwa 75 kg schweren Warmblüter bei oraler Applikation eine ungefähre Tagesdosis von etwa 100 bis etwa 600 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1: Ein Gemisch von 18,2 g (0,1 Mol) 4-Methyl-3-(3-oxobutyl)-maleinsäureanhydrid und 22 g (0,105 Mol) Morpholiniumbenzoat in 400 ml Benzol wird während 48 Stunden am Wasserabscheider unter Rückfluss erhitzt. Das Benzol wird im Vakuum entfernt, der Rückstand in Methylenchlorid aufgenommen und die organische Phase zweimal mit gesättigter Natriumbicarbonatlösung extrahiert. Das nach dem Trocknen und Entfernen des Methylenchlorids verbleibende Rohprodukt wird mit Petrolether/Ether an Kieselgel chromatographiert. Es werden blassgelbe Kristalle erhalten, die aus Methylenchlorid/Ether umkristallisiert werden. Man erhält so das 3-Methyl-6-morpholino-benzofuran-2(3H)-on vom Smp. 118-121°C.

Das Ausgangsmaterial kann wie folgt hergestellt werden:
Ein Gemisch von 341 g (2 Mol) des Hydrochlorids von Imidazo[1,2-a]-pyridin-2-(3H)-on in 700 ml Wasser wird unter Rühren portionenweise

mit einer heissen Lösung von 80 g (2 Mol) Natronlauge in 200 ml Wasser versetzt. Anschliessend wird eine Lösung von 250,7 g (2,16 Mol) Maleinsäure in 600 ml Wasser so zugetropft, dass die Innentemperatur des Reaktionsgemisches zwischen 40°C und 45°C bleibt. Nach 30 Stunden bei Raumtemperatur (20-25°C) wird auf 5°C gekühlt, der gebildete Niederschlag abfiltriert, das Filtrat im Vakuum auf etwa die Hälfte eingeengt und das ausgefallene Produkt abgenutscht. Die vereinigten Rückstände werden mit wenig kaltem Methanol gewaschen und bei 50°C im Vakuum getrocknet. Es resultieren 400 g 3-(1,2-Dicarboxyethyl)-imidazo[1,2-a]pyridin-2(3H)-on vom Smp. 193° (Zers.). Das erhaltene Produkt wird bei Raumtemperatur während 6 Stunden mit 650 ml konz. Salzsäure verrührt. Nach Kühlen auf 5°C wird der Niederschlag abfiltriert, das Filtrat im Vakuum auf etwa die Hälfte eingeengt und das ausgefallene Produkt abgenutscht. Die vereinigten Rückstände werden mit Aceton gewaschen und bei 50°C im Vakuum getrocknet.
Man erhält so das Hydrochlorid von 3-(1,2-Dicarboxyethyl)-imidazo[1,2-a]pyridin-2(3H)-on vom Smp. 205°C (Zers.).

Ein Gemisch von 114,7 g (0,4 Mol) des Hydrochlorids von 3-(1,2-Dicarboxyethyl)-imidazo[1,2-a]pyridin-2(3H)-on, 36,4 g (0,52 Mol) Methyl-vinylketon, 150 ml Methanol und 150 ml Wasser wird während 36 Stunden bei Raumtemperatur gerührt und anschliessend bei ca. 45°C im Vakuum zur Trockene eingedampft. Das erhaltene Rohprodukt wird in 300 ml Eisessig aufgenommen, mit 15 g Natriumacetat versetzt und bis zur beendeten $CO_2$-Entwicklung am Rückfluss gekocht. Anschliessend wird das Lösungsmittel im Vakuum entfernt, der Rückstand mit einem Gemisch von 150 ml 6M Schwefelsäure und 150 ml Tetrahydrofuran versetzt und während 8 Stunden bei 60°C gehalten. Nach dem Entfernen des Tetrahydrofurans im Vakuum wird das Reaktionsgemisch mit Wasser verdünnt, mit Methylenchlorid ausgezogen und über Kieselgel filtriert. Destillation des Rohproduktes im Hochvakuum (115°C-125°C/8 Pa) ergibt das 4-Methyl-3-(3-oxo-butyl)-maleinsäureanhydrid als spektroskopisch einheitliches blassgelbes Oel.

Beispiel 2: Ein Gemisch aus 18,2g(0,1 Mol) 4-Methyl-3-(3-oxobutyl)-maleinsäureanhydrid und 20,3 g (0,105 Mol) Pyrrolidiniumbenzoat in 400 ml Benzol wird während 24 Stunden am Wasserabscheider unter Rückfluss erhitzt. Das Benzol wird anschliessend im Vakuum entfernt und der verbleibende Rückstand zwischen Methylenchlorid und gesättigter Natriumbicarbonatlösung verteilt. Das nach dem Trocknen und Entfernen des Methylenchlorids verbleibende Rohprodukt wird mit Petrolether/Ether an Kieselgel chromatographiert. Anschliessende Umkristallisation aus Ether/Petrolether ergibt 3-Methyl-6-(pyrrolidin-1-yl)-benzofuran-2(3H)-on vom Smp 101-103°C.

Beispiel 3: Ein Gemisch aus 9,1 g (0,05 Mol) 4-Methyl-3-(3-oxobutyl)-maleinsäureanhydrid und 11 g (0,053 Mol) Piperidiniumbenzoat in 200 ml Benzol wird während 48 Stunden am Wasserabscheider unter Rückfluss erhitzt. Das Benzol wird im Vakuum entfernt und der verbleibende Rückstand zwischen Methylenchlorid und gesättigter Natriumbicarbonatlösung verteilt. Das nach dem Trocknen und Entfernen des Methylenchlorids verbleibende Rohprodukt wird mit Petrolether/Ether an Kieselgel chromatographiert. Anschliessende Umkristallisation aus Ether/Petrolether ergibt 3-Methyl-6-(piperidin-1-yl)-benzofuran-2(3H)-on vom Smp. 78-80°C.

Beispiel 4: Ein Gemisch aus 18,2 g (0,1 Mol) 4-Methyl-3-(3-oxobutyl)-maleinsäureanhydrid und 23,2 g (0,105 Mol) Hexahydroazepiniumbenzoat in 400 ml Benzol wird während 48 Stunden am Wasserabscheider unter Rückfluss erhitzt. Das Benzol wird im Vakuum entfernt und der verbleibende Rückstand zwischen Methylenchlorid und gesättigter Natriumbicarbonatlösung verteilt. Das nach dem Trocknen und Entfernen des Methylenchlorids verbleibende Rohprodukt wird mit Petrolether/Ether an Kieselgel chromatographiert. Anschliessende Umkristallisation aus Ether/Petrolether ergibt 6-(Hexahydroazepin-1-yl)-3-methyl-benzofuran-2(3H)-on vom Smp. 57-59°C.

Beispiel 5: Ein Gemisch von 19,6 g (0,1 Mol) 4-Methyl-3-(2-methyl-3-oxobutyl)-maleinsäureanhydrid und 21,3 g (0,11 Mol) Pyrrolidiniumbenzoat in 400 ml Benzol wird während 30 Stunden am Wasserabscheider unter Rückfluss erhitzt. Das Benzol wird im Vakuum entfernt und der Rückstand zwischen Ether und gesättigter Natriumbicarbonatlösung verteilt. Das nach dem Trocknen und Eindampfen der organischen Phase verbleibende Rohprodukt wird an Kieselgel chromatographiert. Elution mit Petrolether/Ether und anschliessende Umkristallisation der reinen Fraktionen aus Ether/Petrolether liefert das 3,5-Dimethyl-6-(pyrrolidin-1-yl)-benzofuran-2(3H)-on vom Smp. 67-69°C.

Das Ausgangsmaterial kann wie folgt hergestellt werden:

Ein Gemisch aus 172 g (0,6 Mol) Hydrochlorid von 3-(1,2-Dicarboxy-ethyl)-imidazo[1,2-a]pyridin-2(3H)-on, 65,5 g (0,78 Mol) 3-Methyl-3-buten-2-on, 220 ml Methanol und 220 ml Wasser wird während 36 Stunden bei Raumtemperatur gerührt und anschliessend bei ca. 45°C im Vakuum zur Trockene eingedampft. Das erhaltene Rohprodukt wird in 400 ml Eisessig aufgenommen, mit 22,5 g Natriumacetat versetzt und bis zur beendeten $CO_2$-Entwicklung am Rückfluss gekocht. Anschliessend wird das Lösungsmittel im Vakuum entfernt, der Rückstand mit einem Gemisch aus 225 ml 6 M Schwefelsäure und 225 ml Tetrahydrofuran versetzt und während 8 Stunden am Rückfluss erhitzt. Nach dem Entfernen von Tetrahydrofuran im Vakuum wird das Reaktionsgemisch mit Wasser verdünnt und mit Methylenchlorid ausgezogen. Das nach dem Trocknen und Eindampfen der organischen Phase verbleibende Rohprodukt wird mit Petrolether/Ether an Kieselgel chromatographiert. Die anschliessende Destillation (100°C/ $8.10^{-2}$ Torr) ergibt 4-Methyl-3-(2-methyl-3-oxobutyl)-maleinsäureanhydrid als blassgelbes Oel.

Beispiel 6: Ein Gemisch aus 19,6 g (0,1 Mol) 4-Methyl-3-(2-methyl-3-oxobutyl)-maleinsäureanhydrid und 23 g (0,11 Mol) Morpholiniumbenzoat in 400 ml Benzol wird während 60 Stunden am Wasserabscheider unter Rückfluss erhitzt. Das Benzol wird im Vakuum entfernt und der Rückstand zwischen Methylenchlorid und gesättigter Natriumbicarbonatlösung verteilt. Das nach dem Trocknen und Eindampfen der organischen Phase verbleibende Rohprodukt wird an Kieselgel chromatographiert. Elution mit Petrolether/Ether und anschliessende Umkristallisation der reinen Fraktionen aus Ether/Petrolether liefert das 3,5-Dimethyl-6-morpholino-benzofuran-2(3H)-on vom Smp. 108-109°C.

Beispiel 7: Ein Gemisch aus 19,6 g (0,1 Mol) 4-Methyl-3-(1-methyl-3-oxobutyl)-maleinsäureanhydrid und 23 g (0,11 Mol) Morpholiniumbenzoat in 400 ml Benzol wird während 44 Stunden am Wasserabscheider unter Rückfluss erhitzt. Das Benzol wird im Vakuum entfernt, der Rückstand in Methylenchlorid aufgenommen und die organische Phase zweimal mit gesättigter Natriumbicarbonatlösung extrahiert. Das nach dem Trocknen und Entfernen des Methylenchlorids verbleibende Rohprodukt wird mit Petrolether/Ether an Kieselgel chromatographiert.

Man erhält so nach Umkristallisation aus Ether/Petrolether das 3,4-Dimethyl-6-morpholino-benzofuran-2(3H)-on vom Smp. 95-96°C.

Das Ausgangsmaterial kann wie folgt hergestellt werden:

Zu einem Gemisch aus 51,2 g (0,3 Mol) Hydrochlorid von Imidazo[1,2-a]-pyridin-2(3H)-on in 120 ml Wasser werden unmittelbar nacheinander Lösungen von 12 g (0,3 Mol) Natronlauge in 90 ml Wasser und 32,7 g (0,39 Mol) frisch destilliertes 3-Penten-2-on in 210 ml Methanol gegeben. Nach Rühren bei Raumtemperatur während 30 Stunden wird bei ca. 45°C im Vakuum zur Trockene eingedampft, der Rückstand in 360 ml Eisessig aufgenommen und nach Zugabe von 32,4 g (0,33 Mol) Maleinsäureanhydrid und 12 g Natriumacetat bis zur beendeten $CO_2$-Entwicklung am Rückfluss gekocht. Das Lösungsmittel wird im Vakuum entfernt, das Rohprodukt in

einem Gemisch aus 180 ml 6 M Schwefelsäure und 180 ml Tetrahydrofuran aufgenommen und während 8 Stunden bei 60°C gehalten. Nach dem Entfernen von Tetrahydrofuran im Vakuum wird mit Wasser verdünnt und mit Methylenchlorid extrahiert. Die organische Phase wird getrocknet, eingedampft und der Rückstand mit Petrolether/Methylenchlorid an Kieselgel chromatographiert. Die anschliessende Destillation (100°C/$6 \cdot 10^{-2}$ Torr) ergibt 4-Methyl-3-(1-methyl-3-oxobutyl)-maleinsäureanhydrid als blassgelbes Oel.

Beispiel 8: Zu einem Gemisch aus 14,7 g (0,063 Mol) 3-Methyl-6-morpholino-benzofuran-2(3H)-on in 100 ml Chloroform wird bei 0-5°C unter Rühren eine kalte Lösung von Chlor in Chloroform getropft, bis im Dünnschichtchromatogramm kein Edukt mehr sichtbar ist. Das Reaktionsgemisch wird mit Methylenchlorid verdünnt und nacheinander mit 10%iger Natriumthiosulfatlösung, verdünnter Natriumbicarbonatlösung und Wasser gewaschen. Das nach dem Trocknen und Eindampfen der organischen Phase verbleibende Rohprodukt wird mit Petrolether/Ether an Kieselgel chromatographiert. Nach Umkristallisation der reinen Fraktionen aus Ether/Petrolether erhält man das 5-Chlor-3-methyl-6-morpholino-benzofuran-2(3H)-on vom Smp. 103-105°C.

Beispiel 9: In analoger Weise wie in Beispiel 8 beschrieben erhält man ausgehend von 3-Methyl-6-(piperidin-1-yl)-benzofuran-2(3H)-on und Chlor das 5-Chlor-3-methyl-6-(piperidin-1-yl)-benzofuran-2(3H)-on vom Smp. 112-113°C.

Beispiel 10: In analoger Weise wie in Beispiel 8 beschrieben erhält man ausgehend von 3-Methyl-6-(pyrrolidin-1-yl)-benzofuran-2(3H)-on und Chlor das 5-Chlor-3-methyl-6-(pyrrolidin-1-yl)-benzofuran-2(3H)-on vom Smp. 60-62°C.

Beispiel 11: Zu einem Gemisch aus 15 g (0,064 Mol) 3-Methyl-6-morpholino-benzofuran-2(3H)-on in 120 ml Chloroform wird bei 0-5°C unter Rühren während einer Stunde eine Lösung von 11 g (0,069 Mol) Brom in 50 ml Chloroform getropft. Anschliessend wird während 30 Minuten bei Raumtemperatur nachgerührt. Das Reaktionsgemisch wird mit Methylenchlorid versetzt und nacheinander mit 10%iger Natriumthiosulfatlösung, verdünnter Natriumbicarbonatlösung und Wasser gewaschen. Das nach dem Trocknen und Eindampfen der organischen Phase verbleibende Rohprodukt wird mit Petrolether/Ether an Kieselgel chromatographiert. Nach Umkristallisation der reinen Fraktionen aus Ether/Petrolether erhält man das 5-Brom-3-methyl-6-morpholino-benzofuran-2(3H)-on vom Smp. 99-100°C.

Beispiel 12: In analoger Weise wie in Beispiel 11 beschrieben erhält man ausgehend von 3-Methyl-6-(pyrrolidin-1-yl)-benzofuran-2(3H)-on und Brom das 5-Brom-3-methyl-6-(pyrrolidin-1-yl)-benzofuran-2(3H)-on vom Smp. 73-75°C.

Beispiel 13: In analoger Weise wie in Beispiel 11 beschrieben erhält man ausgehend von 3,4-Dimethyl-6-morpholino-benzofuran-2(3H)-on und Brom das 5-Brom-3,4-dimethyl-6-morpholino-benzofuran-2(3H)-on vom Smp. 146-149°C (Zers.).

Beispiel 14: Eine Lösung von 8,1 g (30 mMol) roher 5-Chlor-2-hydroxy-4-(piperidin-1-yl)-phenylessigsäure in 50 ml abs. Methylenchlorid wird bei Raumtemperatur während ca. 3 Minuten mit einer Lösung von 6,5 g (31,5 mMol) Dicyclohexylcarbodiimid in 40 ml abs. Methylenchlorid versetzt. Das Reaktionsgemisch wird während 30 Minuten bei Raumtemperatur gerührt. Der ausgefallene Dicyclohexylharnstoff wird abgenutscht und mit Methylenchlorid gewaschen. Das Filtrat wird am Vakuumrotationsverdampfer eingedampft und der Rückstand an Kieselgel mit dem Laufmittel Methylenchlorid/Hexan (1:1) chromatographiert. Umkristallisation aus Hexan ergibt 5-Chlor-6-(piperidin-1-yl)-benzofuran-2(3H)-on vom Smp. 129-131°C.

In analoger Weise erhält man das 6-(4-Morpholino)-benzofuran-2(3H)-on vom Smp. 153-155°C.

Das Ausgangsmaterial kann wie folgt hergestellt werden:

Eine Lösung von 96 g (0,72 Mol) Aluminiumtrichlorid in 180 ml abs. Nitromethan wird unter Stickstoff-Atmosphäre und Eis/Methanol-Kühlung zu einem Gemisch von 106,2 g (0,60 Mol) 3,4-Dichloranisol [H. Jamarlik et al, Comptes Rendus Acad. Sci. Ser. C 273 (25), 1756 (1971)] und 51,1 ml (0,72 Mol) Acetylchlorid während ca. 30 Minuten zugetropft, dass der Innentemperaturbereich zwischen 0 und 5° liegt. Anschliessend wird noch 1 Stunde bei etwa 4 bis 6° weitergerührt, dann auf Eis gegossen und mit Methylenchlorid extrahiert. Die organischen Extrakte werden mit Wasser gewaschen, vereinigt, über Natriumsulfat getrocknet und am Vakuumrotationsverdampfer eingedampft. Nach Umkristallisation aus Methanol/Wasser erhält man das 4,5-Dichlor-2-methoxy-acetophenon vom Smp. 93-95°C.

Eine Lösung von 76,7 g (0,35 Mol) 4,5-Dichlor-2-methoxy-acetophenon in 750 ml Piperidin wird während 7 Stunden bei 170° im Autoklaven gehalten. Das Reaktionsgemisch wird eingedampft, in Essigester aufgenommen und mit Wasser gewaschen. Die Essigester-Extrakte werden vereinigt, über Natriumsulfat getrocknet und am Vakuumrotationsverdampfer eingedampft. Der Rückstand wird an Kieselgel mit Methylenchlorid chromatographiert. Man erhält so das 5-Chlor-2-hydroxy-4-(piperidin-1-yl)-acetophenon vom Smp. 68-70°C.

In analoger Weise erhält man das 5-Chlor-2-hydroxy-4-(4-morpholino)-acetophenon vom Smp. 102-103°C.

Eine Lösung von 32,5 g (128 mMol) 5-Chlor-2-hydroxy-4-(N-piperidino)-acetophenon mit 75 ml (166 mMol) einer etwa 40%igen methanolischen Lösung von Benzyltriethylammoniumhydroxid (Triton B) in 65 ml Tetrahydrofuran wird auf 0° abgekühlt. Während ca. 6 Minuten werden 14,6 ml (154 mMol) Dimethylsulfat so zugetropft, dass die Innentemperatur

nicht über 5° steigt. Das Reaktionsgemisch wird noch 1 Stunde bei 0° gerührt, dann während etwa 30 Minuten unter Rückfluss gekocht. Das Reaktionsgemisch wird nun auf 400 ml Wasser gegossen und mit Essigester extrahiert. Die vereinigten Essigesterphasen werden mit Wasser gewaschen, über Natriumsulfat getrocknet und am Vakuumrotationsverdampfer eingedampft. Der Rückstand wird aus Methylenchlorid/Hexan umkristallisiert, und man erhält das 5-Chlor-2-methoxy-4-(piperidin-1-yl)-acetophenon vom Smp. 119-120°.

In analoger Weise erhält man das 5-Chlor-2-methoxy-4-(4-morpholino)-acetophenon vom Smp. 143-145°.

Eine Lösung von 18,2 g (68 mMol) 5-Chlor-2-methoxy-4-(piperidin-1-yl)-acetophenon und 4,36 g (136 mMol) Schwefel in 68 ml Morpholin wird während 5 Stunden bei 90° gehalten. Das Reaktionsgemisch wird abgekühlt, mit Essigester verdünnt und mit Wasser gewaschen. Die vereinigten Essigester-Extrakte werden über Natriumsulfat getrocknet und am Vakuumrotationsverdampfer eingedampft. Nach Umkristallisation aus Methylenchlorid/Methanol erhält man das 5-Chlor-2-methoxy-4-(piperidin-1-yl)-phenylthioessigsäuremorpholinamid vom Smp. 137-139°.

In analoger Weise erhält man das 5-Chlor-2-methoxy-4-(4-morpholino)-phenylthioessigsäuremorpholinamid vom Smp. 160-162,5°.

Eine Lösung von 11,07 g (30 mMol) 5-Chlor-2-methoxy-4-(piperidin-1-yl)-phenylthioessigsäuremorpholinamid in 120 ml Eisessig und 30 ml konz. Salzsäure wird während 22 Stunden unter Rückfluss gekocht. Das Reaktionsgemisch wird abgekühlt, mit Wasser verdünnt und mit Methylenchlorid extrahiert. Die vereinigten Methylenchloridphasen werden mit Wasser gewaschen, über Natriumsulfat getrocknet und am Hochvakuumrotationsverdampfer eingedampft. Nach Chromatographieren an Kieselgel mit Chloroform/Methanol (19:1) erhält man die 5-Chlor-2-methoxy-4-(piperidin-1-yl)-phenylessigsäure, die nach Umkristallisation mit Methylenchlorid/Hexan bei 120-122° schmilzt.

In analoger Weise erhält man die 5-Chlor-2-methoxy-4-(4-morpholino)-phenylessigsäure vom Smp. 141-143°.

Eine Lösung von 8,5 g (30 mMol) 5-Chlor-2-methoxy-4-(piperidin-1-yl)-phenylessigsäure in 150 ml 48%iger Bromwasserstoffsäure wird während 15 Stunden unter Rückfluss gekocht. Das Reaktionsgemisch wird abgekühlt, mit Wasser verdünnt und mit gesättigter Natriumbicarbonat-Lösung auf pH 3-4 gestellt. Anschliessend wird mit Essigester extrahiert, die vereinigten organischen Phasen mit Wasser gewaschen, über Natriumsulfat getrocknet und am Hochvakuumrotationsverdampfer eingedampft. Man erhält so einen dunkelgrauen Schaum von 5-Chlor-2-hydroxy-4-(piperidin-1-yl)-phenylessigsäure, der ohne Reinigung zum 5-Chlor-6-(piperidin-1-yl)-benzofuran-2(3H)-on umgesetzt wird.

Analog erhält man die 2-Hydroxy-4-(4-morpholino)-phenylessigsäure.

Beispiel 15: Zu einer Lösung von 6,7 g (0,035 Mol) 6-Amino-5-chlor-3-methyl-benzofuran-2(3H)-on in 100 ml Dioxan werden 7 ml (0,045 Mol) 2,5-Dimethoxy-tetrahydrofuran und 5 ml 5 N HCl gegeben. Nach 1 Stunde wird die gebildete wässrige Phase abgetrennt und die obere Dioxan-haltige Phase im Vakuum zur Trockne eingedampft. Der dunkelbraune Rückstand wird mit Methylenchlorid über Kieselgel filtriert. Nach dem Verdampfen des Methylenchlorids erhält man das 5-Chlor-3-methyl-6-(pyrrol-1-yl)benzofuran-2-(3H)-on als farblose Prismen vom Smp. 100-101°.

Das Ausgangsmaterial kann wie folgt hergestellt werden:

In analoger Weise wie in Beispiel 1 beschrieben erhält man ausgehend vom 4-Methyl-3-(3-oxobutyl)-maleinsäureanhydrid und Dibenzylammoniumbenzoat das 6-Dibenzylamino-3-methyl-benzofuran-2(3H)-on als farbloses Oel.

In analoger Weise wie in Beispiel 8 beschrieben erhält man ausgehend von 6-Dibenzylamino-3-methyl-benzofuran-2(3H)-on und Chlor das 5-Chlor-6-dibenzylamino-3-methyl-benzofuran-2(3H)-on vom Smp. 111-112°.

Eine Lösung von 21,8 g (0,058 Mol) 5-Chlor-6-dibenzylamino-3-methyl-benzofuran-2(3H)-on in 220 ml Dioxan wird mit 2,0 g Palladiumkohle (5 %ig) bei Raumtemperatur mit Wasserstoff ohne Ueberdruck reduziert. Nach Aufnahme der theoretischen Menge Wasserstoff wird vom Katalysator abfiltriert und das Dioxan im Vakuum abgedampft. Der Rückstand wird mit 20 ml kaltem Methanol versetzt und der Niederschlag abfiltriert. Es wird 6-Amino-5-chlor-3-methyl-benzofuran-2(3H)-on als farblose Kristalle vom Smp. 139-140° erhalten.

Beispiel 16: Ein Gemisch aus 15,7 g (0,08 Mol) 4-Methyl-3-(2-methyl-3-oxobutyl)-maleinsäureanhydrid und 20,3 g (0,092 Mol) Hexahydroazepiniumbenzoat in 400 ml Benzol wird während 48 Stunden am Wasserabscheider unter Rückfluss erhitzt. Nach Zugabe von nochmals 3,3 g (15 mMol) Hexahydroazepiniumbenzoat wird das Reaktionsgemisch während weiterer 24 Stunden unter Rückfluss am Wasserabscheider belassen. Anschliessend wird das Benzol im Vakuum entfernt und der Rückstand zwischen Methylenchlorid und gesättigter Natriumbicarbonalösung verteilt. Das nach dem Trocknen und Entfernen des Methylenchlorids verbleibende Rohprodukt wird mit Petrolether/Ether an Kieselgel chromatographiert. Anschliessende Destillation im Kugelrohr (150°C/6.10$^{-2}$ Torr) ergibt ein blassgelbes Oel, welches beim Stehen kristallisiert. Durch Umkristallisation aus Petrolether wird 3,5-Dimethyl-6-(hexahydroazepin-1-yl)-benzofuran-2(3H)-on vom Smp. 58-60°C erhalten.

Beispiel 17: Ein Gemisch aus 19,6 g (0,1 Mol) 4-Methyl-3-(2-methyl-3-oxobutyl)-maleinsäureanhydrid und 21,7 g (0,105 Mol) Piperidiniumbenzoat in 400 ml Benzol wird während 48 Stunden am Wasserabscheider unter Rückfluss erhitzt. Das Benzol wird im Vakuum entfernt und der Rückstand zwischen Methylenchlorid und gesättigter Natriumbicarbonatlösung verteilt. Das nach dem Trocknen und Entfernen des Methylenchlorids verbleibende Rohprodukt wird mit Petrolether/Ether an Kieselgel chromatographiert. Anschliessende Umkristallisation aus Petrolether ergibt 3,5-Dimethyl-6-(piperidin-1-yl)-benzofuran-2(3H)-on vom Smp. 61-63°C.

Beispiel 18: Ein Gemisch aus 12,6 g (0,06 Mol) 3-(2-Ethyl-3-oxobutyl)-4-methyl-maleinsäureanhydrid und 12,2 g (0,063 Mol) Pyrrolidiniumbenzoat in 200 ml Benzol wird während 24 Stunden am Wasserabscheider unter Rückfluss erhitzt. Das Benzol wird anschliessend im Vakuum entfernt und der verbleibende Rückstand zwischen Methylenchlorid und gesättigter Natriumbicarbonatlösung verteilt. Das nach dem Trocknen und Entfernen des Methylenchlorids verbleibende Rohprodukt wird mit Petrolether/Ether an Kieselgel chromatographiert. Anschliessende Destillation im Kugelrohr (140°C/6.$10^{-2}$ Torr) ergibt 5-Ethyl-3-methyl-6-(pyrrolidin-1-yl)-benzofuran-2(3H)-on als blassgelbes Oel.
Das Ausgangsmaterial kann wie folgt hergestellt werden:
Ein Gemisch aus 86 g (0,3 Mol) Hydrochlorid von 3-(1,2-Dicarboxyethyl)-imidazo[1,2-a]pyridin-2(3H)-on, 41,2 g (0,42 Mol) 3-Ethyl-3-buten-2-on, 110 ml Methanol und 110 ml Wasser wird während 36 Stunden bei Raumtemperatur gerührt und anschliessend bei ca. 45°C im Vakuum zur Trockene eingedampft. Das erhaltene Rohprodukt wird in 225 ml Eisessig aufgenommen, mit 11 g Natriumacetat versetzt und bis zur beendeten $CO_2$-Entwicklung am Rückfluss gekocht. Anschliessend wird das Lösungsmittel im Vakuum entfernt, der Rückstand, mit einem Gemisch aus 110 ml 6 M Schwefelsäure und 110 ml Tetrahydrofuran versetzt und während 8 Stunden am Rückfluss erhitzt. Nach dem Entfernen von Tetrahydrofuran im Vakuum wird das Reaktionsgemisch mit Wasser verdünnt und mit Methylenchlorid extrahiert. Das nach dem Trocknen und Eindampfen der organischen Phase verbleibende Rohprodukt wird mit Methylenchlorid an Kieselgel chromatographiert. Die anschliessende Destillation (110-120°C/$10^{-1}$Torr) ergibt 3-(2-Ethyl-3-oxobutyl)-4-methyl-maleinsäureanhydrid als blassgelbes Oel.

Beispiel 19: Ein Gemisch aus 15,3 g (0,065 Mol) 4-Methyl-3-(1,2-tetramethylen-3-oxobutyl)-maleinsäureanhydrid und 13,5 g (0,07 Mol) Pyrrolidiniumbenzoat in 400 ml Benzol wird während 60 Stunden am Wasserabscheider unter Rückfluss erhitzt. Das Benzol wird im Vakuum entfernt und der Rückstand zwischen Methylenchlorid und gesättigter Natriumbicarbonatlösung verteilt. Das nach dem Trocknen und Entfernen des Methylenchlorids verbleibende Rohprodukt wird mit Petrolether/Ether an Kieselgel chromatographiert. Anschliessende Umkristallisation aus Ether/Petrolether ergibt 3-Methyl-6-(pyrrolidin-1-yl)-4,5-tetramethylen-benzofuran-2(3H)-on vom Smp. 99-101° C.

Das Ausgangsmaterial kann wie folgt hergestellt werden:

Zu einem Gemisch aus 51,2 g (0,3 Mol) Hydrochlorid von Imidazo[1,2-a]-pyridin-2(3H)-on in 120 ml Wasser werden unmittelbar nacheinander Lösungen von 13,2 g (0,33 Mol) Natronlauge in 90 ml Wasser und 48,4 g (0,39 Mol) 1-Acetylcyclohexen in 210 ml Methanol gegeben. Nach Rühren bei Raumtemperatur während 24 Stunden wird bei ca. 45°C im Vakuum zur Trockene eingedampft, der Rückstand in 240 ml Eisessig aufgenommen und nach Zugabe von 29,4 g(0,3 Mol) Maleinsäureanhydrid und 7,5 g Natriumacetat bis zur beendeten $CO_2$-Entwicklung am Rückfluss gekocht. Das Lösungsmittel wird im Vakuum abdestilliert, das Rohprodukt in einem Gemisch aus 180 ml 6 M Schwefelsäure und 180 ml Tetrahydrofuran aufgenommen und während 8 Stunden am Rückfluss erhitzt. Nach dem Entfernen von Tetrahydrofuran im Vakuum wird mit Wasser verdünnt und mit Methylenchlorid extrahiert. Die organische Phase wird getrocknet, eingedampft und der Rückstand mit Petrolether/Methylenchlorid an Kieselgel chromatographiert. Die anschliessende Destillation (110-115°C/ $10^{-1}$ Torr) ergibt 4-Methyl-3-(1,2-tetramethylen-3-oxobutyl)-maleinsäureanhydrid als blassgelbes Oel.

Beispiel 20: Zu einer Lösung von 18 g (0,1 Mol) 6-Amino-3,5-dimethylbenzofuran-2(3H)-on in 200 ml Essigsäure (96%ig) werden unter Rühren bei 104 - 106° innert 5 Minuten 18 g (0,13 Mol) 2,5-Dimethoxy-tetrahydrofuran getropft und dann 5 Minuten bei dieser Temperatur gerührt. Anschliessend wird rasch abgekühlt und das Lösungsmittel im Vakuum entfernt. Der Rückstand zwischen Ether und Wasser verteilt, der Ether mit gesättigter Natriumbicarbonatlösung gewaschen, getrocknet und im Vakuum eingedampft. Der rohe Rückstand wird mit Methylenchloird über Kieselgel chromatographiert. Die reinen Eluate, bestehend aus 3,5-Dimethyl-6-(pyrrol-1-yl)-benzofuran-2(3H)-on werden im Hochvakuum destilliert, Kp. (0,01 Torr) 190°. Das Destillat schmilzt bei 61-63°.

Beispiel 21: Ein Gemisch aus 19,6 g (0,1 Mol) 4-Methyl-3-(2-methyl-3-oxobutyl)-maleinsäureanhydrid und 20 g (0,105 Mol) 3-Pyrrolinium-benzoat in 250 ml Benzol wird während 5 Stunden am Wasserabscheider unter Rückfluss erhitzt. Anschliessend wird das Benzol im Vakuum abgedampft und der Rückstand zwischen Ether und 1N Salzsäure verteilt. Die organische Phase wird mit Wasser und gesättigter Natriumbicarbonatlösung gewaschen, getrocknet und eingeengt. Das so erhaltene Rohprodukt wird mit Diisopropylether an Kieselgel chromatographiert. Die erhaltenen reinen Eluate werden aus Diisopropylether umkristallisiert. Auf diese Weise wird 3,5-Dimethyl-6-(3-pyrrolin-1-yl)-benzofuran-2(3H)-on und vom Schmelzpunkt 81-82° erhalten.

Beispiel 22: 4 g (0,01 Mol) 2-[5-Methyl-2-hydroxy-4-(indolin-1-yl)-phenyl]-propionsäure-indolinylamid werden im Hochvakuum bei 0,001 Torr und 200 bis 220°destilliert. Das Destillat wird zwischen Ether und 1N Salzsäure verteilt. Nach dem Trocknen und Abdampfen des Ethers wird ein gelbes Oel erhalten,das über Kieselgel mit Methylenchlorid chromatographiert wird. Das so erhaltene 3,5-Dimethyl-6-(indolin-1-yl)-benzofuran-2(3H)-on wird im Hochvakuum destilliert, Kp. (0,001 Torr) 220°.

Das Ausgangsmaterial kann wie folgt erhalten werden:

Ein Gemisch von 19,6 g (0,1 Mol) 4-Methyl-3-(2-methyl-3-oxobutyl)-maleinsäureanhydrid und 48,2 g Indoliniumbenzoat in 52 ml Benzol wird während 6 Stunden am Wasserabscheider unter Rückfluss erhitzt. Anschliessend wird das Benzol im Vakuum abgedampft und der Rückstand zwischen Aether und 1N Salzsäure verteilt. Die organische Phase wird mit gesättiger Natriumbicarbonatlösung gewaschen und nach dem Trocknen eingeengt. Das so erhaltene rohe (2-[5-Methyl-2-hydroxy-4-(indolin-1-yl)-phenyl]-propionsäure-indolinylamid schmilzt bei 176-178° und wird direkt weiterverarbeitet.

Beispiel 23: Zu einer Lösung von 6 g (0,024 Mol) 3,5-Dimethyl-6-(indolin-1-yl)-benzofuran-2(3H)-on in 50 ml Dioxan werden unter Rühren und Stickstoffatomosphäre bei 20 bis 25° innert 45 Minuten 5,9 g (0,026 Mol) 2,3-Dicyano-5,6-dichlor-benzochinon, gelöst in 100 ml Dioxan, getropft,und dann wird 16 Stunden bei Raumtemperatur gerührt.Anschliessend wird vom Niederschlag abfiltriert und das Lösungsmittel im Vakuum abgedampft. Das so erhaltene Rohprodukt wird mit Methylenchlorid an Kieselgel chromatographiert und die reinen Eluate zusammen im Hochvakuum destilliert. Auf diese Weise wird 3,5-Dimethyl-6-(indol-1-yl)-benzofuran-2(3H)-on vom Kp.(0,001 Torr) 220°C erhalten.

Beispiel 24: Ein Gemisch von 2,3 g (0,01 Mol) 3,5-Dimethyl-6-(pyrrol-1-yl)-3a,6-dihydro-benzofuran-2(3H)-on und 13,6 g (0,06 Mol) 2,3-Dicyano-5,6-dichlor-benzochinon in 50 ml Dioxan wird während 5 Stunden unter Rühren unter Rückfluss erhitzt. Nach dem Abkühlen wird filtriert und im Vakuum das Dioxan abgedampft. Das erhaltene Rohprodukt wird mit Methylenchlorid an Kieselgel chromatographiert und aus Butanol kristallisiert. Das so erhaltene 3,5-Dimethyl-6-(pyrrol-1-yl)-benzofuran-2(3H)-on schmilzt bei 61-63°.

Das Ausgangsmaterial kann beispielsweise wie folgt erhalten werden:

Ein Gemisch von 19,6 g (0,1 Mol) 4-Methyl-3-(2-methyl-3-oxobutyl)-maleinsäureanhydrid und 20 g (0,105 Mol) 3-pyrrolinium-benzoat in 250 ml Benzol wird während 5 Stunden am Wasserabscheider unter Rückfluss erhitzt. Das Benzol wird im Vakuum abgedampft und der Rückstand zwischen Ether und gesättigter Natriumbicarbonatlösung verteilt. Das nach dem Trocknen und Eindampfen der organischen Phase verbleibende Rohprodukt wird an Kieselgel chromatographiert. Elution mit Diisopropylether und anschliessende Umkristallisation der reinen Fraktionen aus Isopropylether liefert das 3,5-Dimethyl-6-(pyrrol-1-yl)-3a,6-dihydro-benzofuran-2(3H)-on vom Smp. 116-117°.

Beispiel 25: Ein Gemisch von 9,5 g (0,05 Mol) 4-Methyl-3-(2-methyl-3-oxobutyl)-maleinsäureanhydrid und 17,9 g (0,08 Mol)cis-3,4-dimethyl-pyrrolidinium-benzoat in 250 ml Benzol wird während 24 Stunden am Wasserabscheider unter Rückfluss erhitzt. Anschliessend wird das Benzol im Vakuum abgedampft und der Rückstand zwischen Ether und 1N Salzsäure verteilt. Die organische Phase wird mit Wasser und gesättigter Natriumbicarbonatlösung gewaschen, getrocknet und eingeengt. Das so erhaltene Rohprodukt wird mit Methylenchlorid an Kieselgel chromatographiert, und die reinen Eluate werden im Hochvakuum destilliert. Auf diese Weise erhält man das 3,5-Dimethyl-6-(cis-3,4-dimethyl-pyrrolidin-1-yl)-benzofuran-2(3H)-on vom Kp. (0,001 Torr) 210°.

In analoger Weise erhält man ausgehend von 4-Methyl-3-(2-methyl-3-oxobutyl)-maleinsäureanhydrid und trans-3,4-dimethyl-pyrrolidinium-benzoat ein reines Stereoisomeres von 3,5-Dimethyl-6-(trans-3,4-dimethyl-pyrrolidin-1-yl)-benzofuran-2(3H)-on vom Smp. 104-105° und ein Stereoisomerengemisch vom Schmelzpunkt 62-77°.

Beispiel 26: Ein Gemisch aus 1,8 g (6 mMol) 2-[2-Hydroxy-5-methyl-4-(pyrrolidin-1-yl)-phenyl]-propionsäurepyrrolidid, 2ml Eisessig und 2 ml konz. Salzsäure wird während 40 Minuten am Rückfluss gekocht. Das Reaktionsgemisch wird im Vakuum eingedampft, der Rückstand

mit Wasser versetzt, mit verdünnter Natronlauge auf pH 5 gestellt und 3 mal mit Ether extrahiert. Die vereinigten Etherauszüge werden über Natriumsulfat getrocknet und im Vakuum auf 10 ml eingeengt. Man versetzt mit 1,2 g (6 mMol) N,N'-Dicyclohexylcarbodiimid, rührt eine Stunde bei Raumtemperatur, filtriert den Rückstand ab, wäscht mit Ether nach und dampft das Filtrat im Vakuum ein. Der Rückstand wird mit Petrolether/Ether an Kieselgel chromatographiert. Anschliessende Umkristallisation der reinen Fraktionen aus Ether/Petrolether ergibt 3,5-Dimethyl-6-(pyrrolidin-1-yl)-benzofuran-2(3H)-on vom Smp. 68-69°.

Das Ausgangsmaterial kann beispielsweise wie folgt erhalten werden: Zu 81,1 g (0,5 Mol) 4-Methyl-2-(1-methyl-2- propenyl)-phenol werden bei Raumtemperatur unter Rühren während 1 Stunde 42,6 ml (0,6 Mol) Acetylchlorid getropft. Anschliessend wird das Reaktionsgemisch auf 100° erhitzt und während 2 Stunden bei dieser Temperatur belassen. Nach dem Abkühlen wird vorsichtig mit Wasser versetzt und mit Methylenchlorid extrahiert. Die organische Phase wird über Natriumsulfat getrocknet und eingedampft. Anschliessende Destillation des verbleibenden Rückstands (64-70°/ $4 \times 10^{-2}$ Torr ) ergibt 4-Methyl-2-(1-methyl-2-propenyl)-phenylacetat als blassgelbes Oel.

Ein Gemisch aus 20,4 g (0,1 Mol) 4-Methyl-2-(1-methyl-2-propenyl)-phenylacetat und 100 mg (0,4 mMol) Osmiumtetroxid in 300 ml Dioxan und 100 ml Wasser wird während 30 Minuten bei Raumtemperatur gerührt, anschliessend während 30 Minuten portionenweise mit 42,8 g (0,2 Mol) Natriumperiodat versetzt und eine Stunde nachgerührt. Der entstandene Niederschlag wird abfiltriert und mit Dioxan/Wasser (1:1) nachgewaschen. Die wässrig-organische Phase wird im Vakuum auf etwa 1 Drittel eingeengt und mit Methylenchlorid ausgezogen. Das nach dem Trocknen und Entfernen des Methylenchlorids erhaltene ölige Rohprodukt wird in 100 ml Aceton aufgenommen und durch halbstündiges Eintropfen einer Lösung von 7,2 g (72 mMol) Chromtrioxid und 6,2 ml konz. Schwefelsäure in 40 ml Wasser oxidiert. Anschliessend wird mit 3 ml Methanol und 200 ml Wasser versetzt, das Aceton im Vakuum entfernt, die wässrige Phase mit Ether extrahiert und die Etherlösung 3 ml mit 10%iger Natronlauge ausgezogen. Die alkalische wässrige Lösung wird während 3 Stunden bei Raumtemperatur stehengelassen, anschliessend mit konz. Salzsäure auf pH 3 gestellt und mit Ether extrahiert. Das nach dem Trocknen und Entfernen des Ethers erhaltene Oel wird während 2 Stunden mit 300 ml gesättigter methanolischer Salzsäure verrührt. Anschliessend wird das Methanol im Vakuum entfernt und der Rückstand zwischen Ether und verdünnter Natriumbicarbonatlösung verteilt. Das nach dem Trocknen und Eindampfen der organischen Phase erhaltene Rohprodukt wird mit Methylenchlorid an Kieselgel chromatographiert. Anschliessende Umkristallisation der reinen Fraktionen aus Methylenchlorid/Petrolether ergibt 2-(2-Hydroxy-5-methylphenyl)-propionsäuremethylester vom Smp. 104-106°.

Ein Gemisch aus 5,8 g (30 mMol) 2-(2-Hydroxy-5-methylphenyl)-propionsäuremethylester, 36,5 g (82 mMol) Bleitetraacetat und 150 ml Eisessig wird während 36 Stunden bei Raumtemperatur gerührt. Der Eisessig wird im Vakuum entfernt und der Rückstand mit 300 ml Wasser versetzt. Der entstandene Niederschlag wird abfiltriert und gründlich mit Ether gewaschen. Das Filtrat wird mit Ether ausgezogen. Die vereinigten Etherphasen werden über Natriumsulfat getrocknet und im Vakuum eingedampft. Das verbleibende rötliche Oel wird in 80 ml Dioxan aufgenommen, mit 8,7 ml (106 mMol) Pyrrolidin versetzt und während 5 Stunden unter Rückfluss gekocht. Das Dioxan wird im Vakuum entfernt und der Rückstand mit Methylenchlorid/Aceton an Kieselgel chromatographiert. Nach Umkristallisation der reinen Fraktionen aus Aceton erhält man 2-[2-Hydroxy-5-methyl-2-(pyrrolidin-1-yl)-phenyl]-propionsäurepyrrolidid vom Smp. 178-180°.

Beispiel 27: Eine methanolische Lösung von 26,9 g (0,1 Mol) 5-Chlor-2-methoxy-4-morpholino-acetophenon wird portionenweise unter Rühren mit 3,8 g (0,1 Mol) Natriumborhydrid versetzt und 1 Stunde bei Raumtemperatur gerührt. Das Methanol wird am Vakuumrotationsverdampfer eingeengt und der Rückstand zwischen verdünnter Salzsäure und Methylenchlorid verteilt. Die organischen Phasen werden vereinigt, über Natriumsulfat getrocknet und eingedampft. Der Rückstand wird in 60 ml absolutem Methylenchlorid aufgenommen und während 2 Stunden unter Stickstoffatmosphäre zu einem Gemisch von 17,8 g (0,15 Mol) Thionylchlorid und 120 ml absolutem Methylenchlorid getropft. Anschliessend wird noch eine Stunde gerührt, das Lösungsmittel am Vakuumrotationsverdampfer eingeengt und der Rückstand zwischen Natriumbicarbonatlösung und Methylenchlorid verteilt. Die organischen Phasen werden neutral gewaschen, vereinigt, über Natriumsulfat getrocknet und eingeengt. Der Rückstand wird in 100 ml absolutem Tetrahydrofuran aufgenommen und zu einer Suspension von 2,4 g (0,1 g/Atom) Magnesium-Spänen in 20 ml absolutem Tetrahydrofuran so zugetropft, dass das Reaktionsgemisch leicht unter Rückfluss kocht. Anschliessend wird noch 2 Stunden unter Rückfluss weitergekocht. Die auf Raumtemperatur abgekühlte Lösung wird vorsichtig zu ca. 50 g mit absolutem Tetrahydrofuran überschichteten Trockeneis getropft. Das Reaktionsgemisch wird auf Raumtemperatur erwärmt, mit verdünnter Salzsäure angesäuert und 3 mal mit Methylenchlorid extrahiert. Die organischen Phasen werden neutral gewaschen, vereinigt, über Natriumsulfat getrocknet unter dem Vakuumrotationsverdampfer eingeeingt. Umkristallisation des Rohprodukts aus Essigester/Petrolether ergibt 2-(5-Chlor-2-methoxy-4-morpholino-phenyl)-propionsäure vom Smp. 164-165°.

8,99 g 2-(5-Chlor-2-methoxy-4-morpholino-phenyl)-propionsäure (0,03 Mol) werden in 48%iger Bromwasserstofflösung 2 Stunden gekocht. Das Reaktionsgemisch wird abgekühlt und eingeengt und 3 mal mit Methylenchlorid extrahiert. Die organischen Phasen werden neutral

gewaschen, vereinigt über Natriumsulfat getrocknet und am Vakuumrotationsverdampfer eingeengt. Man erhält so das 5-Chlor-3-methyl-6-(morpholin-4-yl)-benzofuran-2(3H)-on vom Smp. 103-105°.

Beispiel 28: Zu einer unter Stickstoff siedenden Suspension von 530 mg (11 mMol) 50%iger Natriumhydrid-öl-dispersion in 15 ml absolutem 1,2-Dimethoxyethan wird eine Lösung von 2,5 g (10 mMol) 5-Chlor-6-(piperidin-1-yl)-benzofuran-2(3H)-on und 2,1 g (15 mMol) mit Methyliodid in 15 ml absolutem 1,2-Dimethoxyethan während 20 Minuten getropft. Anschliessend wird noch 3 Stunden unter Rückfluss gekocht, das Lösungsmittel am Vakuum entfernt und der Rückstand vorsichtig mit verdünnter Salzsäure angesäuert und dreimal mit Methylenchlorid extrahiert. Die organischen Phasen werden mit Wasser gewaschen, vereinigt, über Natriumsulfat getrocknet und am Vakuumrotationsverdampfer eingeengt. Umkristallisation aus Ether/Petrolether ergibt 5-Chlor-3-methyl-6-(piperidin-1-yl)-benzofuran-2(3H)-on vom Schmelzpunkt 112-113°.

Beispiel 29: Zu einer unter Stickstoff siedenden Suspension von 530 mg (11 mMol) 50%iger Natriumhydrid-öl-dispersion in 15 ml absolutem 1,2-Dimethoxyethan wird eine Lösung von 2,5 g (10 mMol) 5-Chlor-6-(piperidin-1-yl)-benzofuran-2(3H)-on in 15 ml absolutem 1,2-Dimethoxyethan während 20 Minuten getropft. Anschliessend wird noch 3 Stunden unter Rückfluss gekocht. Dann wird auf -20° abgekühlt und mittels Stickstoff wasserfreies Formaldehyd-Gas eingeleitet. Nach weiterem Rühren während 30 Minuten wird mit verdünnter Salzsäure hydrolysiert und das Produkt mit Ether extrahiert. Die Etherextrakte werden mit Wasser gewaschen, vereinigt über Natriumsulfat getrocknet und eingeengt. Der Rückstand, das 5-Chlor-3-hydroxymethyl-6-(piperidin-1-yl)-benzofuran-2(3H)-on, wird in 30 ml absolutem Pyridin aufgenommen und mit 2,1 g (11 mMol) Tosylchlorid versetzt. Nach Rühren während 24 Stunden bei Raumtemperatur wird während 6 Stunden auf Rückfluss erhitzt. Das Reaktionsgemisch wird abgekühlt, das Pyridin

am Vakuumrotationsverdampfer eingeengt und der Rückstand zwischen verdünnter Salzsäure und Methylenchlorid verteilt. Die organischen Phasen werden mit Bicarbonatlösung und mit Wasser neutral gewaschen, vereingt und über Natriumsulfat getrocknet und eingeengt. Der Rückstand wird in 20 ml Dioxan gelöst, mit 200 ml Palladium/Kohle (10%ig) versetzt und bei Raumtemperatur hydriert. Der Katalysator wird abfiltriert, das Filtrat eingeengt und der Rückstand aus Ether/ Petrolether umkristallisiert. Es resultiert 5-Chlor-3-methyl-6-(piperidin-1-yl)-benzofuran-2(3H)-on vom Smp. 108-111°.

Beispiel 30: Eine Lösung von 2,5 g (10 mMol) 2-[2-Hydroxy-5-methyl-4-(pyrrol-1-yl)-phenyl]-propionsäure in 30 ml absolutem Methylenchlorid wird bei Raumtemperatur mit 2,27 g (11 mMol) Dicyclohexylcarbodiimid in 20 ml absolutem Methylenchlorid versetzt. Das Reaktionsgemisch wird ca. 30 Minuten bei Raumtemperatur gerührt, der Niederschlag abfiltriert und das Filtrat am Vakuumrotationsverdampfer eingeengt. Destillation ergibt 3,5-Dimethyl-6-(pyrrol-1-yl)-2-benzofuran-2(3H)-on, Kp. 180°/0,005 Torr, Fp. 61-63°.

Das Ausgangsmaterial kann beispielsweise folgendermassen hergestellt werden:

Eine Lösung von 132,9 g (0,759 Mol) 4-Methyl-3-nitro-anisol in 1,1 Liter Methanol wird mit 12,4 g Palladium auf Kohle versetzt und bei Raumtemperatur hydriert. Der Katalysator wird abfiltriert und das Filtrat am Vakuumrotationsverdampfer eingeengt. Umkristallisation aus Isopropanol/Wasser ergibt 3-Amino-4-methyl-anisol vom Smp. 43-44°.

Eine Lösung von 88,4 g (0,64 Mol) 3-Amino-4-methyl-anisol in 1,4 Liter Eisessig wird auf 106° erwärmt und bei dieser Temperatur während 30 Minuten mit 114 g (0,86 Mol) 2,5-Dimethoxy-tetrahydrofuran versetzt. Anschliessend wird sofort auf Raumtemperatur abgekühlt und am Vakuumrotationsverdampfer eingeengt. Destillation des Rückstandes

am Hochvakuum liefert das 4-Methyl-3-(pyrrol-1-yl)-anisol, Kp. 93-95°/0,04 Torr. $R_f$ (Toluol/Essigester =10:1):0, 57.

Eine Lösung von 86,6 g (0,46 Mol) 4-Methyl-3-(pyrrol-1-yl)-anisol in 1,5 Liter absolutem Methylenchlorid wird mit Aceton/Trockeneis auf -78° abgekühlt. Bei dieser Temperatur werden 231,7 g (0,92 Mol) Bortribromid zugetropft. Anschliessend wird das Kühlbad entfernt und das Reaktionsgemisch auf 0 bis 5° erwärmt, dann auf 2 Liter Eis /Wasser gegossen,die Methylenchloridphase abgetrennt und mit gesättigter Kochsalzlösung gewaschen. Die wässrigen Phasen werden noch 2 mal mit Methylenchlorid nachextrahiert. Die organischen Phasen werden vereinigt, über Natriumsulfat getrocknet und am Vakuumrotationsverdampfer eingeengt. Destillation des Rückstandes am Hochvakuum ergibt das 4-Methyl—3-(pyrrol-1-yl)-phenol, Kp. 105-107°/ 0,03 Torr, $R_f$ (Toluol/Essigester = 10:1):0,38.

Eine Suspension von 53,4 g (0,31 Mol) 4-Methyl-3-(pyrrol-1-yl)-phenol und 53,7 g (0,39 Mol) Kaliumcarbonat in 60 ml absolutem Aceton wird unter Rückfluss während 1 Stunde mit 45,7 g (0,39 Mol) Crotylbromid versetzt und anschliessend noch $4\frac{1}{2}$ Stunden weitergekocht. Das Reaktionsgemisch wird abgekühlt und mit 800 ml Wasser verdünnt. Das Aceton wird am Vakuumrotationsverdampfer abgedampft und der Rückstand mehrmals mit Methylenchlorid extrahiert. Die organischen Phasen werden mit Wasser gewaschen, vereinigt und über Natriumsulfat getrocknet und am Vakuumrotationsverdampfer eingeengt. Kurzfiltration an etwa 800 g Kieselgel mit Methylenchlorid ergibt 1-[4-Methyl-3-(pyrrol-1-yl)]-phenoxy-2-buten als hellgelbes Oel, $R_f$ (Hexan/ Ether = 9:1):0,45, $R_f$ (Toluol/Essigester = 10:1):0,68.

Eine Lösung von 60 g (0,26 Mol) 1-[4-Methyl-3-(pyrrol-1-yl)]-phenoxy-2-buten in 170 ml absolutem N,N-Diethylanilin wird während 5 Stunden unter Rückfluss gekocht. Das Reaktionsgemisch wird abgekühlt, mit Methylenchlorid verdünnt und mit 6N Salzsäure angesäuert. Die Wasserphase wird abgetrennt und nochmals mit Methylenchlorid extrahiert. Die organischen Phasen werden neutral gewaschen, vereinigt, über Natriumsulfat getrocknet und am Vakuumrotationsverdampfer eingeengt. Chromatographie an Kieselgel mit Hexan/Ether (9:1) ergibt 3-[2-Hydroxy-5-methyl-4-(pyrrol-1-yl)-phenyl]-1-buten. $R_f$ (Hexan/Ether = 9:1):0,17, $R_f$ (Toluol/Essigester = 10:1):0,45.

Eine Lösung von 26,7 g (0,12 Mol) 3-[2-Hydroxy-5-methyl-4-(pyrrol-1-yl)-phenyl]-1-buten in 370 ml Essigsäureanhydrid wird mit einigen Tropfen Pyridin versetzt und während 2 Stunden bei Raumtemperatur gerührt. Das Reaktionsgemisch wird auf Eis gegossen und mit Methylenchlorid 3 mal extrahiert.Die Methylenchloridphasen werden mit verdünnter Natriumbicarbontlösung, dann mit Wasser neutral gewaschen, vereinigt über Natriumsulfat getrocknet und am Vakuumrotationsverdampfer eingeengt. Filtration an wenig Kieselgel mit Methylenchlorid ergibt 3-[2-Acetoxy-5-methyl-4-(pyrrol-1-yl)-phenyl]-1-buten, $R_f$ (Toluol/Essigester = 10:1):0,55.

Eine Lösung von 2,7 g (10 mMol) 3-[2-Acetoxy-5-methyl-4-(pyrrol-1-yl)-phenyl]-1-buten in 40 ml absolutem Methylenchlorid wird mit Aceton /Trockeneis auf -78° abgekühlt und solange Ozon durchgeblasen bis die blaue Farbe nicht mehr verschwindet. Dann werden 2 ml Dimethylsulfid zugegeben und das Kühlbad entfernt. Das Reaktionsgemisch wird am Vakuumrotationsverdampfer schonend eingeengt, der Rückstand in 50 ml Ethanol gelöst und mit einer Lösung von 3,7 g (23 mMol) Silbernitrat in 5 ml Wasser versetzt. Zu diesem Gemisch wird während etwa 15 Minuten eine Lösung von 75 ml einer 1N Kaliumhydroxidlösung getropft. Das heterogene Gemisch wird während weiteren 2 Stunden weitergerührt. Das Reaktionsgemisch wird filtriert und der Rückstand mit Ethanol gewaschen. Das alkalische Filtrat wird über Nacht bei Raumtemperatur stehengelassen und mit Methylenchlorid extrahiert. Die alkalische Lösung wird mit 6N Salzsäure unter Kühlung vorsichtig angesäuert und mit Methylenchlorid mehrmals extrahiert. Die organischen

Phasen werden noch zweimal mit Wasser gewaschen, vereinigt, über Natriumsulfat getrocknet und am Vakuumrotationsverdampfer eingeengt. Umkristallisation aus Diisopropylether/Petrolether ergibt 2-[2-Hydroxy-5-methyl-4-(pyrrol-1-yl)-phenyl]-propionsäure vom Smp. 73-74°.

Beispiel 31: 1,8 g 6-Amino-3,5-dimethyl-benzofuran-2(3H)-on werden in 20 ml Dioxan gelöst und unter Rühren bei Raumtemperatur mit 2 ml 2,5-Dimethoxy-tetrahydrofuran und 1,4 ml 37%iger Salzsäure versetzt. Nach 30 Minuten wird die wässrige Phase abgetrennt und die organische Phase im Vakuum zur Trockene eingedampft. Der Rückstand wird in Methylenchlorid über Kieselgel chromatographiert. Das erhaltene hellgelbe Oel kristallisiert aus Butyloxid. Man erhält so das 3,5-Dimethyl-6-(pyrrol-1-yl)-benzofuran-2(3H)-on vom Smp. 61-63°C.

Das Ausgangsmaterial kann wie folgt hergestellt werden:
59 g 4-Methyl-3-(2-methyl-3-oxo-butyl)-maleinsäureanhydrid und 240 g Dibenzylammoniumbenzoat werden in 1000 ml Benzol mit einem Wasserabscheider 48 Stunden am Rückfluss gekocht. Anschliessend wird im Vakuum zur Trockene eingedampft und der Rückstand über Kieselgel chromatographiert. Das erhaltene Oel kristallisiert aus Isopropylether. Man erhält so das 2-(4-Dibenzylamino-2-hydroxy-5-methyl-phenyl)-propionsäure-dibenzylamid vom Smp. 140-141°C.

20 g 2-(4-Dibenzylamino-2-hydroxy-5-methyl-phenyl)-propionsäure-dibenzylamid werden in 40 ml 2n Salzsäure und 40 ml Eisessig 3 Stunden am Rückfluss erhitzt. Anschliessend wird im Vakuum zur Trockene eingedampft und der Rückstand zwischen Ether und 1n Natronlauge verteilt.

Durch Ansäuern auf pH 1 mit Salzsäure und Extrahieren mit Ether erhält man die 2-[4-Dibenzylamino-2-hydroxy-5-methyl-phenyl)-propionsäure, die zur Reinigung in Methylenchlorid über Kieselgel chromatographiert wird. Die farblosen Kristalle schmelzen bei 174-175°C. 5 g davon werden in 50 ml Ether gelöst und mit 6 g Dicyclohexylcarbodiimid versetzt. Nach 30 Minuten wird vom gebildeten Harnstoff abfiltriert und

das Filtrat zur Trockene eingedampft. Man erhält so das 6-Dibenzylamino-3,5-dimethyl-benzofuran-2(3H)-on vom Smp. 122-123°C.

4 g 6-Dibenzylamino-3,5-dimethyl-benzofuran-2(3H)-on werden in 40 ml Dioxan gelöst und mit 0,4 g Palladium auf Kohle (5%ig) bei Raumtemperatur unter Normaldruck mit Wasserstoff reduziert. Anschliessend wird filtriert, das Filtrat zur Trockene eingedampft und aus Methanol umkristallisiert. Man erhält so das 6-Amino-3,5-dimethyl-benzofuran-2(3H)-on vom Smp. 123-124°C.

Beispiel 32: 30 g 2-(4-Dibenzylamino-2-hydroxy-5-methyl-phenyl)-propionsäuredibenzylamid werden in einer Kurzwegdestillationsapparatur im Hochvakuum destilliert. Die Fraktion, die bei 0,01 Torr und 160-175°C destilliert, besteht aus reinem 6-Dibenzylamino-3,5-dimethylbenzofuran-2(3H)-on. Smp. 122-123°C.

Beispiel 33: 5,3 g (0,03 Mol) 6-Amino-3,5-dimethyl-benzofuran-2(3H)-on, 4,1 g (0,037 Mol) Acetonylaceton, 50 ml Benzol und 0,5 ml Eisessig werden 14 Stunden unter Rückfluss erhitzt. Nach dem Abkühlen wird mit Wasser, gesättigter Natriumbicarbonatlösung und 1n Salzsäure gewaschen. Anschliessend wird das Benzol im Vakuum abgedampft und der Rückstand mit Methylenchlorid über Kieselgel chromatographiert. Nach Kristallisation der reinen Eluate erhält man 3,5-Dimethyl-6-(2,5-dimethylpyrrol-1-yl)-benzofuran-2(3H)-on vom Smp. 94-95°.

Beispiel 34: Tabletten, enthaltend 25 mg Wirkstoff, z.B. 3,5-Dimethyl-6-(pyrrolidin-1-yl)-benzofuran-2(3H)-on können folgendermassen hergestellt werden:

Bestandteile (für 1000 Tabletten):

| | |
|---|---|
| Wirkstoff | 25,0 g |
| Lactose | 100,7 g |
| Weizstärke | 7,5 g |

| | |
|---|---|
| Polyethylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 35: Kautabletten, enthaltend 30 mg Wirkstoff, z.B. 5-Brom-3-methyl-6-(pyrrolidin-1-y-)-benzofuran-2(3H)-on, können z.B. folgendermassen hergestellt werden:

Zusammensetzung (für 1000 Tabletten):

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccarin | 1,0 g |
| 5%ige Gelatinelösung | q.s. |

Herstellung Alle festen Ingredienzen werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nachmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpresst.

Beispiel 36: Tabletten enthaltend 100 mg Wirkstoff, z.B. 3,5-Dimethyl-6-(pyrrol-1-yl)-benzofuran-2(3H)-on können folgendermassen hergestellt werden:

Zusammensetzung (für 1000 Tabletten):

| | |
|---|---|
| Wirkstoff | 100,0 g |
| Lactose | 248,5 g |
| Maisstärke | 17,5 g |
| Polyethylenglykol 6000 | 5,0 g |
| Talkum | 15,0 g |
| Magnesiumstearat | 4,0 g |
| entmineralisiertes Wasser | q.s. |

Herstellung: Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösungs des Polyethylenglykols in 260 ml Wasser hinzugegeben. Der erhaltene Kleister wird zu den pulverförmigen Substanzen hinzugefügt, das Ganze vermischt und granuliert,

erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

Die Ausgangsstoffe der Formel

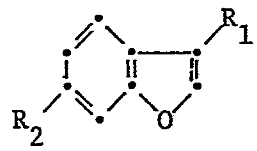

(V'c),

die speziell für die Herstellung der erfindungsgemässen Verbindungen entwickelt wurden, ferner die Ausgangsstoffe der Formeln II, III, IV, V und VI, die Verfahren zu ihrer Herstellung sowie deren Verwendung bilden ebenfalls einen Gegenstand der Erfindung.

Patentansprüche: (für alle benannten Staaten ausser Oesterreich)

1. Benzofuranone der allgemeinen Formel

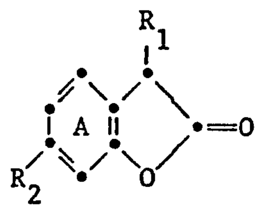

(I),

worin $R_1$ für Wasserstoff oder einen aliphatischen Rest steht, $R_2$ eine durch einen zweiwertigen Kohlenwasserstoffrest disubstituierte Aminogruppe bedeutet und der aromatische Ring A zusätzlich substituiert sein kann, und ihre Salze und/oder Isomeren.

2. Verbindungen der Formel (I) gemäss Anspruch 1, worin $R_1$ Wasserstoff oder einen gesättigten und unsubstituierten aliphatischen Rest bedeutet, $R_2$ eine durch einen zweiwertigen aliphatischen Rest, der auch durch Aza, N-Niederalkylaza, Oxa oder Thia unterbrochen sein kann, disubstituierte Aminogruppe darstellt und der aromatische Ring A zusätzlich durch einen aliphatischen Rest, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze und Isomeren.

3. Verbindungen der Formel (I) gemäss Anspruch 1, worin $R_1$ Wasserstoff oder Niederalkyl bedeutet, $R_2$ eine durch Niederalkylen, Niederalkenylen, Aza-niederalkylen, N'-Niederalkylazaniederalkylen, Aza-niederalkenylen, N'-Niederalkylaza-niederalkenylen, Oxa- bzw. Thianiederalkylen oder Oxa- bzw. Thia-niederalkenylen disubstituierte Aminogruppe, wobei Niederalkylen bzw. Niederalkenylen jeweils 4- bis 10 C-Atome aufweist und auch verzweigt sein kann sowie mit einem oder

zwei Benzosystemen ortho-anelliert sein kann, darstellt und der aromatische Ring A zusätzlich durch Niederalkyl, Hydroxyniederalkyl, Halogenniederalkyl, Niederalkenyl, gegebenenfalls verzweigtes 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze und Isomeren.

4. Verbindungen der Formel (I) gemäss Anspruch 1, worin $R_1$ Wasserstoff oder Niederalkyl bedeutet, $R_2$ eine durch Niederalkylen, Niederalkenylen oder durch Aza, N-Niederalkylaza, Oxa oder Thia unterbrochenes Niederalkylen bzw. durch Aza oder N-Niederalkylaza unterbrochenes Niederalkenylen substituierte Aminogruppe darstellt und der aromatische Ring A zusätzlich durch Niederalkyl, Niederalkoxy, Hydroxy, Halogen, Niederalkanoyloxy, 3- oder 4-gliedriges Alkylen und/oder Trifluormethyl substituiert sein kann, und ihre Salze und Isomeren.

5. Verbindungen der Formel

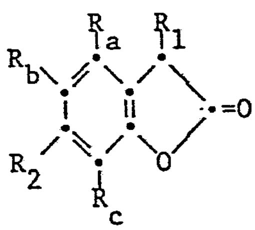

(Ia) gemäss Anspruch 1,

worin $R_1$ Wasserstoff oder Niederalkyl, bedeutet, $R_2$ jeweils 5- bis 8-gliedriges Niederalkylenamino, Niederalkenylenamino, Azaniederalkylen-amino, N'-Niederalkylaza-niederalkylen-amino, Azaniederalkenylen-amino, N'-Niederalkylaza-niederalkenylen-amino, Oxa- bzw. Thia-niederalkylen-amino, Isoindol-2-yl, Isoindolin-2-yl, Indolin-1-yl oder Indol-1-yl darstellt und $R_a$, $R_b$ sowie $R_c$ unabhängig voneinander Wasserstoff, Niederalkyl, Hydroxyniederalkyl, Halogenniederalkyl, Niederalkenyl, Niederalkoxy, Niederalkylthio, Niederalkansulfinyl, Niederalkansulfonyl, Hydroxy, Halogen, Niederalkanoyloxy, Niederalkanoyl oder Nitro bedeuten oder $R_a$ gemeinsam mit $R_b$ 3- oder 4-gliedriges Alkylen, darstellen und $R_c$ die vorstehend für $R_c$ angegebenen Bedeutungen hat, und ihre Salze und Isomeren.

6. Verbindungen der Formel (Ia) gemäss Anspruch 1, worin $R_1$ Wasserstoff oder Niederalkyl bedeutet, $R_2$ jeweils 5- bis 8-gliedriges Niederalkylenamino, Niederalkenylenamino, Monoaza-niederalkylenamino, N'-Niederalkylmonoaza-niederalkylenamino, Monooxa-niederalkylenamino, Monothia-niederalkylenamino, Monoaza-niederalkenylenamino oder N'-Niederalkylaza-niederalkenylenamino darstellt und $R_a$, $R_b$ sowie $R_c$ unabhängig voneinander Wasserstoff, Niederalkyl, Niederalkoxy, Hydroxy, Halogen, Niederalkanoyloxy oder Trifluormethyl bedeuten oder $R_a$ gemeinsam mit $R_b$ 3- oder 4-gliedriges Alkylen darstellen und $R_c$ die vorstehend für $R_c$ angegebenen Bedeutungen hat, und ihre Salze und Isomeren.

7. Verbindungen der Formel (Ia) gemäss Anspruch 1, worin $R_1$ Wasserstoff oder Niederalkyl mit bis und mit 4 C-Atomen bedeutet, $R_2$ 5- bis 8-gliedriges Niederalkylen-amino mit 4 bis 10 C-Atomen, 5- bis 8-gliedriges Niederalkenylen-amino mit einer oder zwei Doppelbindungen und mit 4 bis 10 C-Atomen, Monooxa-niederalkylenamino mit 4 bis 7 C-Atomen, Indolin-1-yl oder Inol-1-yl darstellt und $R_a$ und $R_b$ unabhängig voneinander Wasserstoff, Niederalkyl oder Halogen oder $R_a$ und $R_b$ gemeinsam 3- bis 4-gliedriges Alkylen, bedeuten und $R_c$ Wasserstoff darstellt, und ihre Salze und Isomeren.

8. Verbindungen der Formel (Ia) gemäss Anspruch 1, worin $R_1$ Wasserstoff oder Niederalkyl bedeutet, $R_2$ 5- bis 8-gliedriges Niederalkylenamino, 5- bis 8-gliedriges Niederalkenylenamino oder Monooxa-niederalkylenamino darstellt und $R_a$ sowie $R_b$ unabhängig voneinander Wasserstoff, Niederalkyl oder Halogen oder

$R_a$ gemeinsam mit $R_b$ 3- bis 4-gliedriges Alkylen bedeuten, und $R_c$ Wasserstoff darstellt, und ihre Salze und Isomeren.

9. Verbindungen der Formel (Ia) gemäss Anspruch 1, worin $R_1$ Wasserstoff oder Niederalkyl mit bis und mit 4 C-Atomen bedeutet, $R_2$ 5- bis 8-gliedriges Niederalkylenamino, 5- bis 8-gliedriges Niederalkenylenamino oder Monooxa-niederalkylenamino darstellt und $R_a$ und $R_c$ Wasserstoff bedeuten und $R_b$ Wasserstoff, Niederalkyl mit bis und mit 4 C-Atomen oder Halogen bis und mit Atomnummer 35 darstellt, und ihre Salze und Isomeren.

10. Verbindungen der Formel (Ia) gemäss Anspruch 1, worin $R_1$ Wasserstoff oder Niederalkyl mit bis und mit 4 C-Atomen, bedeutet, $R_2$ 1-Pyrrolyl, 3-Pyrrolidin-1-yl, Pyrrolidin-1-yl oder Piperidin-1-yl darstellt, $R_a$ und $R_c$ jeweils Wasserstoff bedeuten und $R_b$ Niederalkyl mit bis und mit 4 C-Atomen, oder Halogen bis und mit Atomnummer 35 darstellt, und ihre Salze und Isomeren.

11. Verbindungen der Formel (Ia) gemäss Anspruch 1, worin $R_1$ Wasserstoff oder Niederalkyl mit bis und mit 4 C-Atomen, bedeutet, $R_2$ 1-Pyrrolyl, 4-Morpholinyl, 3-Pyrrolin-1-yl oder unverzweigtes 4- bis 6-gliedriges Alkylenamino darstellt, $R_a$ und $R_c$ jeweils Wasserstoff bedeuten und $R_b$ Wasserstoff, Niederalkyl mit bis und mit 4 C-Atomen oder Halogen bis und mit Atomnummer 35 darstellt, oder $R_c$ Wasserstoff bedeutet und $R_a$ und $R_b$ gemeinsam 3- bis 4-gliedriges Alkylen oder einer der Reste $R_a$ und $R_b$ Halogen bis und mit Atomnummer 35 und der andere Niederalkyl mit bis 4 C-Atomen, darstellen, und ihre Salze und Isomeren.

12. Verbindungen der Formel (Ia) gemäss Anspruch 1, worin $R_1$ Niederalkyl mit bis und mit 4 C-Atomen bedeutet, $R_2$ 5- bis 8-gliedriges Niederalkenylenamino darstellt, $R_a$ und $R_c$ Wasser-

stoff bedeuten und $R_b$ Niederalkyl mit bis und mit 4 C-Atomen darstellt, und ihre Salze und ihre Isomeren.

13. 3-Methyl-6-morpholino-benzofuran-2(3H)-on oder ein Salz oder Isomeres davon.

14. 3-Methyl-6-(pyrrolidin-1-yl)-benzofuran-2(3H)-on oder ein Salz oder Isomeres davon.

15. 3-Methyl-6-(piperidin-1-yl)-benzofuran-2(3H)-on oder ein Salz oder Isomeres davon.

16. 6-(Hexahydroazepin-1-yl)-3-methyl-benzofuran-2(3H)-on oder ein Salz oder Isomeres davon.

17. 3,5-Dimethyl-6-(pyrrolidin-1-yl)-benzofuran-2(3H)-on oder ein Salz oder Isomeres davon.

18. 3,5-Dimethyl-6-morpholino-benzofuran-2(3H)-on oder ein Salz oder Isomeres davon.

19. 3,4-Dimethyl-6-morpholino-benzofuran-2(3H)-on oder ein Salz oder Isomeres davon.

20. 5-Chlor-3-methyl-6-morpholino-benzofuran-2(3H)-on oder ein Salz oder Isomeres davon.

21. 5-Chlor-3-methyl-6-(piperidin-1-yl)-benzofuran-2(3H)-on oder ein Salz oder Isomeres davon.

22. 5-Chlor-3-methyl-6-(pyrrolidin-1-yl)-benzofuran-2(3H)-on oder ein Salz oder Isomeres davon.

23. 5-Brom-3-methyl-6-morpholino-benzofuran-2(3H)-on oder ein Salz oder Isomeres davon.

24. 5-Brom-3-methyl-6(pyrrolidin-1-yl)-benzofuran-2(3H)-on oder ein Salz oder Isomeres davon.

25. 5-Brom-3,4-dimethyl-6-morpholino-benzofuran-2(3H)-on oder ein Salz oder Isomeres davon.

26. 5-Chlor-6-(piperidin-1-yl)-benzofuran-2(3H)-on oder ein Salz oder Isomeres davon.

27. 6-(4-Morpholino)-benzofuran-2(3H)-on oder ein Salz oder Isomeres davon.

28. 5-Chlor-3-methyl-6-(pyrrol-1-yl)-benzofuran-2(3H)-on oder ein Salz oder Isomeres davon.

29. 3,5-Dimethyl-6-(hexahydroazepin-1-yl)-benzofuran-2(3H)-on oder ein Salz oder Isomeres davon.

30. 3,5-Dimethyl-6-(piperidin-1-yl)-benzofuran-2(3H)-on oder ein Salz oder Isomeres davon.

31. 5-Ethyl-3-methyl-6-(pyrrolidin-1-yl)-benzofuran-2(3H)-on oder ein Salz oder Isomeres davon.

32. 3-Methyl-6-(pyrrolidin-1-yl)-4,5-tetramethylen-benzofuran-2(3H)-on oder ein Salz oder Isomeres davon.

33. 3,5-Dimethyl-6-(pyrrol-1-yl)-benzofuran-2(3H)-on oder ein Salz oder Isomeres davon.

34. 3,5-Dimethyl-6-(3-pyrrolin-1-yl)-benzofuran-2(3H)-on oder ein Salz oder Isomeres davon.

35. 3,5-Dimethyl-6-(indolin-1-yl)-benzofuran-2(3H)-on oder ein Salz oder Isomeres davon.

36. 3,5-Dimethyl-6-(indol-1-yl)-benzofuran-2(3H)-on oder ein Salz oder Isomeres davon.

37. 3,5-Dimethyl-6-(cis-3,4-dimethyl-pyrrolidin-1-yl)-benzofuran-2(3H)-on oder ein Salz oder Isomeres davon.

38. 3,5-Dimethyl-6-(trans-3,4-dimethyl-pyrrolidin-1-yl)- und benzofuran-2(3H)-on oder ein Salz oder Isomeres davon.

39. 3,5-Dimethyl-6-(2,5-dimethylpyrrol-1-yl)-benzofuran-2(3H)-on oder ein Salz oder Isomeres davon.

40. Verbindung gemäss einem der Ansprüche 2, 3, 5, 7, 10, 11 und 34-39 mit antiinflammatorischer und/oder analgetischer Wirkung.

41. Verbindung gemäss einem der Ansprüche 1, 4, 6, 8, 9 und 12-33 mit antiinflammatorischer und/oder analgetischer Wirkung.

42. Verbindung gemäss einem der Ansprüche 1-39 mit Wirkung als Lichtschutzmittel.

43. Die in den Beispielen 20-30 und 33 genannten neuen Verbindungen.

44. Die in den Beispielen 1-19 und 31 genannten neuen Verbindungen.

45. Verbindung gemäss einem der Ansprüche 1-41 zur therapeutischen Behandlung des menschlichen und tierischen Körpers.

46. Pharmazeutische Präparate, enthaltend eine Verbindung gemäss einem der Ansprüche 2, 3, 5, 7, 10, 11 und 34-39 neben üblichen pharmazeutischen Hilfs- und Trägerstoffen.

47. Pharmazeutische Präparate, enthaltend eine Verbindung gemäss einem der Ansprüche 1, 4, 6, 8, 9, 12-33 und 41 neben üblichen pharmazeutischen Hilfs- und Trägerstoffen.

48. Lichtschutzmittel, enthaltend eine Verbindung gemäss einem der Ansprüche 1-39 und 42 neben üblichen Hilfs- und Trägerstoffen.

49. Verfahren zur Herstellung von Benzofuranone der allgemeinen Formel

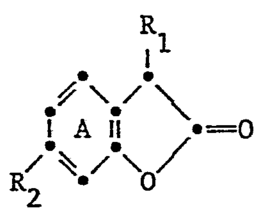

(I),

worin $R_1$ für Wasserstoff oder einen aliphatischen Rest steht, $R_2$ eine durch einen zweiwertigen Kohlenwasserstoffrest disubstituierte Aminogruppe bedeutet und der aromatische Ring A zusätzlich substituiert sein kann, und ihrer Salze und/oder Isomeren, dadurch gekennzeichnet, dass man

a) eine Verbindung der Formel

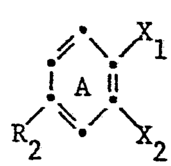

(II)

oder ein Salz davon, worin $X_1$ eine Gruppe der Formel $-CH(R_1)-X_3$ und $X_3$ Carboxy oder funktionell abgewandeltes Carboxy bedeutet und $X_2$ Hydroxy oder funktionell abgewandeltes Hydroxy darstellt oder worin $X_1$ Wasserstoff und $X_2$ eine Gruppe der Formel $-O-CO-CH(R_1)-X_4$ bedeutet, in der $X_4$ Hydroxy oder funktionell abgewandeltes Hydroxy darstellt, cyclisiert oder

b) in einer Verbindung der Formel

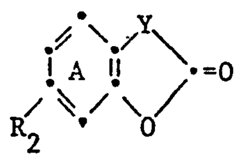

(III),

einem Salz oder Isomeren davon, worin Y einen in die Gruppe der Formel $>CH(R_1)$ überführbaren Rest darstellt, Y in die Gruppe der Formel $>CH(R_1)$ überführt oder

c) in einer Verbindung der Formel

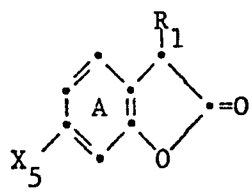

(IV),

oder einem Salz oder Isomeren davon, worin $X_5$ eine in $R_2$ überführbare Gruppe darstellt, $X_5$ in $R_2$ überführt oder

d) in einer Verbindung der Formel

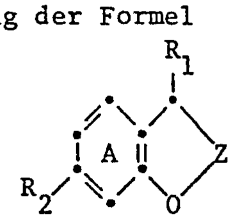

(V)

oder einem Salz davon, worin Z eine in die Carbonylgruppe überführbare Gruppe bedeutet, Z in die Carbonylgruppe überführt oder

e) in einer Verbindung der Formel

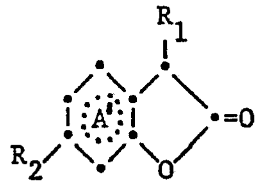

(VI)

oder einem Salz davon, worin der Ring A' ein in den Ring A überführbarer Ring ist, den Ring A' in den Ring A überführt und/oder, wenn erwünscht, ein verfahrensgemäss erhältliches Salz in die freie Verbindung oder in ein anderes Salz überführt, eine verfahrensgemäss erhältliche freie Verbindung in eine andere freie Verbindung oder in ein Salz überführt und/oder gewünschtenfalls ein verfahrensgemäss erhältliches Isomerengemisch in ihre Komponenten auftrennt.

50. Verfahren nach Anspruch 49, dadurch gekennzeichnet, dass man eine Verbindung der Formel (III), worin Y für eine Gruppe der Formel $>C(R_4)-COOH$ steht, decarboxyliert oder in einer Verbindung der Formel (III), worin Y für eine Gruppe der Formel $>C=R_1'$ steht und $R_1'$ einen bivalenten aliphatischen Rest oder eine tautomere Form davon bedeutet, die Doppelbindung reduziert, oder eine Verbindung der Formel (III), worin Y für eine Gruppe der Formel $>C(R_1)-X_{11}$ steht und $X_{11}$ Hydroxy, Alkylthio, Dialkylamino oder im Phenylteil jeweils gegebenenfalls substituiertes Di-phenyl-sulfamoyl bedeutet oder worin Y Carbonyl darstellt, mit Hilfe eines Reduktionsmittels in die Gruppe der Formel $>CH(R_1)$ überführt.

51. Verfahren nach Anspruch 49, dadurch gekennzeichnet, dass man eine Verbindung der Formel (IV), worin $X_5$ eine Gruppe der Formel $-NH-A_1-X_7$ darstellt, wobei $A_1$ Niederalkylen, Niederalkenylen, durch Aza, N-Niederalkylaza, Oxa oder Thia unterbrochenes Niederalkylen oder durch Aza, N-Niederalkylaza, Oxa oder Thia unterbrochenes Niederalkenylen bedeutet und $X_7$ Hydroxy oder reaktionsfähiges ver-

estertes Hydroxy darstellt, mit einer Base behandelt, oder in eine Verbindung der Formel (IV), worin $X_5$ für Amino steht, durch Umsetzung mit einem 2,5-Diniederalkoxy-tetrahydrofuran oder durch Umsetzung mit 2-Buten-1,4-diol oder einem reaktionsfähig veresterten Derivat davon, Pyrrolin-1-yl einführt und dieses in Gegenwart eines Dehydrierungskatalysators zum Pyrrol-1-yl $R_2$ dehydriert oder durch Umsetzung mit 1,4-Dioxo-butan den Pyrrolring $R_2$ einführt, oder in eine Verbindung der Formel (IV), worin $X_5$ für einen durch $R_2$ ersetzbaren Rest steht, und der gegebenenfalls in Nachbarposition von $X_5$ mindestens einen Substituenten mit -I- oder -M-Effekt aufweist, nucleophile Amine $R_2$-H einführt, oder eine Verbindung der Formel (IV), worin $X_5$ für Wasserstoff steht und sich in Nachbarposition von $X_5$ mindestens ein Substituent mit -I- oder -M-Effekt befindet, zunächst mit einem Oxidationsmittel und anschliessend mit einem nucleophilen Amin $R_2$-H behandelt.

52. Verfahren nach Anspruch 49, dadurch gekennzeichnet, dass man eine Verbindung der Formel (V), worin Z für die Methylengruppe steht, Z mit Hilfe eines Oxidationsmittels zur Carbonylgruppe oxidiert oder in einer Verbindung der Formel (V), worin Z Thiocarbonyl oder gegebenenfalls N-substituierte Iminomethylen darstellt, Z hydrolytisch in die Carbonylgruppe überführt.

53. Verfahren nach Anspruch 49, dadurch gekennzeichnet, dass man eine Verbindung der Formel (VI), worin der Ring A' das Substitutionsmuster des Rings A und zwei Doppelbindungen sowie zusätzlich an zwei C-Atomen jeweils ein Wasserstoffatom enthält, in Gegenwart eines Dehydrierungsmittels dehydriert.

54. Verfahren nach Anspruch 49 zur Herstellung von Verbindungen der Formel

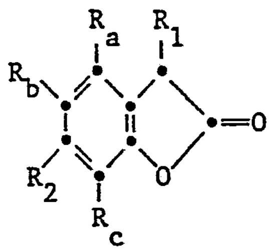

(Ia),

worin $R_1$ Methyl bedeutet, $R_a$, $R_b$ und $R_c$ Wasserstoff oder Niederalkyl bedeuten oder $R_a$ und $R_b$ gemeinsam 3- oder 4-gliedriges Alkylen darstellen, $R_c$ die vorstehende Bedeutung haben und $R_2$ die in Anspruch 1 angegebene Bedeutung hat, und deren Salzen und Isomeren, dadurch gekennzeichnet, dass man Verbindungen der Formel

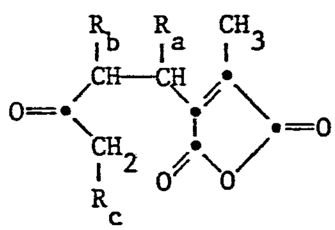

(IIb)

mit Aminen der Formel $R_2$-H (IIc) oder deren Salzen umsetzt.

55. Verfahren nach einem der Ansprüche 49 - 54, dadurch gekennzeichnet, dass man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

56. Verfahren zur Herstellung von pharmazeutischen Präparaten, dadurch gekennzeichnet, dass man eine Verbindung gemäss einem der Ansprüche 1-41 mit üblichen Hilfs- und Trägerstoffen vermischt.

57. Verwendung von Verbindungen gemäss einem der Ansprüche 1-41 in einem Verfahren zur Behandlung entzündlicher und/oder rheumatischer Erkrankungen und/oder von Schmerzzuständen.

58. Das Verfahren der Beispiele 1 - 33 und die danach erhältlichen neuen Verbindungen.

Patentansprüche : (für Oesterreich)

1. Verfahren zur Herstellung von Benzofuranone der allgemeinen Formel

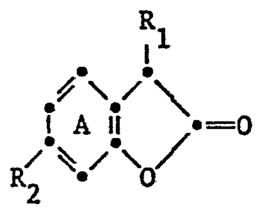

(I),

worin $R_1$ für Wasserstoff oder einen aliphatischen Rest steht, $R_2$ eine durch einen zweiwertigen Kohlenwasserstoffrest disubstituierte Aminogruppe bedeutet und der aromatische Ring A zusätzlich substituiert sein kann, und ihrer Salze und/oder Isomeren, dadurch gekennzeichnet, dass man

a) eine Verbindung der Formel

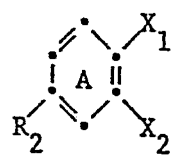

(II)

oder ein Salz davon, worin $X_1$ eine Gruppe der Formel $-CH(R_1)-X_3$ und $X_3$ Carboxy oder funktionell abgewandeltes Carboxy bedeutet und $X_2$ Hydroxy oder funktionell abgewandeltes Hydroxy darstellt oder worin $X_1$ Wasserstoff und $X_2$ eine Gruppe der Formel $-O-CO-CH(R_1)-X_4$ bedeutet, in der $X_4$ Hydroxy oder funktionell abgewandeltes Hydroxy darstellt, cyclisiert oder

b) in einer Verbindung der Formel

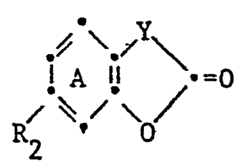

(III),

einem Salz oder Isomeren davon, worin Y einen in die Gruppe der Formel $>CH(R_1)$ überführbaren Rest darstellt, Y in die Gruppe der Formel $>CH(R_1)$ überführt oder

c) in einer Verbindung der Formel

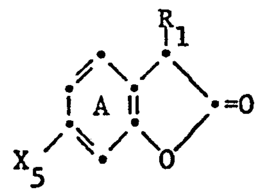

(IV),

oder einem Salz oder Isomeren davon, worin $X_5$ eine in $R_2$ überführbare Gruppe darstellt, $X_5$ in $R_2$ überführt oder

d) in einer Verbindung der Formel

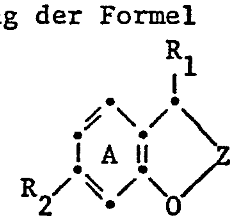

(V)

oder einem Salz davon, worin Z eine in die Carbonylgruppe überführbare Gruppe bedeutet, Z in die Carbonylgruppe überführt oder

e) in einer Verbindung der Formel

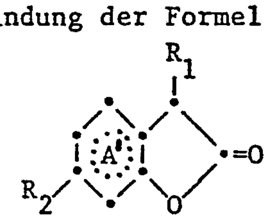

(VI)

oder einem Salz davon, worin der Ring A' ein in den Ring A überführbarer Ring ist, den Ring A' in den Ring A überführt und/oder, wenn erwünscht, ein verfahrensgemäss erhältliches Salz in die freie Verbindung oder in ein anderes Salz überführt, eine verfahrensgemäss erhältliche freie Verbindung in eine andere freie Verbindung oder in ein Salz überführt und/oder gewünschtenfalls ein verfahrensgemäss erhältliches Isomerengemisch in ihre Komponenten auftrennt.

2. Verfahren nach Anspruch 1 zur Herstellung von Benzofuranone der allgemeinen Formel

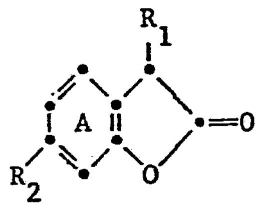

(I),

worin $R_1$ für Wasserstoff oder einen aliphatischen Rest steht, $R_2$ eine durch einen zweiwertigen Kohlenwasserstoffrest disubstituierte Aminogruppe bedeutet und der aromatische Ring A zusätzlich substituiert sein kann, und ihre Salze und/oder Isomeren, dadurch gekennzeichnet, dass man

a) eine Verbindung der Formel

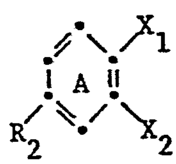

(II)

oder ein Salz davon, worin $X_1$ eine Gruppe der Formel $-CH(R_1)-X_3$ und $X_3$ Carboxy oder funktionell abgewandeltes Carboxy bedeutet und $X_2$ Hydroxy oder funktionell abgewandeltes Hydroxy darstellt oder worin $X_1$ Wasserstoff und $X_2$ eine Gruppe der Formel $-O-CO-CH(R_1)-X_4$ bedeutet, in der $X_4$ Hydroxy oder funktionell abgewandeltes Hydroxy darstellt, cyclisiert oder

b) in einer Verbindung der Formel

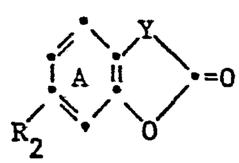

(III),

einem Salz oder Isomeren davon, worin Y einen in die Gruppe der Formel $>CH(R_1)$ überführbaren Rest darstellt, Y in die Gruppe der Formel $>CH(R_1)$ überführt oder

c) in einer Verbindung der Formel

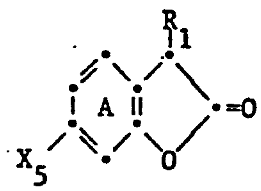

(IV),

oder einem Salz oder Isomeren davon, worin $X_5$ eine in $R_2$ überführbare Gruppe darstellt, $X_5$ in $R_2$ überführt und/oder, wenn erwünscht, ein verfahrensgemäss erhältliches Salz in die freie Verbindung oder in ein anderes Salz überführt, eine verfahrensgemäss erhältliche freie Verbindung in eine andere freie Verbindung oder in ein Salz überführt und/oder gewünschtenfalls ein verfahrensgemäss erhältliches Isomerengemisch in ihre Komponenten auftrennt.

3. Verfahren nach Anspruch 1, dadurch gekennzeichnet, dass man eine Verbindung der Formel (III), worin Y für eine Gruppe der Formel $>C(R_4)-COOH$ steht, decarboxyliert oder in einer Verbindung der Formel (III), worin Y für eine Gruppe der Formel $>C=R_1'$ steht und $R_1'$ einen bivalenten aliphatischen Rest oder eine tautomere Form davon bedeutet, die Doppelbindung reduziert, oder eine Verbindung der Formel (III), worin Y für eine Gruppe der Formel $>C(R_1)-X_{11}$ steht und $X_{11}$ Hydroxy, Alkylthio, Dialkylamino oder im Phenylteil jeweils gegebenenfalls substituiertes Di-phenyl-sulfamoyl bedeutet oder worin Y Carbonyl darstellt, mit Hilfe eines Reduktionsmittels in die Gruppe der Formel $>CH(R_1)$ überführt.

4. Verfahren nach Anspruch 1, dadurch gekennzeichnet, dass man eine Verbindung der Formel (IV), worin $X_5$ eine Gruppe der Formel $-NH-A_1-X_7$ darstellt, wobei $A_1$ Niederalkylen, Niederalkenylen, durch Aza, N-Niederalkylaza, Oxa oder Thia unterbrochenes Niederalkylen oder durch Aza, N-Niederalkylaza, Oxa oder Thia unterbrochenes Niederalkenylen bedeutet und $X_7$ Hydroxy oder reaktionsfähiges verestertes Hydroxy darstellt, mit einer Base behandelt, oder in eine Verbindung der Formel (IV), worin $X_5$ für Amino steht, durch Umsetzung mit einem 2,5-Diniederalkoxy-tetrahydrofuran oder durch Umsetzung mit 2-Buten-1,4-diol oder einem reaktionsfähig veresterten Derivat davon, Pyrrolin-1-yl einführt und dieses in Gegenwart eines Dehydrierungskatalysators zum Pyrrol-1-yl $R_2$ dehydriert oder durch Umsetzung mit 1,4-Dioxo-butan den Pyrrolring $R_2$ einführt, oder in eine Verbindung der Formel (IV), worin $X_5$ für einen durch $R_2$ ersetzbaren Rest steht, und der gegebenenfalls in Nachbarposition von $X_5$ mindestens einen Substituenten mit -I- oder -M-Effekt aufweist, nucleophile Amine $R_2$-H einführt, oder eine Verbindung der Formel (IV), worin $X_5$ für Wasserstoff steht und sich in Nachbarposition von $X_5$ mindestens ein Substituent mit -I- oder -M-Effekt befindet, zunächst mit einem Oxidationsmittel und anschliessend mit einem nucleophilen Amin $R_2$-H behandelt.

5. Verfahren nach Anspruch 1, dadurch gekennzeichnet, dass man eine Verbindung der Formel (V), worin Z für die Methylengruppe steht, Z mit Hilfe eines Oxidationsmittels zur Carbonylgruppe oxidiert oder in einer Verbindung der Formel (V), worin Z Thiocarbonyl oder gegebenenfalls N-substituierte Iminomethylen darstellt, Z hydrolytisch in die Carbonylgruppe überführt.

6. Verfahren nach Anspruch 1, dadurch gekennzeichnet, dass man eine Verbindung der Formel (VI), worin der Ring A' das Substitutionsmuster des Rings A und zwei Doppelbindungen sowie zusätzlich an zwei C-Atomen jeweils ein Wasserstoffatom enthält, in Gegenwart eines Dehydrierungsmittels dehydriert.

7. Verfahren nach einem der Ansprüche 1 und 2 - 6, dadurch gekennzeichnet, dass man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

8. Verfahren nach Anspruch 2 zur Herstellung von Verbindungen der Formel

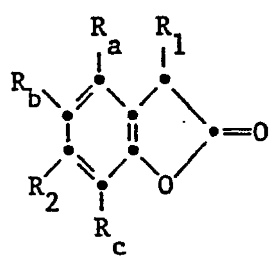

(Ia),

worin $R_1$ Methyl bedeutet, $R_a$, $R_b$ und $R_c$ Wasserstoff oder Niederalkyl bedeuten oder $R_a$ und $R_b$ gemeinsam 3- oder 4-gliedriges Alkylen darstellen, $R_c$ die vorstehende Bedeutung haben und $R_2$ die in Anspruch 1 angegebene Bedeutung hat, und deren Salzen und Isomeren, dadurch gekennzeichnet, dass man Verbindungen der Formel

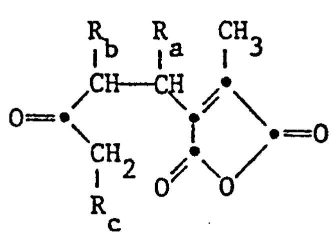

(IIb)

mit Aminen der Formel $R_2$-H (IIc) oder deren Salzen umsetzt.

9 . Verfahren nach Anspruch 2 , dadurch gekennzeichnet, dass man eine Verbindung der Formel (II), worin $X_1$ eine Gruppe der Formel $-CH(R_1)-X_3$ und $X_3$ Carboxy oder verestertes oder amidiertes Carboxy bedeutet und $X_2$ gegebenenfalls funktionell abgewandeltes Hydroxy darstellt, gegebenenfalls in Gegenwart eines katalytischen Mittels cyclisiert.

10. Verfahren nach Anspruch 2 , dadurch gekennzeichnet, dass man eine Verbindung der Formel (III), worin Y für eine Gruppe der Formel $>C(R_1)-COOH$ steht, decarboxyliert oder in einer Verbindung der Formel (III), worin Y für eine Gruppe der Formel $>C=R_1'$ steht und $R_1'$ einen bivalenten aliphatischen Rest oder eine tautomere Form davon bedeutet, Y zu einer Gruppe der Formel $>CH(R_1)$ reduziert, wobei $R_1$ von Wasserstoff verschieden ist.

11. Verfahren nach einem der Ansprüche 2 und 8 - 10, dadurch gekennzeichnet, dass man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

12. Verfahren gemäss Anspruch 1, dadurch gekennzeichnet, dass man Verbindungen der Formel I,

worin $R_1$ Wasserstoff oder Niederalkyl bedeutet, $R_2$ eine durch Niederalkylen, Niederalkenylen, Aza-niederalkylen, N'-Niederalkylaza-niederalkylen, Aza-niederalkenylen, N'-Niederalkylaza-niederalkenylen oder Oxa- bzw. Thia-niederalkenylen disubstituierte Aminogruppe, wobei Niederalkylen bzw. Niederalkenylen jeweils 4- bis 10 C-Atome aufweist und auch verzweigt sein kann sowie mit einem oder zwei Benzosystemen ortho-anelliert sein kann, darstellt und der aromatische Ring A zusätzlich durch Niederalkyl, Hydroxyniederalkyl,

Halogenniederalkyl, Niederalkenyl, gegebenenfalls verzweigtes 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze und Isomeren.

13. Verfahren nach einem der Ansprüche 1 und 3-7, dadurch gekennzeichnet, dass man Verbindungen der Formel (I), worin $R_1$ Wasserstoff oder Niederalkyl bedeutet, $R_2$ eine durch Niederalkylen, Niederalkenylen, Aza-niederalkylen, N'-Niederalkylazaniederalkylen, Aza-Niederalkenylen, N'-Niederalkylaza-niederalkenylen, Oxa- bzw. Thianiederalkylen oder Oxa- bzw. Thia-niederalkenylen disubstituierte Aminogruppe, wobei Niederalkylen bzw. Niederalkenylen jeweils 4- bis 10 C-Atome aufweist und auch verzweigt sein kann sowie mit einem oder zwei Benzosystemen ortho-anelliert sein kann, darstellt und der aromatische Ring A zusätzlich durch Niederalkyl, Hydroxyniederalkyl, Halogenniederalkyl, Niederalkenyl, gegebenenfalls verzweigtes 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze und Isomeren herstellt.

14. Verfahren nach einem der Ansprüche 2 und 8 - 11, dadurch gekennzeichnet, dass man Verbindungen der Formel (I), worin $R_1$ Wasserstoff oder Niederalkyl bedeutet, $R_2$ eine durch Niederalkylen, Niederalkenylen oder durch Aza, N-Niederalkylaza, Oxa oder Thia unterbrochenes Niederalkylen bzw. durch Aza oder N-Niederalkylaza unterbrochenes Niederalkylen substituierte Aminogruppe darstellt und der aromatische Ring A zusätzlich durch Niederalkyl, Niederalkoxy, Hydroxy, Halogen, Niederalkanoyloxy, 3- oder 4-gliedriges Alkylen und/oder Trifluormethyl substituiert sein kann, und ihre Salze und Isomeren herstellt.

15. Verfahren nach einem der Ansprüche 1 und 3 - 7, dadurch gekennzeichnet, dass man Verbindungen der Formel (Ia)

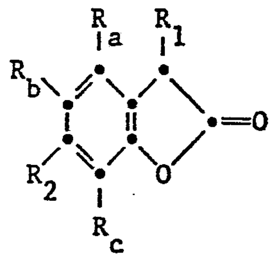

(Ia) gemäss Anspruch 1,

worin $R_1$ Wasserstoff oder Niederalkyl bedeutet, $R_2$ jeweils 5- bis 8-gliedriges Niederalkylenamino, Niederalkenylenamino, Azaniederalkylen-amino, N'-Niederalkylaza-niederalkylen-amino, Azaniederalkenylen-amino, N'-Niederalkylaza-niederalkenylen-amino, Oxa- bzw. Thia-niederalkylen-amino, Isoindol-2-yl, Isoindolin-2-yl, Indolin-1-yl oder Indol-1-yl darstellt und $R_a$, $R_b$ sowie $R_c$ unabhängig voneinander Wasserstoff, Niederalkyl, Hydroxyniederalkyl, Halogenniederalkyl, Niederalkenyl, Niederalkoxy, Niederalkylthio, Niederalkansulfinyl, Niederalkansulfonyl, Hydroxy, Halogen, Niederalkanoyloxy, Niederalkanoyl oder Nitro bedeuten oder $R_a$ gemeinsam mit $R_b$ 3- oder 4-gliedriger Alkylen, darstellen und $R_c$ die vorstehend für $R_c$ angegebenen Bedeutungen hat, und ihre Salze und Isomeren herstellt.

16. Verfahren nach einem der Ansprüche 2 und 8 - 11, dadurch gekennzeichnet, dass man Verbindungen der Formel (Ia) worin $R_1$ Wasserstoff oder Niederalkyl bedeutet, $R_2$ jeweils 5- bis 8-gliedriges Niederalkylenamino, Niederalkenylenamino, Monoazaniederalkylenamino, N'-Niederalkylmonoaza-niederalkylen-amino, Monooxa-niederalkylenamino, Monothia-niederalkylenamino, Monoaza-niederalkenylenamino oder N'-Niederalkylaza-niederalkenylen-amino darstellt und $R_a$, $R_b$ sowie $R_c$ unabhängig voneinander Wasserstoff, Niederalkyl, Niederalkoxy, Hydroxy, Halogen, Niederalkanoyloxy oder Trifluormethyl bedeuten oder $R_a$ gemeinsam mit $R_b$ 3- oder 4-gliedriges Alkylen darstellen und $R_c$ die vorstehend für $R_c$ angegebenen Bedeutungen hat, und ihre Salze und Isomeren herstellt.

17. Verfahren nach einem der Ansprüche 1 und 3 - 7, dadurch gekennzeichnet, dass man Verbindungen der Formel (Ia), worin $R_1$ Wasserstoff oder Niederalkyl mit bis und mit 4 C-Atomen bedeutet, $R_2$ 5- bis 8-gliedriges Niederalkylen-amino mit 4 bis 10 C-Atomen, 5- bis 8-gliedriges Niederalkenylen-amino mit einer oder zwei Doppelbindungen und mit 4 bis 10 C-Atomen, Monooxa-niederalkylen-amino mit 4 bis 7 C-Atomen, Indolin-1-yl oder Indol-1-yl darstellt und $R_a$ und $R_b$ unabhängig voneinander Wasserstoff, Niederalkyl oder Halogen oder $R_a$ und $R_b$ gemeinsam 3- bis 4-gliedriges Alkylen bedeuten und $R_c$ Wasserstoff darstellt, und ihre Salze und Isomeren herstellt.

18. Verfahren nach einem der Ansprüche 2 und 8 11, dadurch gekennzeichnet, dass man Verbindungen der Formel (Ia), worin $R_1$ Wasserstoff oder Niederalkyl bedeutet, $R_2$ 5- bis 8-gliedriges Niederalkylenamino, 5- bis 8-gliedriges Niederalkenylenamino oder Monooxa-niederalkylenamino darstellt und $R_a$ sowie $R_b$ unabhängig voneinander Wasserstoff, Niederalkyl oder Halogen oder $R_a$ gemeinsam mit $R_b$ 3- bis 4-gliedriges Alkylen bedeuten, und $R_c$ Wasserstoff darstellt, und ihre Salze und Isomeren herstellt.

19. Verfahren nach einem der Ansprüche 2 und 8 ÷ 11, dadurch gekennzeichnet, dass man Verbindungen der Formel (Ia), worin $R_1$ Niederalkyl mit bis und mit 4 C-Atomen bedeutet, $R_2$ 5- bis 8-gliedriges Niederalkenylenamino darstellt, $R_a$ und $R_c$ Wasserstoff bedeuten und $R_b$ Niederalkyl mit bis und mit 4 C-Atomen darstellt, und ihre Salze und ihre Isomeren herstellt.

20. Verfahren nach einem der Ansprüche 1 und 3 - 7, dadurch gekennkennzeichnet, dass man Verbindungen der Formel (Ia), worin $R_1$ Wasserstoff oder Niederalkyl mit bis und mit 4 C-Atomen bedeutet, $R_2$ 1-Pyrrolyl, 3-Pyrrolidin-1-yl, Pyrrolidin-1-yl oder Piperidin-1-yl darstellt, $R_a$ und $R_c$ jeweils Wasserstoff bedeuten und $R_b$ Niederalkyl mit bis und mit 4 C-Atomen oder Halogen bis und mit Atomnummer 35 darstellt, und ihre Salze und Isomeren herstellt.

21. Verfahren nach einem der Ansprüche 1 und 3 - 7, dadurch gekenn-zeichnet, dass man Verbindungen der Formel (Ia), worin $R_1$ Wasserstoff oder Niederalkyl mit bis und mit 4 C-Atomen bedeutet, $R_2$ 1-Pyrrolyl, 4-Morpholinyl, 3-Pyrrolin-1-yl oder unver-zeigtes 4- bis 6-gliedriges Alkylenamino darstellt, $R_a$ und $R_c$ jeweils Wasserstoff bedeuten und $R_b$ Wasserstoff, Niederalkyl mit bis und mit

22. Verfahren nach einem der Ansprüche 2 und 8 - 11, dadurch gekenn-zeichnet, dass man Verbindungen der Formel (Ia), worin $R_1$ Wasserstoff oder Niederalkyl mit bis und mit 4 C-Atomen bedeutet, $R_2$ 5- bis 8-gliedriges Niederalkylenamino, 5- bis 8-gliedriges Niederalkenylenamino oder Monooxa-niederalkylenamino darstellt und $R_a$ und $R_c$ Wasserstoff bedeuten und $R_b$ Wasserstoff, Niederalkyl mit bis und mit 4 C-Atomen oder Halogen bis und mit Atomnummer 35 darstellt, und ihre Salze und Isomeren herstellt.

23. Verfahren nach einem der Ansprüche 1 und 3 - 7, dadurch gekennzeichnet, dass man 3-Methyl-6-morpholino-benzofuran-2(3H)-on oder ein Salz oder Isomeres davon herstellt.

24. Verfahren nach einem der Ansprüche 1 und 3 - 7, dadurch gekennzeichnet, dass man 3-Methyl-6-(pyrrolidin-1-yl)-benzofuran-2(3H)-on oder ein Salz oder Isomeres davon herstellt.

25. Verfahren nach einem der Ansprüche 1 und 3 - 7, dadurch gekennzeichnet, dass man 3-Methyl-6-(piperidin-1-yl)-benzofuran-2(3H)-on oder ein Salz oder Isomeres davon herstellt.

26. Verfahren nach einem der Ansprüche 1 und 3 - 7, dadurch gekennzeichnet, dass man 6-(Hexahydroazepin-1-yl)-3-methyl-benzofuran-2(3H)-on oder ein Salz oder Isomeres davon herstellt.

27. Verfahren nach einem der Ansprüche 1 und 3 - 7, dadurch gekennzeichnet, dass man 3,5-Dimethyl-6-(pyrrolidin-1-yl)-benzofuran-2(3H)-on oder ein Salz oder Isomeres davon herstellt.

28. Verfahren nach einem der Ansprüche 1 und 3 - 7, dadurch gekennzeichnet, dass man 3,5-Dimethyl-6-morpholino-benzofuran-2(3H)-on oder ein Salz oder Isomeres davon herstellt.

29. Verfahren nach einem der Ansprüche 1 und 3 - 7, dadurch gekennzeichnet, dass man 3,4-Dimethyl-6-morpholino-benzofuran-2(3H)-on oder ein Salz oder Isomeres davon herstellt.

30. Verfahren nach einem der Ansprüche 1 und 3 - 7, dadurch gekennzeichnet, dass man 5-Chlor-3-methyl-6-morpholino-benzofuran-2(3H)-on oder ein Salz oder Isomeres davon herstellt.

31. Verfahren nach einem der Ansprüche 1 und 3 - 7, dadurch gekennzeichnet, dass man 5-Chlor-3-methyl-6-(piperidin-1-yl)-benzofuran-2(3H)-on oder ein Salz oder Isomeres davon herstellt.

32. Verfahren nach einem der Ansprüche 1 und 3 - 7, dadurch gekennzeichnet, dass man 5-Chlor-3-methyl-6-(pyrrolidin-1-yl)-benzofuran-2(3H)-on oder ein Salz oder Isomeres davon herstellt.

33. Verfahren nach einem der Ansprüche 1 und 3 - 7, dadurch gekennzeichnet, dass man 5-Brom-3-methyl-6-morpholino-benzofuran-2(3H)-on oder ein Salz oder Isomeres davon herstellt.

34. Verfahren nach einem der Ansprüche 1 und 3 - 7, dadurch gekennzeichnet, dass man 5-Brom-3-methyl-6-(pyrrolidin-1-yl)-benzofuran-2(3H)-on oder ein Salz oder Isomeres davon herstellt.

35. Verfahren nach einem der Ansprüche 1 und 3 - 7, dadurch gekennzeichnet, dass man 5-Brom-3,4-dimethyl-6-morpholino-benzofuran-2(3H)-on oder ein Salz oder Isomeres davon herstellt.

36. Verfahren nach einem der Ansprüche 1 und 3 - 7, dadurch gekennzeichnet, dass man 5-Chlor-6-(piperidin-1-yl)-benzofuran-2(3H)-on oder ein Salz oder Isomeres davon herstellt.

37. Verfahren nach einem der Ansprüche 1 und 3 - 7, dadurch gekennzeichnet, dass man 6-(4-Morpholino)-benzofuran-2(3H)-on oder ein Salz oder Isomeres davon herstellt.

38. Verfahren nach einem der Ansprüche 1 und 3 - 7, dadurch gekennzeichnet, dass man 5-Chlor-3-methyl-6-(pyrrol-1-yl)-benzofuran-2(3H)-on oder ein Salz oder Isomeres davon herstellt.

39. Verfahren nach einem der Ansprüche 1 und 3 - 7, dadurch gekennzeichnet, dass man 3,5-Dimethyl-6-(hexahydroazepin-1-yl)-benzofuran-2(3H)-on oder ein Salz oder Isomeres davon herstellt.

40. Verfahren nach einem der Ansprüche 1 und 3 - 7, dadurch gekennzeichnet, dass man 3,5-Dimethyl-6-(piperidin-1-yl)-benzofuran-2(3H)-on oder ein Salz oder Isomeres davon herstellt.

41. Verfahren nach einem der Ansprüche 1 und 3 - 7, dadurch gekennzeichnet, dass man 5-Ethyl-3-methyl-6-(pyrrolidin-1-yl)-benzofuran-2(3H)-on oder ein Salz oder Isomeres davon herstellt.

42. Verfahren nach einem der Ansprüche 2 und 8 - 11, dadurch gekennzeichnet, dass man 3-Methyl-6-(pyrrolidin-1-yl)-4,5-tetramethylen-benzofuran-2(3H)-on oder ein Salz oder Isomeres davon herstellt.

43. Verfahren nach einem der Ansprüche 2 und 8 - 11, dadurch gekennzeichnet, dass man 3,5-Dimethyl-6-(pyrrol-1-yl)-benzofuran-2(3H)-on oder ein Salz oder Isomeres davon herstellt.

44. Verfahren nach einem der Ansprüche 2 und 8 - 11, dadurch gekennzeichnet, dass man 3,5-Dimethyl-6-(3-pyrrolin-1-yl)-benzofuran-2(3H)-on oder ein Salz oder Isomeres davon herstellt.

45. Verfahren nach einem der Ansprüche 2 und 8 - 11, dadurch gekennzeichnet, dass man 3,5-Dimethyl-6-(indolin-1-yl)-benzofuran-2(3H)-on oder ein Salz oder Isomeres davon herstellt.

46. Verfahren nach einem der Ansprüche 2 und 8 - 11, dadurch gekennzeichnet, dass man 3,5-Dimethyl-6-(indol-1-yl)-benzofuran-2(3H)-on oder ein Salz oder Isomeres davon herstellt.

47. Verfahren nach einem der Ansprüche 2 und 8 - 11, dadurch gekennzeichnet, dass man 3,5-Dimethyl-6-(cis-3,4-dimethyl-pyrrolidin-1-yl)-benzofuran-2(3H)-on oder ein Salz oder Isomeres davon herstellt.

48. Verfahren nach einem der Ansprüche 2 und 8 - 11, dadurch gekennzeichnet, dass man 3,5-Dimethyl-6-(trans-3,4-dimethyl-pyrrolidin-1-yl)- und benzofuran-2(3H)-on oder ein Salz oder Isomeres davon herstellt.

49. Verfahren nach einem der Ansprüche 2 und 8 - 11, dadurch gekennzeichnet, dass man 3,5-Dimethyl-6-(2,5-dimethyl-pyrrol-1-yl)-benzofuran-2(3H)-on oder ein Salz oder Isomeres davon herstellt.

50. Verfahren nach einem der Ansprüche 2 und 8 - 11, dadurch gekennzeichnet, dass man 3-Methyl-6-morpholino-benzofuran-2(3H)-on oder ein Salz oder Isomeres davon herstellt.

51. Verfahren nach einem der Ansprüche 2 und 8 - 11, dadurch gekennzeichnet, dass man 3-Methyl-6-(pyrrolidin-1-yl)-benzofuran-2(3H)-on oder ein Salz oder Isomeres davon herstellt.

52. Verfahren nach einem der Ansprüche 2 und 8 - 11, dadurch gekennzeichnet, dass man 3-Methyl-6-(piperidin-1-yl)-benzofuran-2(3H)-on oder ein Salz oder Isomeres davon herstellt.

53. Verfahren nach einem der Ansprüche 2 und 8 - 11, dadurch gekennzeichnet, dass man 6-(Hexahydroazepin-1-yl)-3-methyl-benzofuran-2(3H)-on oder ein Salz oder Isomeres davon herstellt.

54. Verfahren nach einem der Ansprüche 2 und 8 - 11, dadurch gekennzeichnet, dass man 3,5-Dimethyl-6-(pyrrolidin-1-yl)-benzofuran-2(3H)-on oder ein Salz oder Isomeres davon herstellt.

55. Verfahren nach einem der Ansprüche 2 und 8 - 11, dadurch gekennzeichnet, dass man 3,5-Dimethyl-6-morpholino-benzofuran-2(3H)-on oder ein Salz oder Isomeres davon herstellt.

56. Verfahren nach einem der Ansprüche 2 und 8 - 11, dadurch gekennzeichnet, dass man 3,4-Dimethyl-6-morpholino-benzofuran-2(3H)-on oder ein Salz oder Isomeres davon herstellt.

57. Verfahren nach einem der Ansprüche 2 und 8 - 11, dadurch gekennzeichnet, dass man 5-Chlor-3-methyl-6-morpholino-benzofuran-2(3H)-on oder ein Salz oder Isomeres davon herstellt.

58. Verfahren nach einem der Ansprüche 2 und 8 - 11, dadurch gekennzeichnet, dass man 5-Chlor-3-methyl-6-(piperidin-1-yl)-benzofuran-2(3H)-on oder ein Salz oder Isomeres davon herstellt.

59. Verfahren nach einem der Ansprüche 2 und 8 - 11, dadurch gekennzeichnet, dass man 5-Chlor-3-methyl-6-(pyrrolidin-1-yl)-benzofuran-2(3H)-on oder ein Salz oder Isomeres davon herstellt.

60. Verfahren nach einem der Ansprüche 2 und 8 - 11, dadurch gekennzeichnet, dass man 5-Brom-3-methyl-6-morpholino-benzofuran-2(3H)-on oder ein Salz oder Isomeres davon herstellt.

61. Verfahren nach einem der Ansprüche 2 und 8 - 11, dadurch gekennzeichnet, dass man 5-Brom-3-methyl-6-(pyrrolidin-1-yl)-benzofuran-2(3H)-on oder ein Salz oder Isomeres davon herstellt.

62. Verfahren nach einem der Ansprüche 2 und 8 - 11, dadurch gekennzeichnet, dass man 5-Brom-3,4-dimethyl-6-morpholino-benzofuran-2(3H)-on oder ein Salz oder Isomeres davon herstellt.

63. Verfahren nach einem der Ansprüche 2 und 8 - 11, dadurch gekennzeichnet, dass man 5-Chlor-6-piperidin-1-yl)-benzofuran-2(3H)-on oder ein Salz oder Isomeres davon herstellt.

64. Verfahren nach einem der Ansprüche 2 und 8 - 11, dadurch gekennzeichnet, dass man 6-(4-Morpholino)-benzofuran-2(3H)-on oder ein Salz oder Isomeres davon herstellt.

65. Verfahren nach einem der Ansprüche 2 und 8 - 11, dadurch gekennzeichnet, dass man 5-Chlor-3-methyl-6-(pyrrol-1-yl)-benzofuran-2(3H)-on oder ein Salz oder Isomeres davon herstellt.

66. Verfahren nach einem der Ansprüche 2 und 8 - 11, dadurch gekennzeichnet, dass man 3,5-Dimethyl-6-(hexahydroazepin-1-yl)-benzofuran-2(3H)-on oder ein Salz oder Isomeres davon herstellt.

67. Verfahren nach einem der Ansprüche 2 und 8 - 11, dadurch gekennzeichnet, dass man 3,5-Dimethyl-6-(piperidin-1-yl)-benzofuran-2(3H)-on oder ein Salz oder Isomeres davon herstellt.

68. Verfahren nach einem der Ansprüche 2 und 8 - 11, dadurch gekennzeichnet, dass man 5-Ethyl-3-methyl-6-(pyrrolidin-1-yl)-benzofuran-2(3H)-on oder ein Salz oder Isomeres davon herstellt.

69. Verfahren nach einem der Ansprüche 2 und 8 - 11, dadurch gekennzeichnet, dass man 3-Methyl-6-(pyrrolidin-1-yl)-4,5-tetramethylen-benzofuran-2(3H)-on oder ein Salz oder Isomeres davon herstellt.

70. Verfahren nach einem der Ansprüche 2 und 8 - 11, dadurch gekennzeichnet, dass man 3,5-Dimethyl-6-(pyrrol-1-yl)-benzofuran-2(3H)-on oder ein Salz oder Isomeres davon herstellt.

71. Verfahren zur Herstellung pharmazeutischer Präparate, dadurch gekennzeichnet, dass man eine Verbindung gemäss einem der Ansprüche 1-40 mit üblichen Hilfs- und Trägerstoffen vermischt.

72. Das Verfahren der Beispiele 1-33 und die danach erhältlichen neuen Verbindungen.